# EUROPEAN PATENT SPECIFICATION

(11) **EP 3 054 498 B1**
(45) Date of publication and mention of the grant of the patent: **20.09.2017**
(21) Application number: 15154166.1
(22) Date of filing: 06.02.2015
(51) Int. Cl.: H01L 51/50, C07D 491/00, H01L 31/00, C07D 487/14, C09K 11/06, H01L 51/00

(54) **BISIMIDAZODIAZOCINES**
BISIMIDAZODIAZOZINE
BISIMIDAZODIAZOCINES

(43) Date of publication of application: 10.08.2016
(73) Proprietor: Idemitsu Kosan Co., Ltd., Chiyoda-ku Tokyo 100-8321 (JP)
(72) Inventor: FLORES, Jean-Charles, 68170 Rixheim (FR); SCHÄFER, Thomas, 4410 Liestal (CH); NAGASHIMA, Hideaki, 4057 Basel (CH)
(74) Representative: Hollah, Dorothee

(56) References cited:
- WO-A1-2014/009317
- THOMAS KAUFFMANN ET AL: "Heterocyclopolyaromaten, XII. Kondensation von 3-(1-Imidazolyl)- und 3-(1-Benzimidazolyl)-chinolin zu Heterocyclotetraaromaten", CHEMISCHE BERICHTE, vol. 115, no. 2, 1 February 1982 (1982-02-01), pages 452-458, XP055184097, ISSN: 0009-2940, DOI: 10.1002/cber.19821150207
- Thomas Kauffmann ET AL: "Printed in Great Britain. HETEROCYCLOTETRAAROMATEN AUS AZOLID-ANALOGEN VORSTUFEN (1)", Tetrahedron Letters No, 1 January 1977 (1977-01-01), pages 741-744, XP055184102, DOI: 10.1016/S0040-4039(01)92741-8 Retrieved from the Internet: URL:http://www.sciencedirect.com/science/a rticle/pii/S0040403901927418/pdf?md5=afd7b 5feee79c16abf9f8ca15ff86e19&pid=1-s2.0-S00 40403901927418-main.pdf [retrieved on 2015-04-20]
- HASSANIEN ABU ZEID A ET AL: "Aminomethylene ketones and enamines in heterocyclic synthesis: synthesis of functionally substituted pyridine, pyrazole, fused pyrimidine and fused [1,5]diazocine derivatives", JOURNAL OF CHEMICAL RESEARCH, SCIENCE REVIEWS 2000 LTD, GB, vol. 2005, no. 7, 1 July 2005 (2005-07-01) , pages 440-445, XP008176039, ISSN: 1747-5198, DOI: 10.3184/030823405774309186
- WAN YUE ET AL: "Synthesis and Properties of a New Class of Fully Conjugated Azahexacene Analogues", ANGEWANDTE CHEMIE INTERNATIONAL EDITION, vol. 53, no. 24, 5 May 2014 (2014-05-05), pages 6159-6162, XP055184091, ISSN: 1433-7851, DOI: 10.1002/anie.201403227

## Description

The present invention relates to an organic electronic device, preferably an OLED, comprising a Bisimidazolodiazocine; charge transport layer, a charge/exciton blocker layer, or an emitting layer comprising the Bisimidazolodiazocine; an apparatus selected from the group consisting of stationary visual display units; mobile visual display units; illumination units; keyboards; items of clothing; furniture; wallpaper, comprising the inventive organic electronic device, or the inventive charge transport layer, the charge/exciton blocker layer, or the emitting layer; the use of the Bisimidazolodiazocines for electrophotographic photoreceptors, photoelectric converters, organic solar cells, switching elements, organic light emitting field effect transistors, image sensors, dye lasers and electroluminescent devices; a process for the production of the Bisimidazolodiazocines and specific Bisimidazolodiazocines.

Efficient and stable organic electronic devices like electrophotographic photoreceptors, photoelectric converters, organic solar cells, switching elements, organic light emitting field effect transistors, image sensors, dye lasers and electroluminescent devices (OLEDs) are highly desired in the art. The basic principles of the way in which such organic electronic devices work and suitable structures (layers) of said organic electronic devices are known to those skilled in the art and are specified - concerning OLEDs - , for example, in WO 2005/113704 and the literature cited therein.

Organic light-emitting diodes (OLEDs) exploit the property of materials of emitting light when they are excited by electrical current. OLEDs are of particular interest as an alternative to cathode ray tubes and to liquid-crystal displays for producing flat visual display units. Owing to the very compact design and the intrinsically low power consumption, devices comprising OLEDs are suitable especially for mobile applications, for example for applications in cellphones, laptops, etc., and for illumination.

The light-emitting materials (emitters) used in OLEDs may, as well as fluorescent materials (fluorescence emitters), be phosphorescent materials (phosphorescence emitters). The phosphorescence emitters are typically organometallic complexes which, in contrast to the fluorescence emitters which exhibit singlet emission, exhibit triplet emission (triplet emitters) (M.A. Baldow et al., Appl. Phys. Lett. 1999, 75, 4 to 6). In order to implement the advantages of the use of OLEDs in practice, it is necessary to provide device compositions which have a high operative lifetime, a good efficiency, a high stability to thermal stress and a low use and operating voltage.

Such device compositions may, for example, comprise specific matrix materials in which the actual light emitter is present in distributed form. In addition, the compositions may comprise blocker materials, it being possible for hole blockers, exciton blockers and/or electron blockers to be present in the device compositions. Additionally or alternatively, the device compositions may further comprise hole injection materials and/or electron injection materials and/or hole transport materials and/or electron transport materials. The selection of the aforementioned materials which are used in combination with the actual light emitter has a significant influence on parameters including the efficiency and the lifetime of the OLEDs.

The prior art proposes numerous different materials for use in OLEDs and other organic electronic devices. However, among the proposed materials no bisimidazolodiazocines are mentioned.

WO 2014/009317 A1 relates to compounds of formula I, a process for their production and their use in electronic devices, especially electroluminescent devices. When used as hole transport material in electroluminescent devices, the compounds of formulae I may provide improved efficiency, stability, manufacturability, or spectral characteristics of electroluminescent devices.

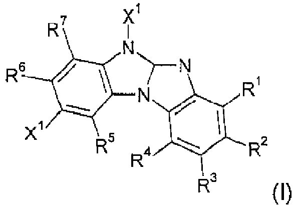

The synthesis of bisbenzimidazo[1,2-a:1',2'-e][1,5]diazocine-6,14-dicarbonitrile 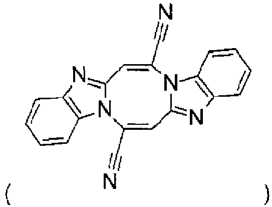 is described in Hassanien, Abu Zeid A. et al., Journal of Chemical Research 7 (2005) 440-445. However, said compound has been provided according to Hassanien et al. in the hope of obtaining new compounds of enhanced biological and pharmacological activities.

In Kaufmann et al., Tetrahedron Letters No. 9, 741-744, 1977 and Kaufmann et al., Chem. Ber. 115, 452-458, 1982, the synthesis of of bisbenzimidazo[1',2':1,8;2",1":4,5][1,5]diazocino[3,2-b:7,6- b']diquinoline 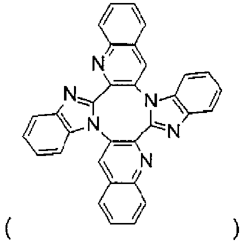 and
bisimidazo[1',2':1,8;2",'1":4,5][9,5]diazocino[3,2-b:7,6-b']diquinoline 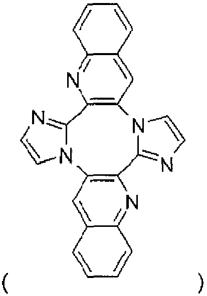 is described. However, no specific use of said compounds is described in Kaufmann et al.

It is an object of the present invention to provide materials which are suitable for use in organic electronic devices, especially OLEDs, especially for use as a matrix material, especially as a matrix material in the light-emitting layer, hole/exciton blocker material, electron/exciton blocker material, hole injection material, electron injection material, hole transport material and/or electron transport material, which have high triplet energies, and also to provide organic electronic devices, especially OLEDs, with an improved property profile which is manifested in a high efficiency a longlifetime as well as a low driving voltage.

The object is achieved by an organic electronic device, preferably an OLED, comprising a compound of formula (I) 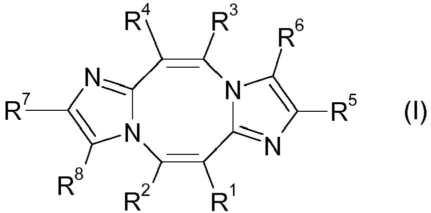 wherein
R¹, R², R³, R⁴, R⁵, R⁶, R⁷ and R⁸
are independently hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
   or
R¹ and R² and/or R³ and R⁴ an/or R⁵ and R⁶ and/or R⁷ and R⁸
independently of each other form together with the carbon atoms to which they are bonded a 5-or 6-membered (hetero)aromatic ring, which can optionally be part of an annelated ring system, wherein the (hetero)aromatic ring and/or annelated ring system can optionally be substituted by G, or by a group of formula -(Ar¹)ₒ-(Ar²)ₚ-(Ar³)_{q}-A; and the hetero atoms of the 5- or 6-membered heteroaromatic ring are selected from N, S and O; preferably,
R¹ and R² and/or R³ and R⁴,
independently of each other form a 5- or 6-membered (hetero)aromatic ring selected from 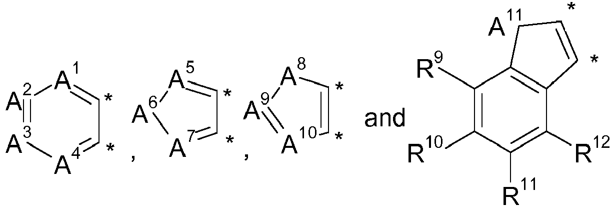 and/or
R⁵ and R⁶ and/or R⁷ and R⁸
independently of each other form a 5- or 6-membered (hetero)aromatic ring selected from 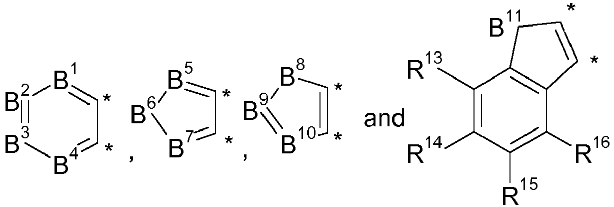 wherein
   A¹ and B¹ are independently N, or CR¹⁷,
   A² and B² are independently N, or CR¹⁸,
   A³ and B³ are independently N, or CR¹⁹,
   A⁴ and B⁴ are independently N, or CR²⁰,
   A⁵ and B⁵ are independently N, or CR²¹,
   A⁶ and B⁶ are independently O, S, NR²⁵ or CR²⁶R²⁷,
   A⁷ and B⁷ are independently N, or CR²²,
   A⁸ and B⁸ are independently O, S, NR²⁵ or CR²⁶R²⁷,
   A⁹ and B⁹ are independently N, or CR²³,
   A¹⁰ and B¹⁰ are independently N, or CR²⁴,
   A¹¹ and B¹¹ are independently O, S, NR²⁵ or CR²⁶R²⁷,
   R⁹, R¹⁰, R¹¹, R¹², R¹³, R¹⁴, R¹⁵, R¹⁶, R¹⁷, R¹⁸, R¹⁹, R²⁰, R²¹, R²², R²³ and R²⁴ are independently hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G, or a group of formula -(Ar¹)ₒ-(Ar²)ₚ-(Ar³)_{q}-A;
   R²⁵, R²⁶, R²⁷
   are independently hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
   * represents the carbon atoms of the basic sceleton;
   D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C-, E is F; Cl; Br; CN; an unsubstituted C₆-C₂₂aryl group, a C₆-C₂₂aryl group, which is substituted by F, C₁-C₁₈alkyl, C₁-C₁₈alkyl which is interrupted by O; an unsubstituted heteroaryl group having 5 to 22 ring atoms, a heteroaryl group having 5 to 22 ring atoms, which is substituted by F, -OR⁶⁹, -SR⁶⁹, -NR⁶⁵R⁶⁶, -COR⁶⁸, -COOR⁶⁷, -CONR⁶⁵R⁶⁶, -CN, or F;
   G is E, -OR⁶⁹; -SR⁶⁹; -NR⁶⁵R⁶⁶;
   R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-;
   R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
   R⁶⁵ and R⁶⁶ together form a five or six membered ring,
   R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
   R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
   R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
   R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
   R⁷² is a C₁-C₁₈alkyl group, a C₆-C₁₈aryl group, or a C₆-C₁₈aryl group, which is substituted by C₁-C₁₈alkyl;
   Ar¹, Ar², Ar³ are are independently of each other a C₆-C₂₄arylen group, which can optionally be substituted by G, or a C₂-C₃₀heteroarylen group, which can optionally be substituted by G;
   A is hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
   o is 0, or 1;
   p is 0, or 1; and
   q is 0, or 1.

The organic electronic device, preferably the OLED, according to the present invention is characterized by a superior property profile which is especially manifested in a high efficiency and a prolonged lifetime as well as a low driving voltage. The benzimidazolodiazocines of formula (I) are especially characterized by high triplet energies.

In the definition of R¹ and R² and/or R³ and R⁴ an/or R⁵ and R⁶ and/or R⁷ and R⁸ "5- or 6-membered (hetero)aromatic ring" means "5- or 6-membered aromatic ring" or "5- or 6-membered heteroaromatic ring". The hetero atoms of the 5- or 6-membered heteroaromatic ring are preferably selected from N, S and O. Preferred 5- or 6-membered (hetero)aromatic rings are mentioned below.

The "5- or 6-membered aromatic ring" represents an aromatic ring system. Aromatic rings may be present as a single ring system or as bicyclic or tricyclic ring systems; preferably as single ring system or as benz-annelated ring system, more preferably as single ring system. Examples of aromates, from which the 5- or 6-membered aromatic rings are derived, are: benzene and naphthalene.

The "5- or 6-membered heteroaromatic ring" represents an aromatic ring system containing at least one hetero atom. The heteroaromatic rings consist of 5, or 6 ring atoms of which 1-3 ring atoms are hetero atoms. The heteroaromatic rings may be present as a single ring system or as bicyclic or tricyclic ring systems; preferably as single ring system or as benz-annelated ring system, more preferably as single ring system. Bicyclic or tricyclic ring systems may be formed by annelation of two or more rings. Examples of heteroaromates, from which the 5- or 6-membered heteroaromatic rings are derived, are: pyrrole, pyrazole, imidazole, triazole, furane, oxadiazole, thiophene, oxazole, isoxazole, thiazole, isothiazol, thiadiazole, pyridine, pyrazine, pyrimidine, purine, pteridine, and the corresponding benz-annelated heterocycles, e.g. benzofurane, benzothiophene, indole, isoquinoline, quinoline and the like.

The "5- or 6-membered heteroaromatic ring", or the "annelated ring system" may be substituted by one or more substituents G, preferably selected from the group consisting of -F, -C≡N, a C₁-C₂₅alkyl group, which can optionally be substituted by E and or interrupted by D; a C₃-C₂₅cycloalkyl group, which can optionally be substituted by G and/or interrupted by D; a C₆-C₂₂aryl group, preferably a C₆-C₁₄aryl group, which can optionally be substituted by at least one substituent G; or a heteroaryl group comprising 5 to 22, preferably 5 to 16, ring atoms, which can optionally be substituted by at least one substituent G, interrupted by at least one of O, S, N and NR⁶⁵; or by a group of formula -(Ar¹)ₒ-(Ar²)ₚ-(Ar³)_{q}-A.

The residues mentioned in the specification of the present application generally have the following preferred meanings, if not defined differently in a specific residue:
A C₁-C₂₅alkyl group, which can optionally be substituted by at least one substituent E and/or interrupted by D: preferably a C₁-C₁₈alkyl group, which can optionally be substituted by at least one substituent E and/or interrupted by D; more preferably a C₁-C₁₂alkyl group, which can optionally be substituted by at least one substituent E and/or interrupted by D; even more preferably a C₁-C₈alkyl group, which can optionally be substituted by at least one substituent E and/or interrupted by D; most preferably a C₁-C₈alkyl group, which can optionally be substituted by at least one substituent E, or a C₁-C₈alkyl group, which is interrupted by O and can optionally be substituted by at least one substituent E; even more preferably an unsubstituted C₁-C₈alkyl group; further even more preferably an unsubstituted C₁-C₅alkyl group, e.g. methyl, ethyl, propyl, iso-propyl, sec-butyl, iso-butyl, or neopentyl. The alkyl groups may be linear or branched.

A C₃-C₂₅cycloalkyl group, which can optionally be substituted by at least one substituent G and/or interrupted by D: preferably a C₃-C₁₂cycloalkyl group, which can optionally be substituted by at least one substituent G and/or interrupred by D; more preferably a C₃-C₆cycloalkyl group, which can optionally be substituted by at least one substituent G; most preferably an unsubstituted C₃-C₆cycloalkyl group, e.g. cyclohexyl or cyclopentyl.

A C₆-C₂₂aryl group, preferably a C₆-C₁₄aryl group, which can optionally be substituted by at least one substituent G: preferably a C₆-C₁₄aryl group, which can optionally be substituted by one or two groups G; more preferably a phenyl group, which can optionally be substituted by one or two groups G. Examples are phenyl, 4-methylphenyl, 4-methoxyphenyl, 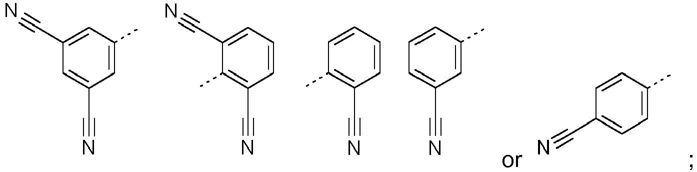 naphthyl, especially 1-naphthyl, or 2-naphthyl, biphenylyl, terphenylyl, pyrenyl, 2- or 9-fluorenyl, phenanthryl, anthryl, or triphenylenyl (especially triphenylen-2-yl), which may be unsubstituted or substituted by at least one substituent G, wherein G is preferably selected from a C₁-C₅alkyl group, a C₃-C₆cycloalkyl group and a C₁-C₄fluoroalkyl group.

A heteroaryl group comprising 5 to 22, preferably 5 to 16, ring atoms, which can optionally be substituted by at least one substituent G, interrupted by at least one of O, S, N and NR⁶⁵: preferably a heteroaryl group comprising 5 to 16 ring atoms, which can optionally be substituted by one or two groups G, interrupted by at least one of O, S, N and NR⁶⁵. Examples are 9H-pyrido[2,3-b]indolyl, benzofuro[2,3-b]pyridyl, benzothiopheno[2,3-b]pyridyl, 9H-pyrido[2,3-c]indolyl, benzofuro[2,3-c]pyridyl, benzothiopheno[2,3-c]pyridyl, furo[3,2-b:4,5-b']dipyridyl, pyrrolo[3,2-b:4,5-b']dipyridyl, thieno[3,2-b:4,5-b']dipyridyl, thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, benzimidazo[1,2-a]benzimidazo-5-yl, benzimidazo[1,2-a]benzimidazo-2-yl, benzimidazolo[2,1-b][1,3]benzothiazolyl, carbazolyl, 9-phenylcarbazolyl, azabenzimidazo[1,2-a]benzimidazolyl, benzimidazolyl, benzothiazolyl or phenoxazinyl, which can be unsubstituted or substituted by at least one substituent G, wherein G is preferably selected from a C₁-C₅alkyl group, a C₃-C₆cycloalkyl group and a C₁-C₄fluoroalkyl group. More preferably pyridyl, methylpyridyl, pyrimidinyl, pyrazinyl, carbazolyl, 9-phenylcarbazolyl, dibenzofuranyl, dibenzothiophenyl, indolyl, methylindolyl, benzofuranyl, benzothiophenyl, benzimidazolyl, benzothiazolyl, benzimidazo[1,2-a]benzimidazo-5-yl, benzimidazo[1,2-a]benzimidazo-2-yl, benzimidazolo[2,1-b][1,3]benzothiazolyl orazabenzimidazo[1,2-a]benzimidazolyl, which can optionally be substituted by one, or more groups selected from a phenyl, C₁-C₅alkyl group, a C₃-C₆cycloalkyl group, a C₁-C₄fluoroalkyl group, carbazolyl, benzimidazo[1,2-a]benzimidazo-5-yl, benzimidazo[1,2-a]benzimidazo-2-yl, benzimidazolo[2,1-b][1,3]benzothiazolyl and azabenzimidazo[1,2-a]benzimidazolyl;
especially 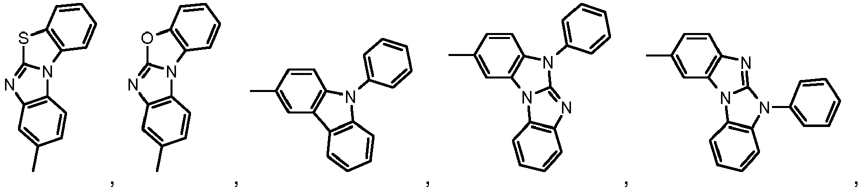 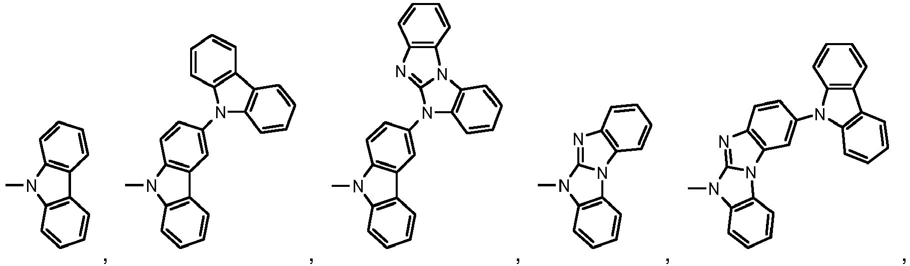 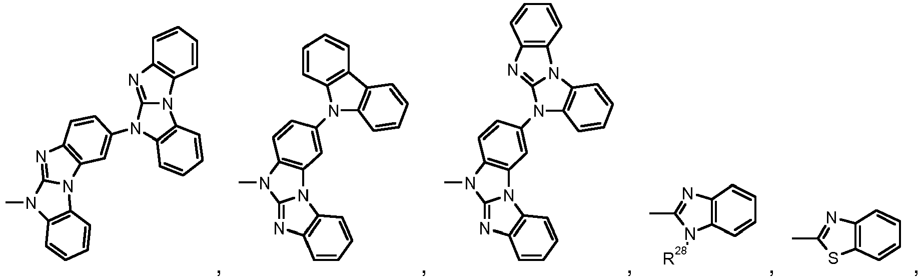 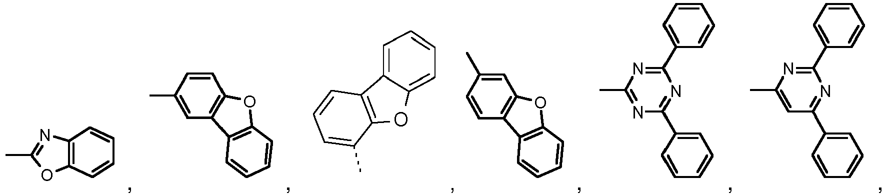 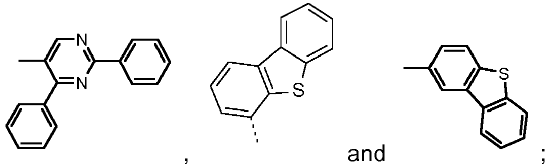 R²⁸ is hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
preferably hydrogen; C₁-C₁₈alkyl, such as methyl, ethyl, n-propyl, iso-propyl, n-butyl, isobutyl, sec-butyl, hexyl, octyl, or 2-ethyl-hexyl; a phenyl group, which can optionally be substituted by one or two groups G, such as phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, or triphenylenyl (especially triphenylen-2-yl), which may be unsubstituted or substituted by at least one substituent G, wherein G is preferably selected from a C₁-C₅alkyl group, a C₃-C₆cycloalkyl group and a C₁-C₄fluoroalkyl group.

Halogen: F, Cl, Br, I, preferably F, Br or Cl, more preferably F.

A C₁-C₁₈alkoxy group: preferably a C₁-C₄alkoxy group, more preferably methoxy or ethoxy. The alkyl groups may be linear or branched.

A C₁-C₁₈haloalkyl group; preferably a fluoroC₁-C₄alkyl group, more preferably CF₃. The alkyl groups may be linear or branched.

D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C, preferably -CO-, -COO-, -S-, -SO-, -SO₂-, -O-, -NR⁶⁵-, wherein R⁶⁵ is C₁-C₁₈alkyl, such as methyl, ethyl, n-propyl, iso-propyl, n-butyl, isobutyl, or sec-butyl, or C₆-C₁₄aryl, such as phenyl, tolyl, naphthyl, or biphenylyl, or heteroaryl having 5 to 22 ring atoms, such as, for example, benzimidazo[1,2-a]benzimidazo-2-yl 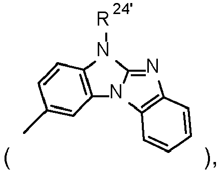 carbazolyl, dibenzofuranyl, which can be unsubstituted or substituted especially by C₆-C₁₀aryl, or C₆-C₁₀aryl, which is substituted by C₁-C₄alkyl; or heteroary having 5 to 16 ring atoms.

E is F; Cl; Br; CN; an unsubstituted C₆-C₂₂aryl group, a C₆-C₂₂aryl group, which is substituted by F, C₁-C₁₈alkyl, C₁-C₁₈alkyl which is interrupted by O; an unsubstituted heteroaryl group having 5 to 22 ring atoms, a heteroaryl group having 5 to 22 ring atoms, which is substituted by F, -OR⁶⁹, -SR⁶⁹, -NR⁶⁵R⁶⁶, -COR⁶⁸, -COOR⁶⁷, -CONR⁶⁵R⁶⁶, -CN, or F; preferably an unsubstituted C₆-C₁₈aryl group, a C₆-C₁₈aryl group, which is substituted by F, or C₁-C₁₈alkyl; an unsubstituted heteroaryl group having 5 to 16 ring atoms, or a heteroaryl group having 5 to 16 ring atoms, which is substituted by F, or C₁-C₁₈alkyl, -OR⁶⁹; -SR⁶⁹; -NR⁶⁵R⁶⁶; -COR⁶⁷; -COOR⁶⁷; -CONR⁶⁵R⁶⁶; or -CN; wherein R⁶⁵, R⁶⁶, R⁶⁷ and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl, or C₆-C₁₄aryl, such as phenyl, tolyl, naphthyl, or biphenylyl. More preferably, E is a C₆-C₁₄aryl group, a C₆-C₁₄aryl group, which is substituted by F, or C₁-C₁₈alkyl; 9H-pyrido[2,3-b]indolyl, benzofuro[2,3-b]pyridyl, benzothiopheno[2,3-b]pyridyl, 9H-pyrido[2,3-c]indolyl, benzofuro[2,3-c]pyridyl, benzothiopheno[2,3-c]pyridyl, furo[3,2-b:4,5-b']dipyridyl, pyrrolo[3,2-b:4,5-b']dipyridyl, thieno[3,2-b:4,5-b']dipyridyl, thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, benzimidazo[1,2-a]benzimidazo-5-yl, benzimidazo[1,2-a]benzimidazo-2-yl, benzimidazolo[2,1-b][1,3]benzothiazolyl, carbazolyl, 9-phenylcarbazolyl, azabenzimidazo[1,2-a]benzimidazolyl, benzimidazolyl, benzothiazolyl or phenoxazinyl, which can be unsubstituted or substituted by at least one substituent G, wherein G is preferably selected from a C₁-C₅alkyl group, a C₃-C₆cycloalkyl group and a C₁-C₄fluoroalkyl group. Even more preferably, E is a C₆-C₁₄aryl group, a C₆-C₁₄aryl group, which is substituted by F, or C₁-C₁₈alkyl; pyridyl, methylpyridyl, pyrimidinyl, pyrazinyl, carbazolyl, 9-phenylcarbazolyl, dibenzofuranyl, dibenzothiophenyl, indolyl, methylindolyl, benzofuranyl, benzothiophenyl, benzimidazolyl, benzothiazolyl, benzimidazo[1,2-a]benzimidazo-5-yl, benzimidazo[1,2-a]benzimidazo-2-yl, benzimidazolo[2,1-b][1,3]benzothiazolyl or azabenzimidazo[1,2-a]benzimidazolyl, which can optionally be substituted by one, or more groups selected from a phenyl, C₁-C₅alkyl group, a C₃-C₆cycloalkyl group, a C₁-C₄fluoroalkyl group, carbazolyl, benzimidazo[1,2-a]benzimidazo-5-yl, benzimidazo[1,2-a]benzimidazo-2-yl, benzimidazolo[2,1-b][1,3]benzothiazolyl and azabenzimidazo[1,2-a]benzimidazolyl.

Most preferably, E is phenyl, tolyl, naphthyl, or biphenylyl, phenyl, tolyl, naphthyl, or biphenylyl which is each substituted by F, or C₁-C₁₈alkyl; 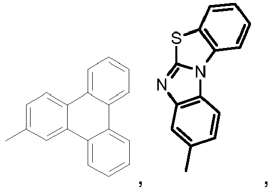 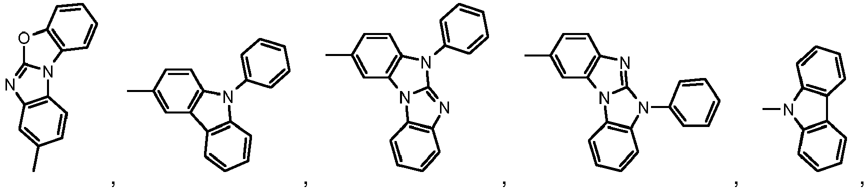 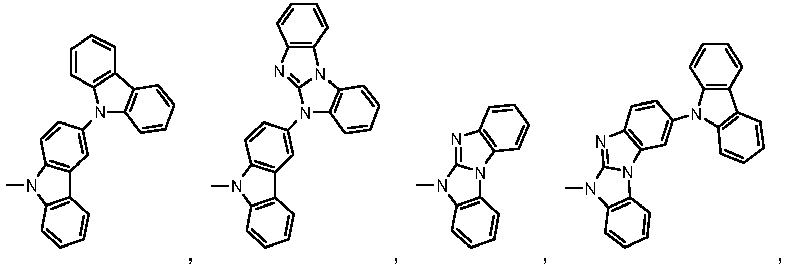 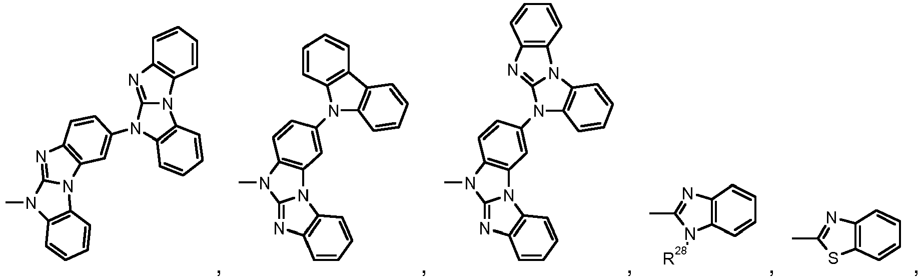 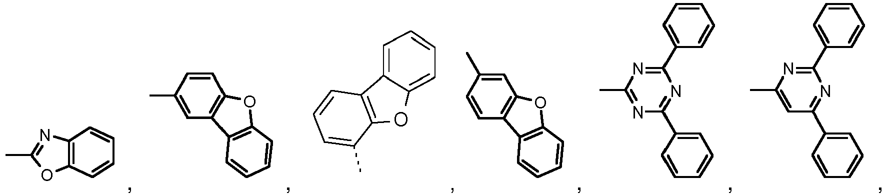 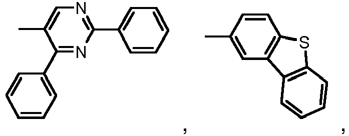 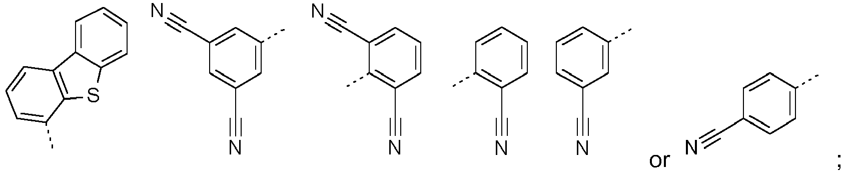 R²⁸ is hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
preferably hydrogen; C₁-C₁₈alkyl, such as methyl, ethyl, n-propyl, iso-propyl, n-butyl, isobutyl, sec-butyl, hexyl, octyl, or 2-ethyl-hexyl; a phenyl group, which can optionally be substituted by one or two groups G, such as phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, or triphenylenyl (especially triphenylen-2-yl), which may be unsubstituted or substituted by at least one substituent G, wherein G is preferably selected from a C₁-C₅alkyl group, a C₃-C₆cycloalkyl group and a C₁-C₄fluoroalkyl group.

G is preferably E or-OR⁶⁹; -SR⁶⁹; -NR⁶⁵R⁶⁶; wherein R⁶⁵, R⁶⁶, and R⁶⁹ are independently of each other C₁-C₁₈alkyl, such as methyl, ethyl, n-propyl, iso-propyl, n-butyl, isobutyl, sec-butyl, hexyl, octyl, or 2-ethyl-hexyl.

A is is hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
preferably, A is selected from the group consisting of H, CN, 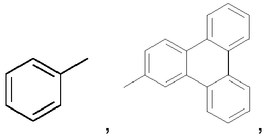 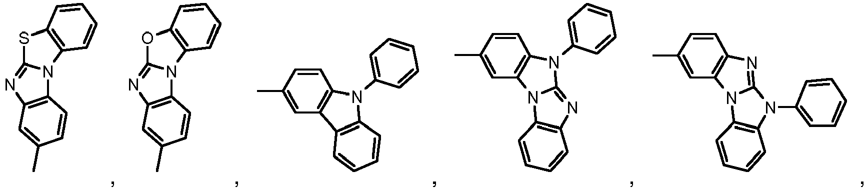 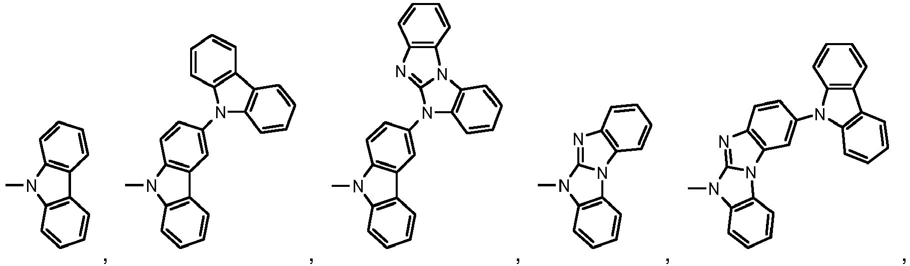 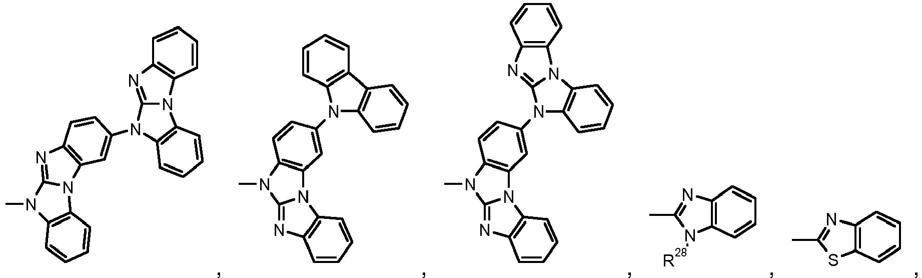 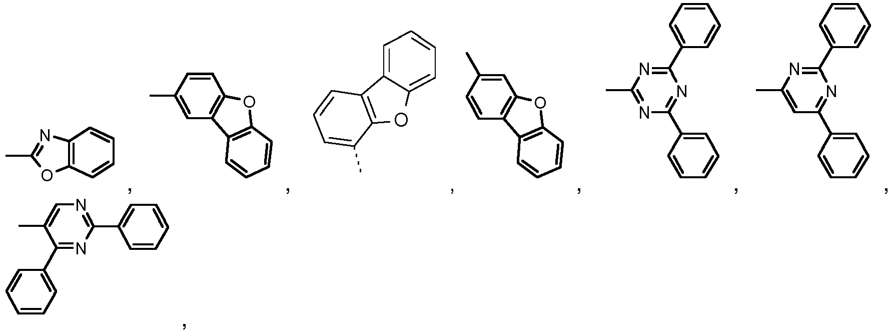 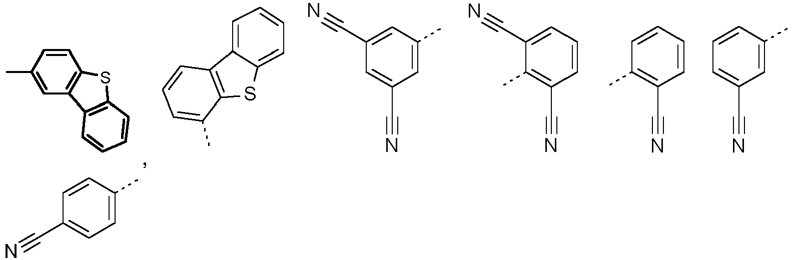 and
wherein
R²⁸ is hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
more preferably, A is selected from the group consisting of H, CN, 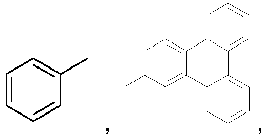 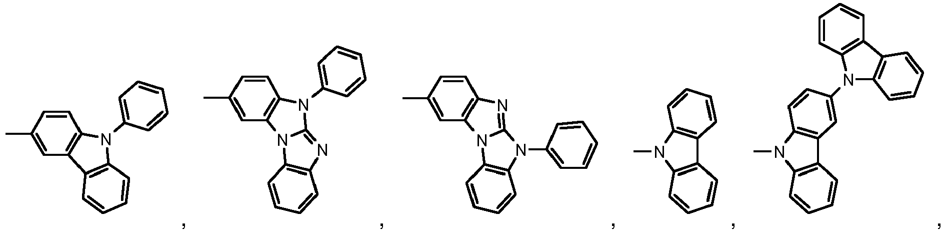 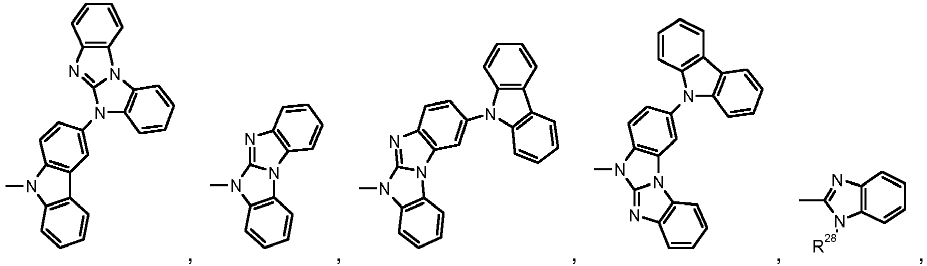 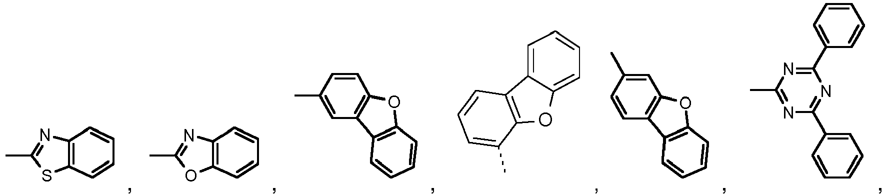 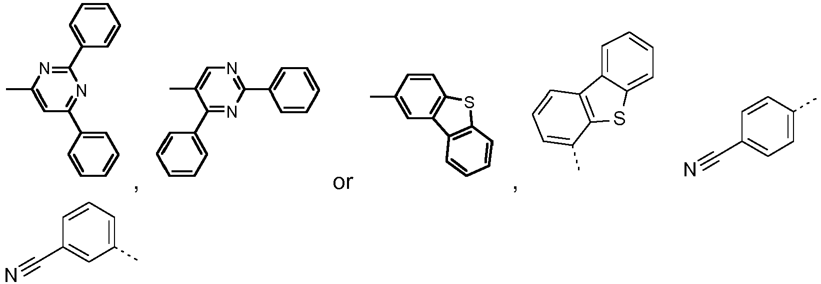 and
wherein
R²⁸ is hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G.

Ar¹, Ar² and Ar³ are independently of each other a group of formula 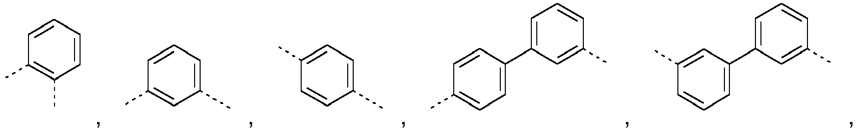 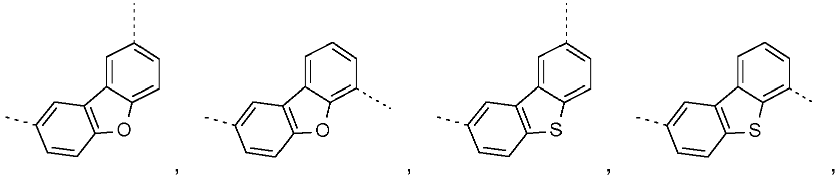 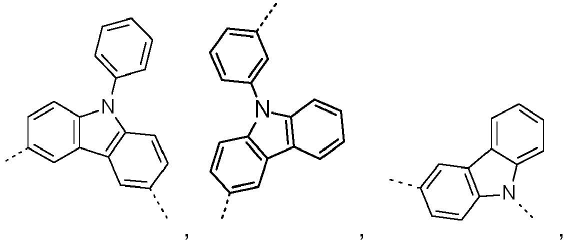 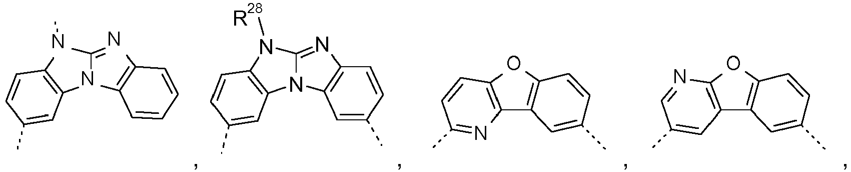 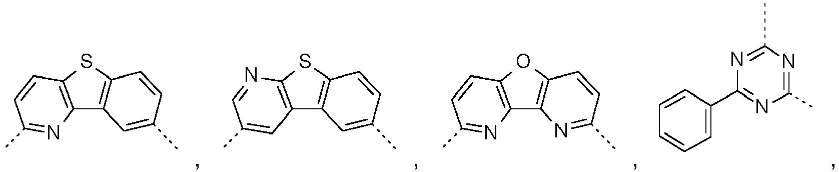 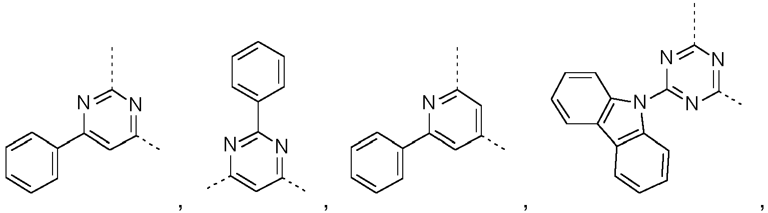 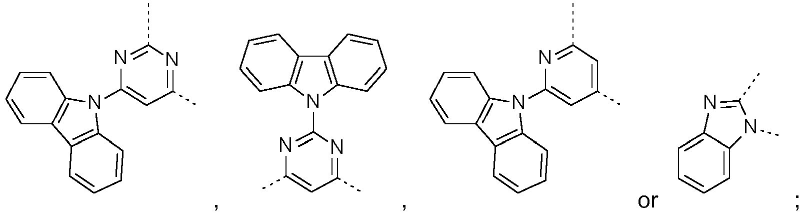 wherein
R²⁸ is hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
preferably, Ar¹, Ar² and Ar³ are independently of each other a group of formula 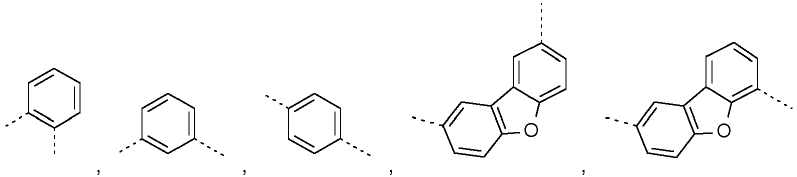 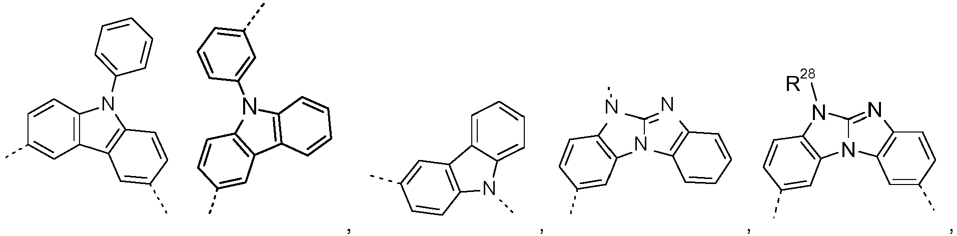 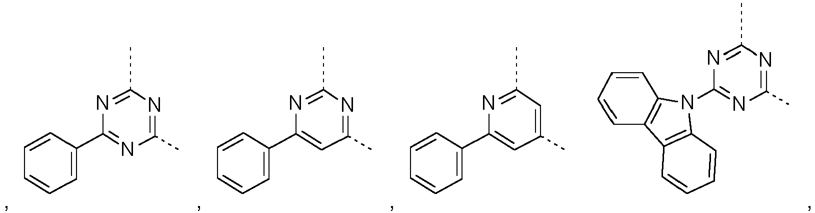 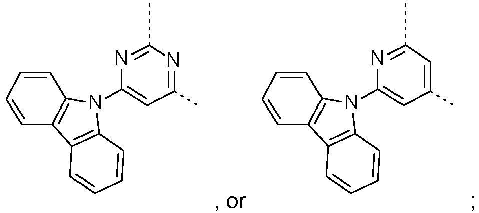 more preferably, Ar¹, Ar² and Ar³ are independently of each other a group of formula

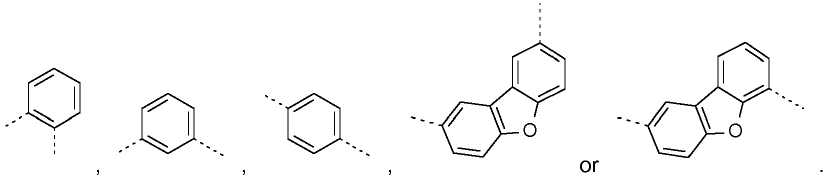

In the alkyl groups and aryl groups mentioned in the present application one or more hydrogen atoms may be substituted by deuterium atoms.

If a substituent occurs more than one time in a group, it can be different in each occurrence.

Benzimidazolodiazocines of formula (I)

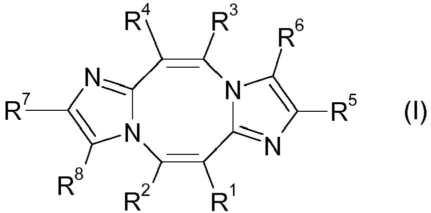

In the benzimidazolodiazocines of formula (I),
R¹, R², R³, R⁴, R⁵, R⁶, R⁷ and R⁸
are independently hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
preferably hydrogen, F, Cl, Br, a C₁-C₈alkyl group, which is interrupted by O and can optionally be substituted by at least one substituent E; a C₃-C₆cycloalkyl group, which can optionally be substituted by at least one substituent G; a C₆-C₁₄aryl group, which can optionally be substituted by one or two groups G; a heteroaryl group comprising 5 to 16 ring atoms, which can optionally be substituted by at least one group G, interrupted by at least one of O, S, N and NR⁶⁵;
more preferably an unsubstituted C₁-C₈alkyl group; an unsubstituted C₃-C₆cycloalkyl group; a phenyl group, which can optionally be substituted by one or two groups G; a heteroaryl group comprising 5 to 16 ring atoms, which can optionally be substituted by one or two groups G, interrupted by at least one of O, S, N and NR⁶⁵;
most preferably methyl, ethyl, propyl, iso-propyl, sec-butyl, iso-butyl, or neopentyl; cyclohexyl or cyclopentyl; phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, or triphenylenyl (especially triphenylen-2-yl), which may be unsubstituted or substituted by at least one substituent selected from a C₁-C₅alkyl group, a C₃-C₆cycloalkyl group and a C₁-C₄fluoroalkyl group; 9H-pyrido[2,3-b]indolyl, benzofuro[2,3-b]pyridyl, benzothiopheno[2,3-b]pyridyl, 9H-pyrido[2,3-c]indolyl, benzofuro[2,3-c]pyridyl, benzothiopheno[2,3-c]pyridyl, furo[3,2-b:4,5-b']dipyridyl, pyrrolo[3,2-b:4,5-b']dipyridyl, thieno[3,2-b:4,5-b']dipyridyl, thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, benzimidazo[1,2-a]benzimidazo-5-yl, benzimidazo[1,2-a]benzimidazo-2-yl, benzimidazolo[2,1-b][1,3]benzothiazolyl, carbazolyl, 9-phenylcarbazolyl, azabenzimidazo[1,2-a]benzimidazolyl, benzimidazolyl, benzothiazolyl or phenoxazinyl, which can be unsubstituted or substituted by at least one substituent G, wherein G is preferably selected from a C₁-C₅alkyl group, a C₃-C₆cycloalkyl group and a C₁-C₄fluoroalkyl group;
or
R¹ and R² and/or R³ and R⁴ an/or R⁵ and R⁶ and/or R⁷ and R⁸
independently of each other form together with the carbon atoms to which they are bonded a 5-or 6-membered (hetero)aromatic ring, which can optionally be part of an annelated ring system, wherein the (hetero)aromatic ring and/or annelated ring system can optionally be substituted by G or by a group of formula -(Ar¹)ₒ-(Ar²)ₚ-(Ar³)_{q}-A; and the heteroatoms of the 5- or 6-membered heteroaromatic ring are selected from N, S and O.

Preferably, the 5- or 6-membered (hetero)aromatic ring formed by R¹ and R² and/or R³ and R⁴ together with the carbon atoms to which they are bonded is independently selected from 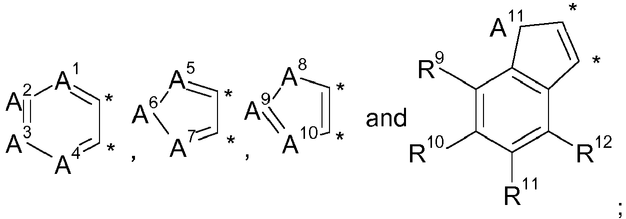 preferably, the 5- or 6-membered (hetero)aromatic ring formed by R⁵ and R⁶ and/or R⁷ and R⁸ together with the carbon atoms to which they are bonded is independently selected from 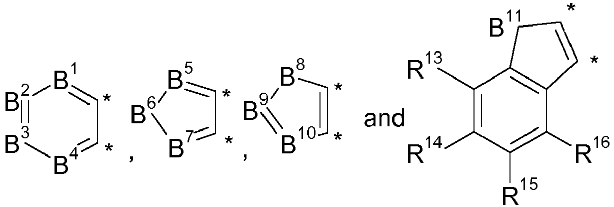 wherein
A¹ and B¹ are independently N, or CR¹⁷,
A² and B² are independently N, or CR¹⁸,
A³ and B³ are independently N, or CR¹⁹,
A⁴ and B⁴ are independently N, or CR²⁰,
A⁵ and B⁵ are independently N, or CR²¹,
A⁶ and B⁶ are independently O, S, NR²⁵ or CR²⁶R²⁷,
A⁷ and B⁷ are independently N, or CR²²,
A⁸ and B⁸ are independently O, S, NR²⁵ or CR²⁶R²⁷,
A⁹ and B⁹ are independently N, or CR²³,
A¹⁰ and B¹⁰ are independently N, or CR²⁴,
A¹¹ and B¹¹ are independently O, S, NR²⁵ or CR²⁶R²⁷,
wherein R⁹, R¹⁰, R¹¹, R¹², R¹³, R¹⁴, R¹⁵, R¹⁶, R¹⁷, R¹⁸, R¹⁹, R²⁰, R²¹, R²², R²³ and R²⁴ are defined above and preferred embodiments of R⁹, R¹⁰, R¹¹, R¹², R¹³, R¹⁴, R¹⁵, R¹⁶, R¹⁷, R¹⁸, R¹⁹, R²⁰, R²¹, R²², R²³ and R²⁴ are defined below.

Preferably, 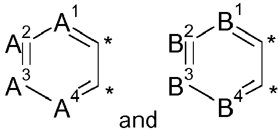 are independently selected from 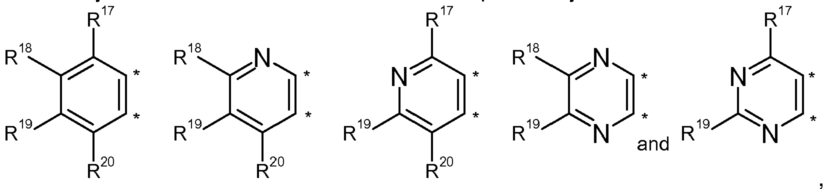 more preferably

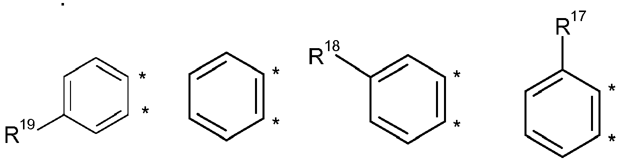

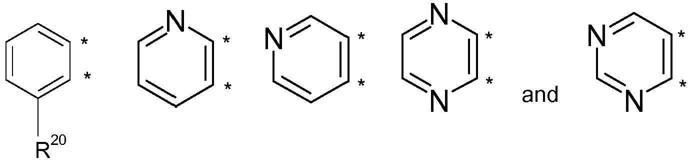

Preferably, 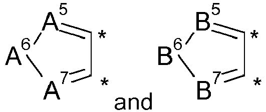 are independently selected from 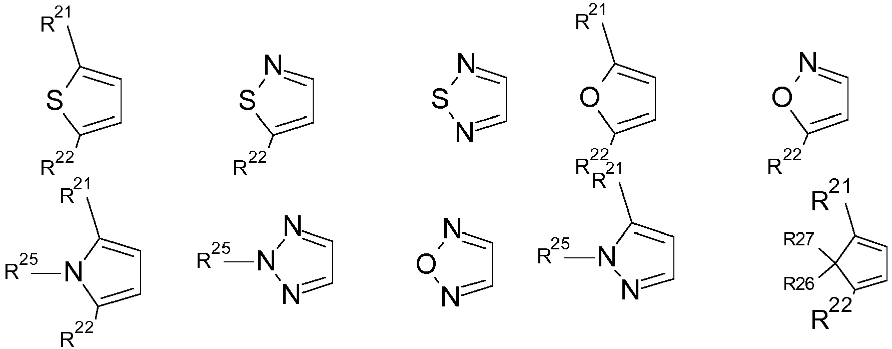 more preferably

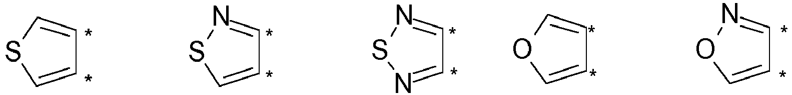

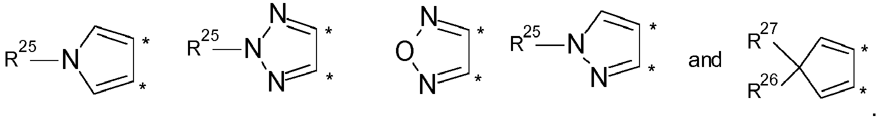

Preferably, 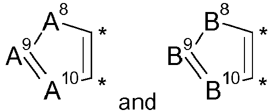 are independently selected from 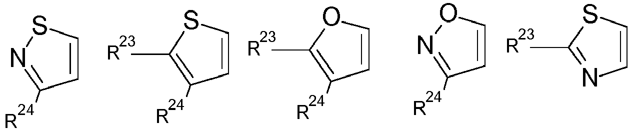 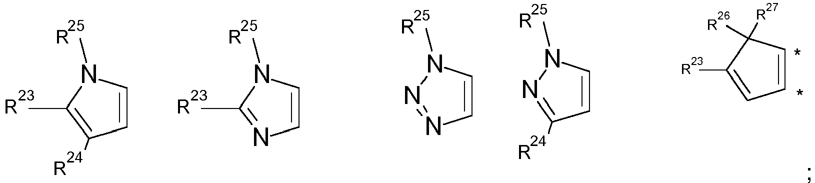 more preferably

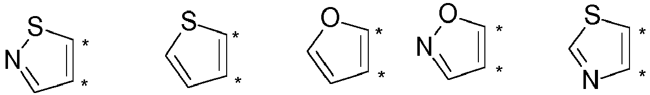

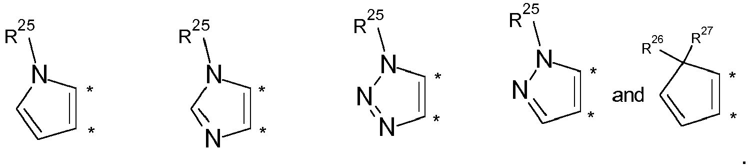

Preferably, 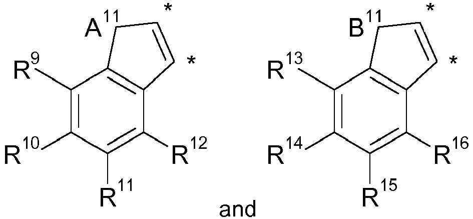 are independently selected from 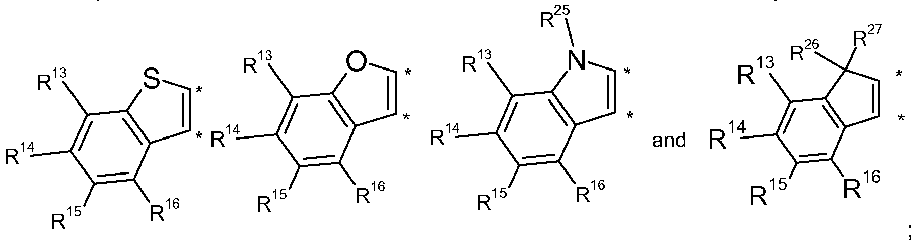 more preferably ; 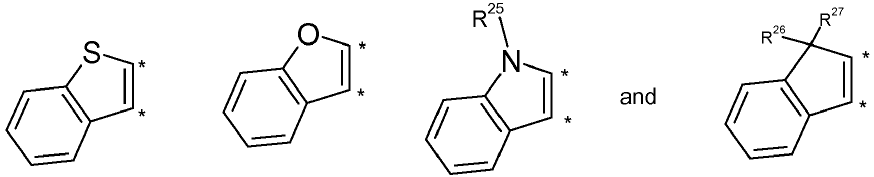 wherein
* represents the carbon atoms of the basic skeleton.

R⁹, R¹⁰, R¹¹, R¹², R¹³, R¹⁴, R¹⁵, R¹⁶, R¹⁷, R¹⁸, R¹⁹, R²⁰, R²¹, R²², R²³ and R²⁴ are independently hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G, or a group of formula -(Ar¹)ₒ-(Ar²)ₚ-(Ar³)_{q}-A;
and
R²⁵, R²⁶, R²⁷
are independently hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G.

Preferably, R⁹, R¹⁰, R¹¹, R¹², R¹³, R¹⁴, R¹⁵, R¹⁶, R¹⁷, R¹⁸, R¹⁹, R²⁰, R²¹, R²², R²³ and R²⁴
are independently hydrogen, F, Cl, Br, a C₁-C₈alkyl group, which is interrupted by O and can optionally be substituted by at least one substituent E; a C₃-C₆cycloalkyl group, which can optionally be substituted by at least one substituent G; a C₆-C₁₄aryl group, which can optionally be substituted by one or two groups G; a heteroaryl group comprising 5 to 16 ring atoms, which can optionally be substituted by at least one group G, interrupted by at least one of O, S, N and NR⁶⁵; or a group of formula -(Ar¹)ₒ-(Ar²)ₚ-(Ar³)_{q}-A;
more preferably an unsubstituted C₁-C₈alkyl group; an unsubstituted C₃-C₆cycloalkyl group; a phenyl group, which can optionally be substituted by one or two groups G; a heteroaryl group comprising 5 to 16 ring atoms, which can optionally be substituted by one or two groups G, interrupted by at least one of O, S, N and NR⁶⁵; or a group of formula -(Ar¹)ₒ-(Ar²)ₚ-(Ar³)_{q}-A; most preferably methyl, ethyl, propyl, iso-propyl, sec-butyl, iso-butyl, or neopentyl; cyclohexyl or cyclopentyl; phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, or triphenylenyl (especially triphenylen-2-yl), which may be unsubstituted or substituted by at least one substituent selected from a C₁-C₅alkyl group, a C₃-C₆cycloalkyl group and a C₁-C₄fluoroalkyl group; 9H-pyrido[2,3-b]indolyl, benzofuro[2,3-b]pyridyl, benzothiopheno[2,3-b]pyridyl, 9H-pyrido[2,3-c]indolyl, benzofuro[2,3-c]pyridyl, benzothiopheno[2,3-c]pyridyl, furo[3,2-b:4,5-b']dipyridyl, pyrrolo[3,2-b:4,5-b']dipyridyl, thieno[3,2-b:4,5-b']dipyridyl, thienyl, benzothiophenyl, dibenzothiophenyl, thianthrenyl, furyl, furfuryl, 2H-pyranyl, benzofuranyl, isobenzofuranyl, dibenzofuranyl, phenoxythienyl, pyrrolyl, imidazolyl, pyrazolyl, pyridyl, bipyridyl, triazinyl, pyrazinyl, pyridazinyl, indolizinyl, isoindolyl, indolyl, indazolyl, purinyl, quinolizinyl, chinolyl, isochinolyl, phthalazinyl, naphthyridinyl, chinoxalinyl, chinazolinyl, cinnolinyl, pteridinyl, carbolinyl, benzotriazolyl, benzoxazolyl, phenanthridinyl, acridinyl, pyrimidinyl, phenanthrolinyl, phenazinyl, isothiazolyl, phenothiazinyl, isoxazolyl, furazanyl, benzimidazo[1,2-a]benzimidazo-5-yl, benzimidazo[1,2-a]benzimidazo-2-yl, benzimidazolo[2,1-b][1,3]benzothiazolyl, carbazolyl, 9-phenylcarbazolyl, azabenzimidazo[1,2-a]benzimidazolyl, benzimidazolyl, benzothiazolyl or phenoxazinyl, which can be unsubstituted or substituted by at least one substituent G, wherein G is preferably selected from a C₁-C₅alkyl group, a C₃-C₆cycloalkyl group and a C₁-C₄fluoroalkyl 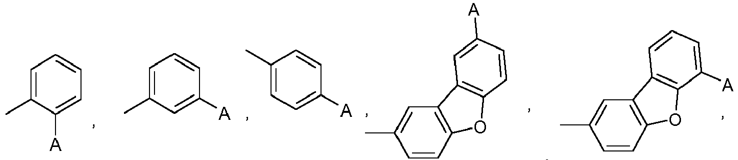 group; or 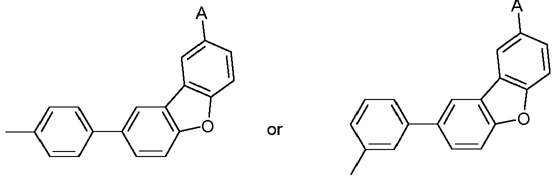 even more preferably phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, or triphenylenyl (especially triphenylen-2-yl), which may be unsubstituted or substituted by at least one substituent selected from a C₁-C₅alkyl group, a C₃-C₆cycloalkyl group and a C₁-C₄fluoroalkyl group; pyridyl, methylpyridyl, pyrimidinyl, pyrazinyl, carbazolyl, 9-phenylcarbazolyl, dibenzofuranyl, dibenzothiophenyl, indolyl, methylindolyl, benzofuranyl, benzothiophenyl, benzimidazolyl, benzothiazolyl, benzimidazo[1,2-a]benzimidazo-5-yl, benzimidazo[1,2-a]benzimidazo-2-yl, benzimidazolo[2,1-b][1,3]benzothiazolyl or azabenzimidazo[1,2-a]benzimidazolyl, which can optionally be substituted by one, or more groups selected from a phenyl, C₁-C₅alkyl group, a C₃-C₆cycloalkyl group, a C₁-C₄fluoroalkyl group, carbazolyl, benzimidazo[1,2-a]benzimidazo-5-yl, benzimidazo[1,2-a]benzimidazo-2-yl, benzimidazolo[2,1-b][1,3]benzothiazolyl and azabenzimidazo[1,2-a]benzimidazolyl; or;

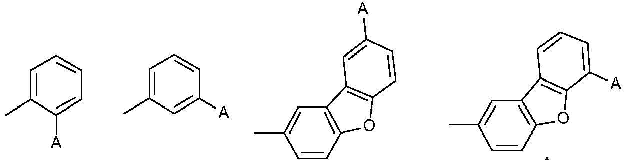

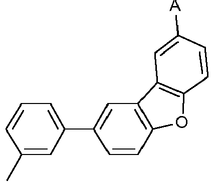

Most preferably phenyl, tolyl, naphthyl, or biphenylyl; phenyl, tolyl, naphthyl, or biphenylyl which is each substituted by F, or C₁-C₁₈alkyl; 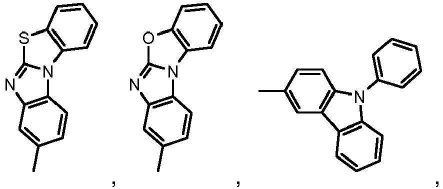 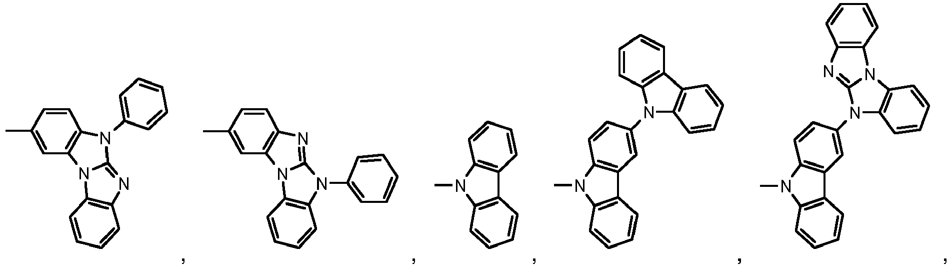 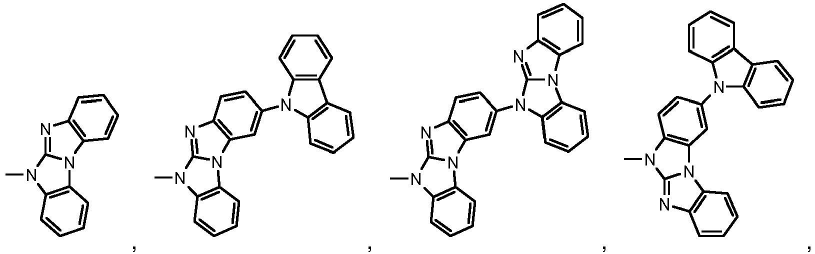 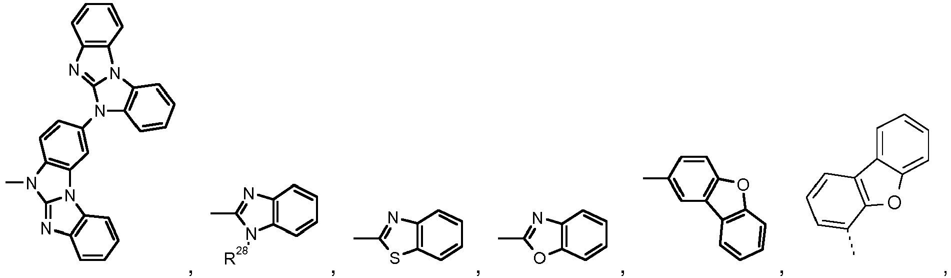 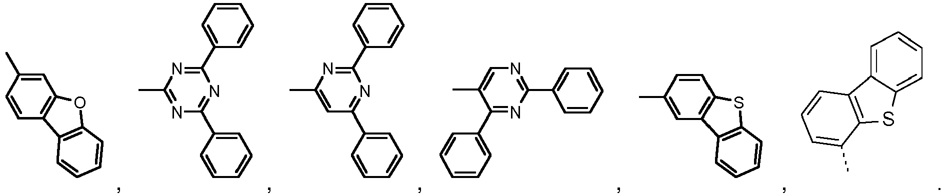 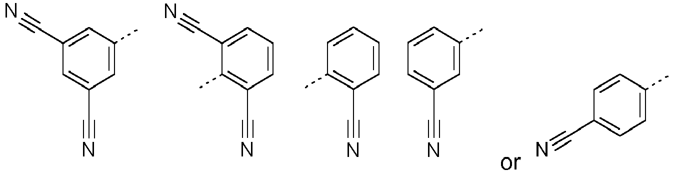 R²⁸ is hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
preferably hydrogen; C₁-C₁₈alkyl, such as methyl, ethyl, n-propyl, iso-propyl, n-butyl, isobutyl, sec-butyl, hexyl, octyl, or 2-ethyl-hexyl; a phenyl group, which can optionally be substituted by one or two groups G, such as phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, or triphenylenyl (especially triphenylen-2-yl), which may be unsubstituted or substituted by at least one substituent G, wherein G is preferably selected from a C₁-C₅alkyl group, a C₃-C₆cycloalkyl group and a C₁-C₄fluoroalkyl group.

Preferably, R²⁵, R²⁶, R²⁷
are independently hydrogen; C₁-C₁₈alkyl, such as methyl, ethyl, n-propyl, iso-propyl, n-butyl, isobutyl, sec-butyl, hexyl, octyl, or 2-ethyl-hexyl; a phenyl group, which can optionally be substituted by one or two groups G, such as phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, or triphenylenyl (especially triphenylen-2-yl), which may be unsubstituted or substituted by at least one substituent G, wherein G is preferably selected from a C₁-C₅alkyl group, a C₃-C₆cycloalkyl group and a C₁-C₄fluoroalkyl group.

In a preferred embodiment of the present invention, the residue R¹ is identical with R⁴ and the residue R² is identical with R³, respectively in the case that R¹ and R² as well as R³ and R⁴ form together with the carbon atoms to which they are bonded a 5- or 6-membered (hetero)aromatic ring as defined above, said (hetero)aromatic rings are identical.

In a further preferred embodiment of the present invention, the residue R⁵ is identical with R⁷ and the residue R⁶ is identical with R⁸, respectively in the case that R⁵ and R⁶ as well as R⁷ and R⁸ form together with the carbon atoms to which they are bonded a 5- or 6-membered (hetero)aromatic ring as defined above, said (hetero)aromatic rings are identical.

In a further preferred embodiment of the present invention, the residue R¹ is identical with R⁴ and the residue R² is identical with R³, and the residue R⁵ is identical with R⁷ and the residue R⁶ is identical with R⁸; respectively in the case that R¹ and R² as well as R³ and R⁴ form together with the carbon atoms to which they are bonded a 5- or 6-membered (hetero)aromatic ring as defined above, said (hetero)aromatic rings are identical; and in the case that R⁵ and R⁶ as well as R⁷ and R⁸ form together with the carbon atoms to which they are bonded a 5- or 6-membered (hetero)aromatic ring as defined above, said (hetero)aromatic rings are identical.

*The group of formula*

-*(Ar¹)*ₒ-*(Ar²)ₚ*-*(Ar³)_{q}*-*A:*

Groups A and Ar¹, Ar² and Ar³ of the group of formula -(Ar¹)ₒ-(Ar²)ₚ-(Ar³)_{q}-A and preferred groups of formula -(Ar¹)ₒ-(Ar²)ₚ-(Ar³)_{q}-A have been defined above.

o, p and q in the group of formula -(Ar¹)ₒ-(Ar²)ₚ-(Ar³)_{q}-A are independently of each othe 0 or 1. Preferably, o and p are independently of each other 0 or 1 and q is 0

Most preferably, the groups and indices in the group of formula -(Ar¹)ₒ-(Ar²)ₚ-(Ar³)_{q}-A have the following meanings:
o, p are independently of each other 0 or 1;
q is 0;
A is phenyl, tolyl, naphthyl, or biphenylyl; phenyl, tolyl, naphthyl, or biphenylyl which is each substituted by F, or C₁-C₁₈alkyl; 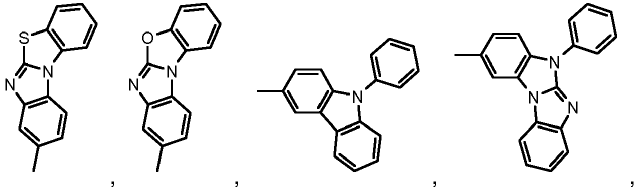 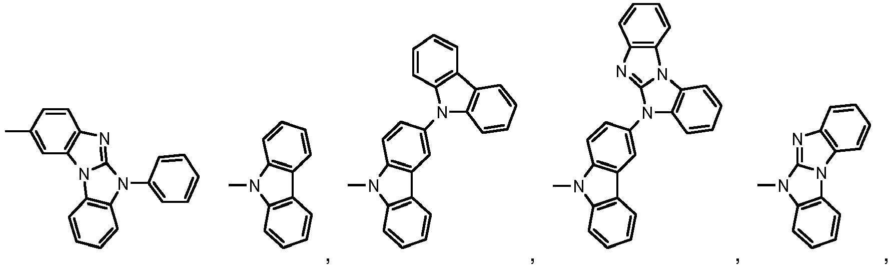 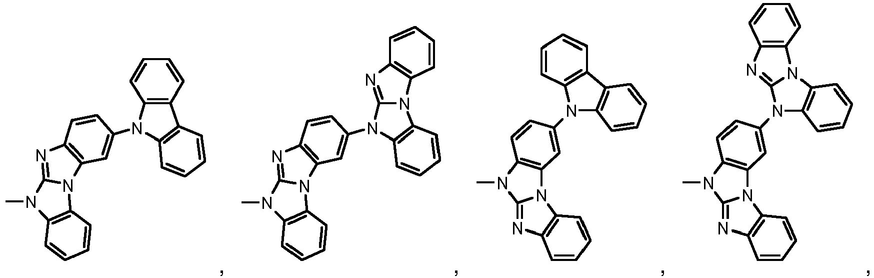 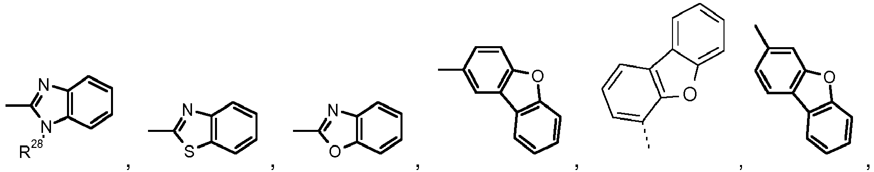 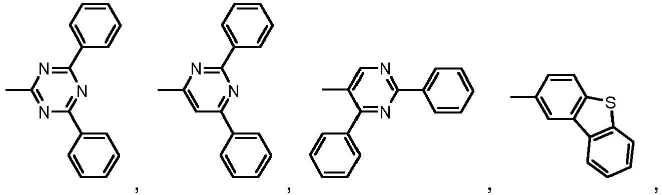 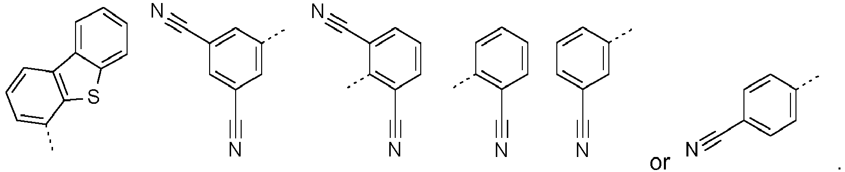
R²⁸ is hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
preferably hydrogen; C₁-C₁₈alkyl, such as methyl, ethyl, n-propyl, iso-propyl, n-butyl, isobutyl, sec-butyl, hexyl, octyl, or 2-ethyl-hexyl; a phenyl group, which can optionally be substituted by one or two groups G, such as phenyl, 4-methylphenyl, 4-methoxyphenyl, naphthyl, especially 1-naphthyl, or 2-naphthyl, biphenylyl, terphenylyl, pyrenyl, 2- or 9-fluorenyl, phenanthryl, or anthryl, or triphenylenyl (especially triphenylen-2-yl), which may be unsubstituted or substituted by at least one substituent G, wherein G is preferably selected from a C₁-C₅alkyl group, a C₃-C₆cycloalkyl group and a C₁-C₄fluoroalkyl group;
Ar¹, Ar² and Ar³ are independently of each other a group of formula 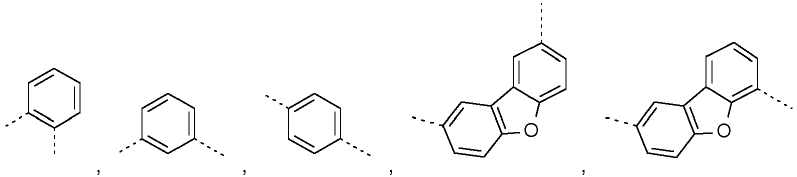 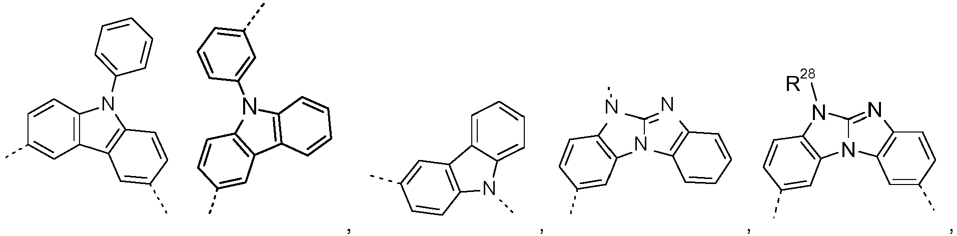 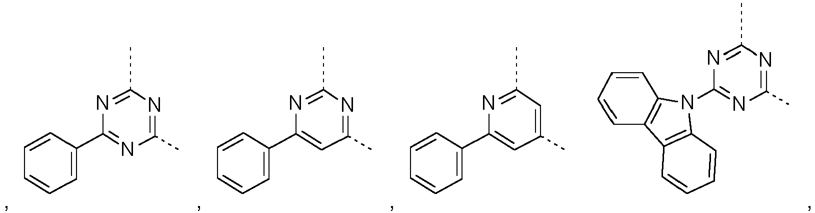 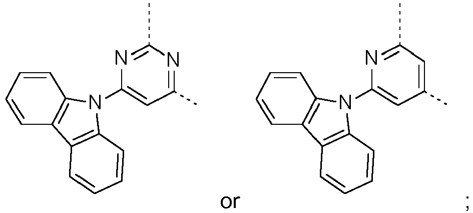
preferably, Ar¹, Ar² and Ar³ are independently of each other a group of formula

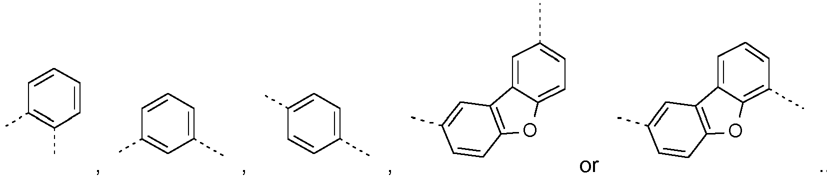

Examples for especially preferred groups -(Ar¹)ₒ-(Ar²)ₚ-(Ar³)_{q}-A are:

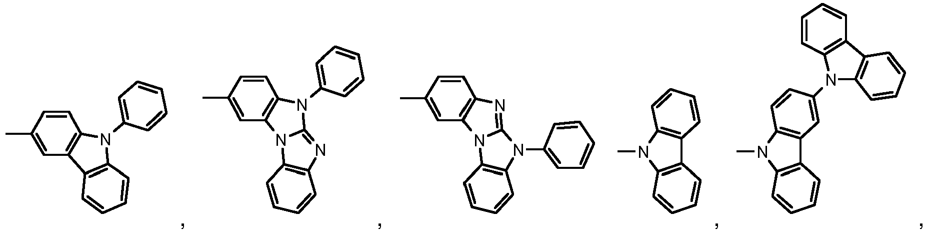

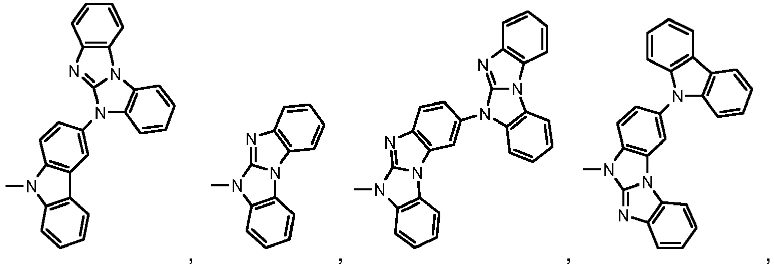

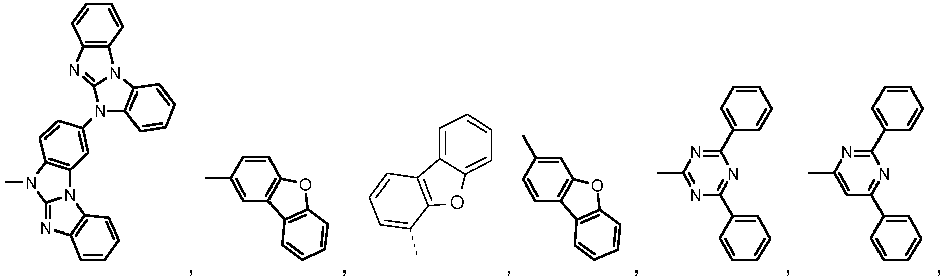

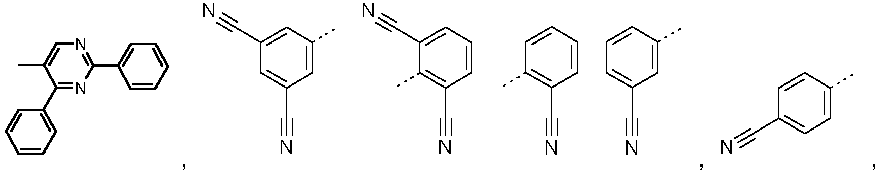

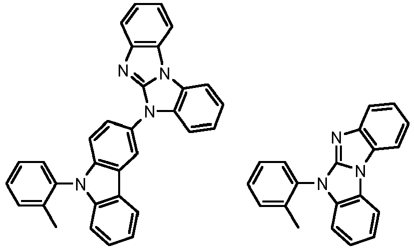

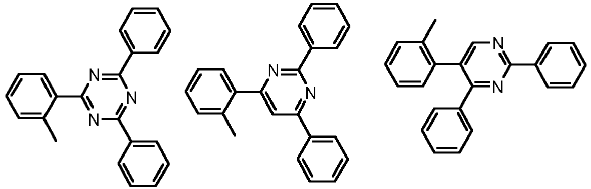

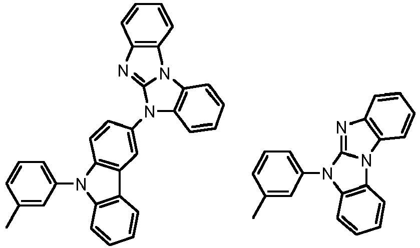

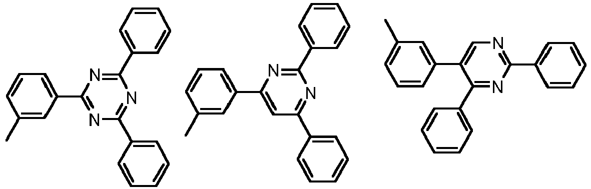

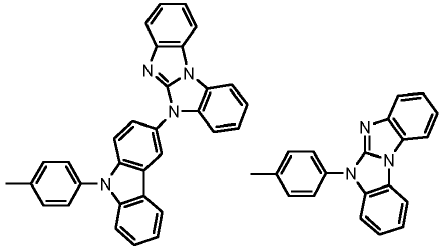

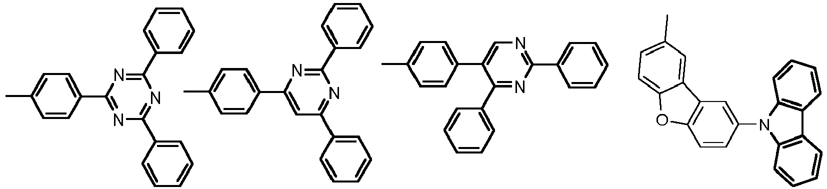

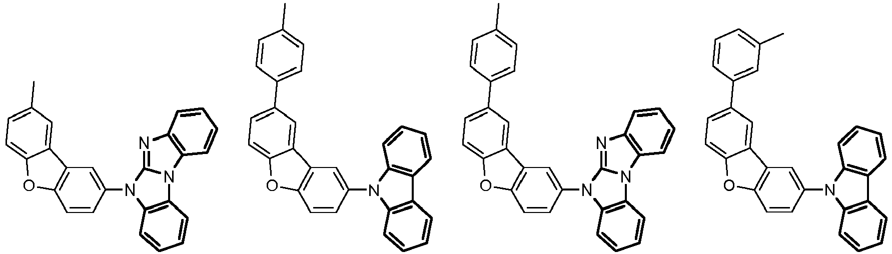

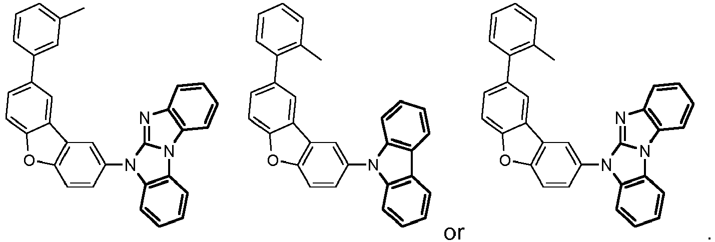

Preferably, the compound of formula (I) is selected from a compound of formula (1.1) and (1.2): 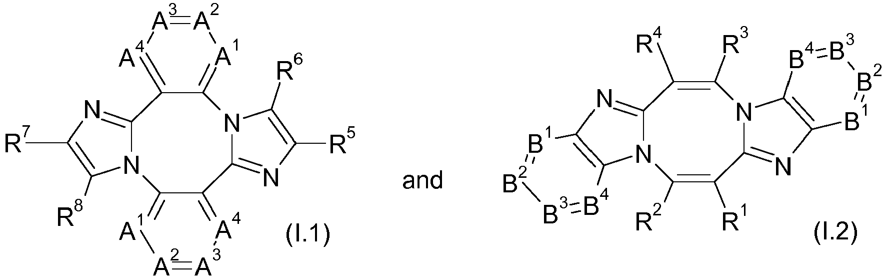 wherein preferred groups A¹, A², A³, A⁴, B1, B², B³ and B⁴ and preferred residues R¹, R², R³, R⁴, R⁵, R⁶, R⁷ and R⁸ are mentioned above; and
wherein each group A¹, A², A³, A⁴, B¹, B², B³ is mentioned more than one time in formulae (I.1) and (1.2) and may be the same or different.

More preferably, the compound of formula (I) is a compound of formula (Ia): 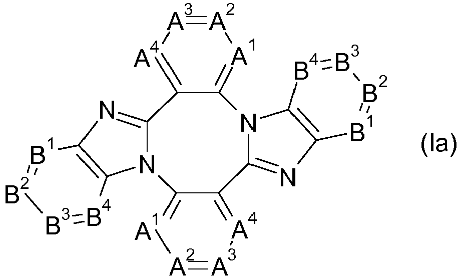 wherein each group A¹, A², A³, A⁴, B¹, B², B³ and B⁴ is mentioned more than one time in formula (Ia) and may be the same or different.

More preferably, the compound of formula (Ia) is selected from the following compounds: 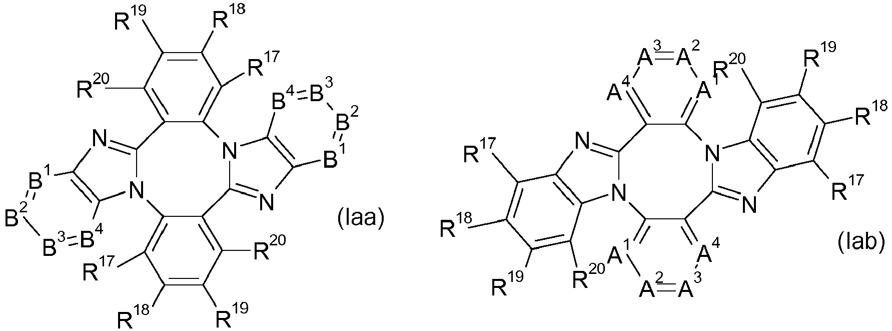 wherein each group A¹, A², A³, A⁴, B¹, B², B³ and B⁴ and each residue R¹⁷, R¹⁸, R¹⁹, R²⁰ is mentioned more than one time in formulae (Iaa) and (lab) and may be the same or different.

Most preferably, the compound of formula (I) is the following compound: 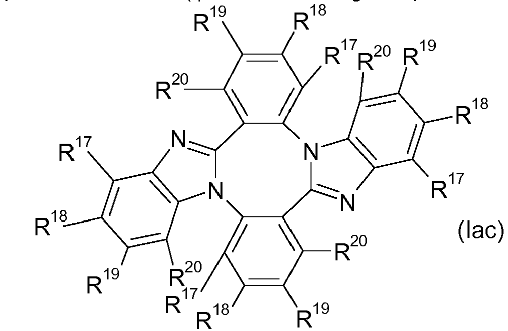 wherein each residue R¹⁷, R¹⁸, R¹⁹, R²⁰ is mentioned more than one time in formula (lac) and may be the same or different.

Especially preferably, the compound of formula (1a) is selected from the following compounds 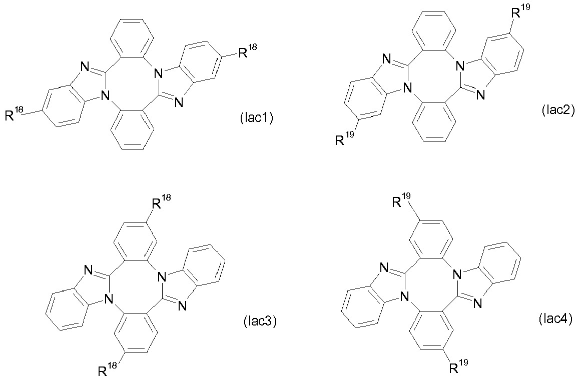 wherein each residue R¹⁸ and R¹⁹ is mentioned more than one time in formulae (Iac1), (Iac2), (Iac3), (Iac4) may be the same or different.

Examples for suitable compounds of formula (I) are: 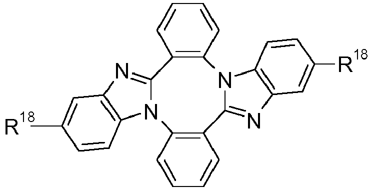 wherein both residues R¹⁸ are identical

| **Compound** | **R¹⁸** |
|---|---|
| **A-1** | H |
| **A-2** | CN |

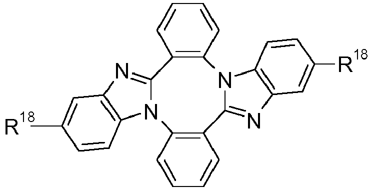

wherein both residues R¹⁸ are identical and are a group of formula -(Ar¹)ₒ-(Ar²)ₚ-(Ar³)_{q}-A :

| Compound | -(Ar¹)ₒ-(Ar²)ₚ-(Ar³)_{q}- | A |
|---|---|---|
| B-1 | - | 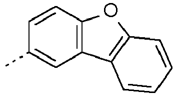 |
| B-2 | - | 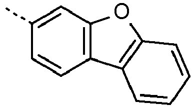 |
| B-3 | - | 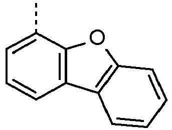 |
| B-4 | - | 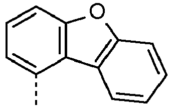 |
| B-5 | - | 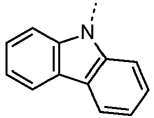 |
| B-6 | - | 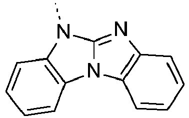 |
| B-7 | - | 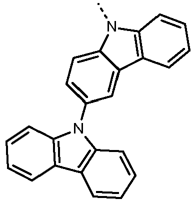 |
| B-8 | - | 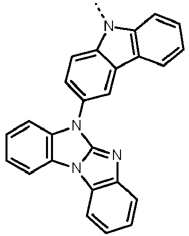 |
| B-9 | - | 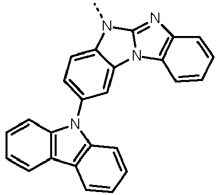 |
| B-10 | - | 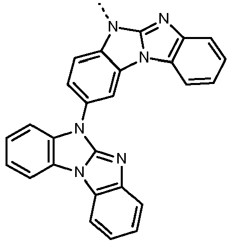 |
| B-11 | - | 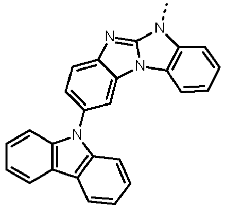 |
| B-12 | - | 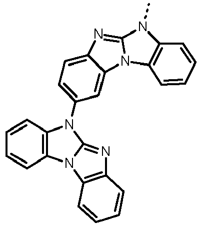 |
| B-13 | - | 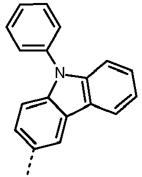 |
| B-14 | - | 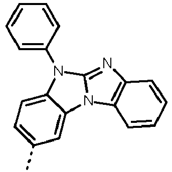 |
| B-15 | - | 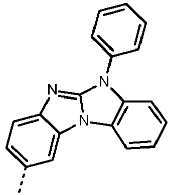 |
| B-16 | 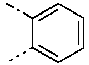 | 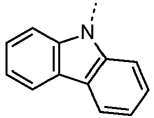 |
| B-17 | 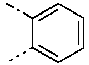 | 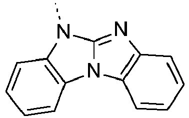 |
| B-18 | 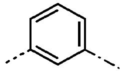 | 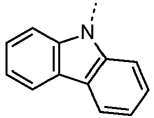 |
| B-19 | 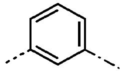 | 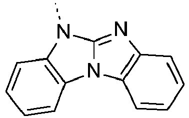 |
| B-20 | 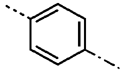 | 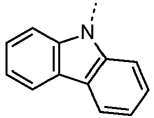 |
| B-21 | 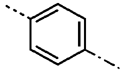 | 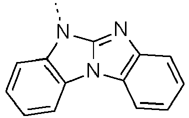 |
| B-22 | 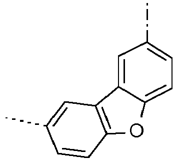 | 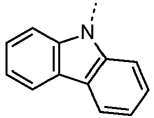 |
| B-23 | 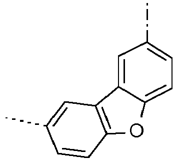 | 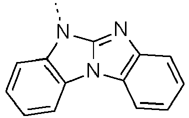 |
| B-24 | 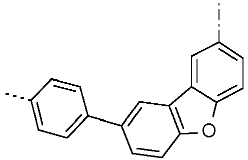 | 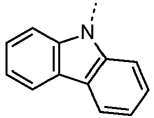 |
| B-25 | 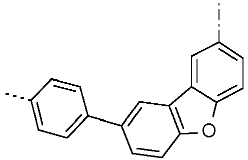 | 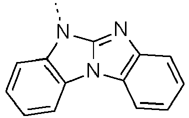 |
| B-26 | 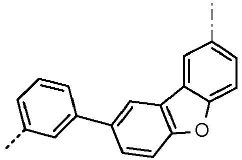 | 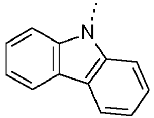 |
| B-27 | 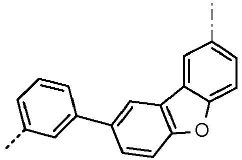 | 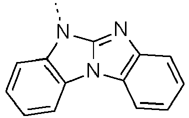 |
| B-28 | 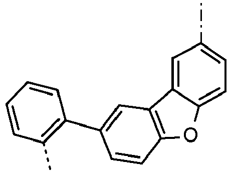 | 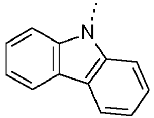 |
| B-29 | 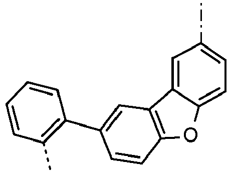 | 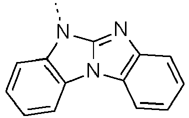 |
| B-30 | - | 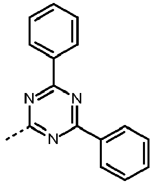 |
| B-31 | - | 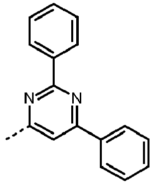 |
| B-32 | - | 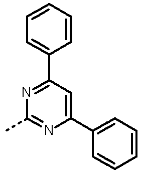 |
| B-33 | 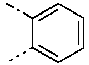 | 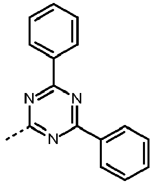 |
| B-34 | 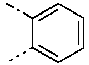 | 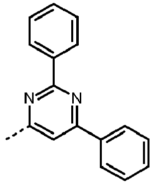 |
| B-35 | 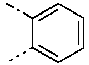 | 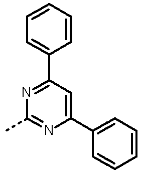 |
| B-36 | 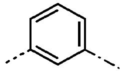 | 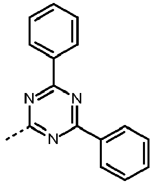 |
| B-37 | 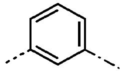 | 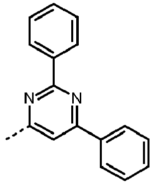 |
| B-38 | 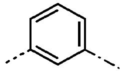 | 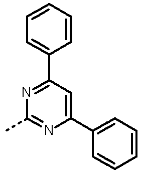 |
| B-39 | 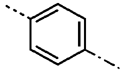 | 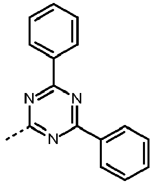 |
| B-40 | 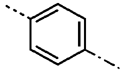 | 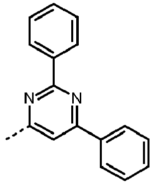 |
| B-41 | 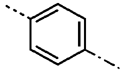 | 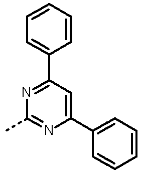 |
| B-42 | - | 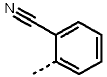 |
| B-43 | - | 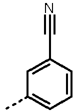 |
| **B-44** | - | 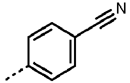 |
| **B-45** | - | 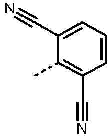 |
| **B-46** | - | 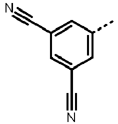 |

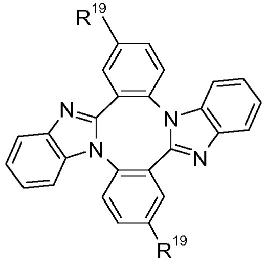

| **Compound** | **R¹²** | **R¹³** |
|---|---|---|
| **C-1** | H | H |
| **C-2** | CN | CN |

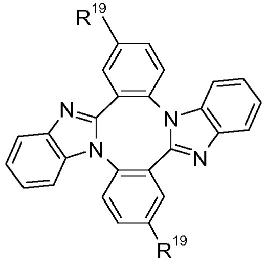

wherein both residues R¹⁹ are identical and are a group of formula -(Ar¹)ₒ-(Ar²)ₚ-(Ar³)_{q}-A :

| Compound | -(Ar¹)ₒ-(Ar²)ₚ-(Ar³)_{q}- | A |
|---|---|---|
| **D-1** | - | 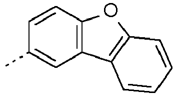 |
| **D-2** | - | 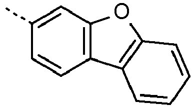 |
| **D-3** | - | 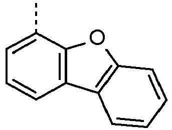 |
| **D-4** | - | 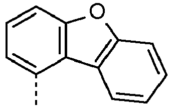 |
| **D-5** | - | 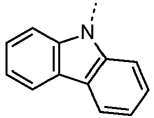 |
| **D-6** | - | 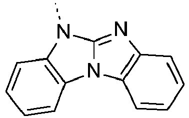 |
| **D-7** | - | 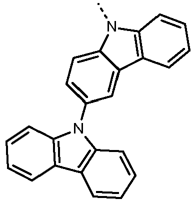 |
| **D-8** | - | 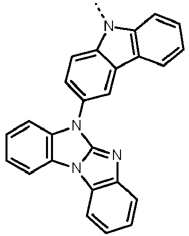 |
| **D-9** | - | 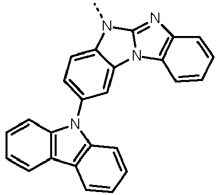 |
| **D-10** | - | 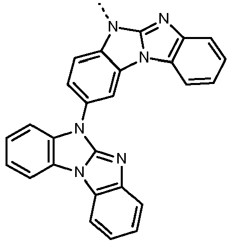 |
| **D-11** | - | 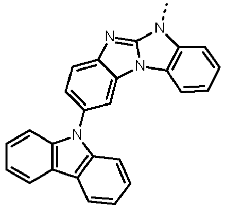 |
| **D-12** | - | 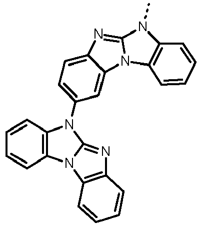 |
| **D-13** | - | 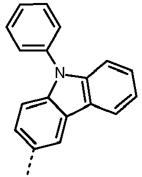 |
| **D-14** | - | 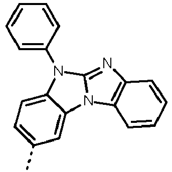 |
| **D-15** | - | 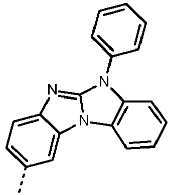 |
| **D-16** | 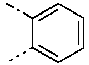 | 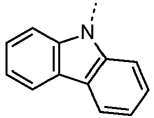 |
| **D-17** | 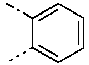 | 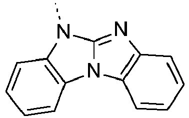 |
| **D-18** | 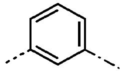 | 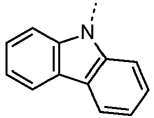 |
| **D-19** | 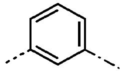 | 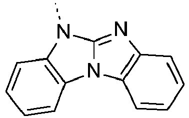 |
| **D-20** | 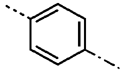 | 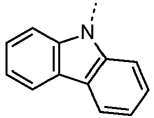 |
| **D-21** | 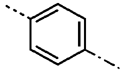 | 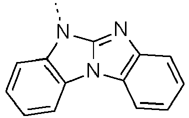 |
| **D-22** | 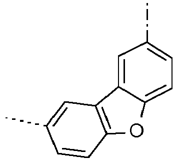 | 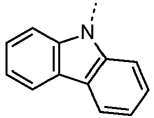 |
| **D-23** | 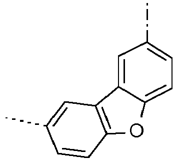 | 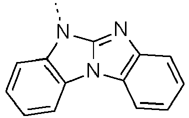 |
| **D-24** | 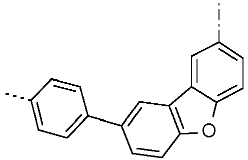 | 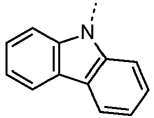 |
| **D-25** | 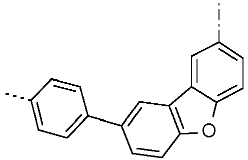 | 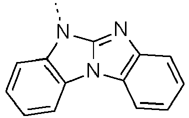 |
| **D-26** | 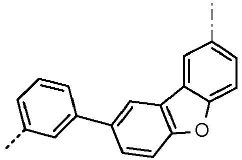 | 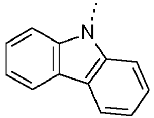 |
| **D-27** | 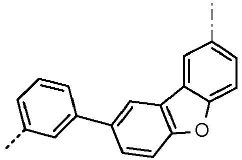 | 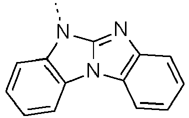 |
| **D-28** | 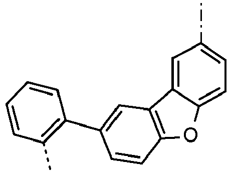 | 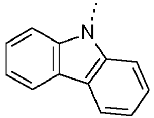 |
| **D-29** | 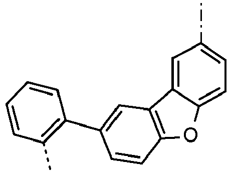 | 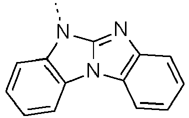 |
| **D-30** | - | 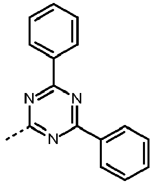 |
| **D-31** | - | 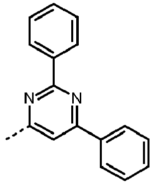 |
| **D-32** | - | 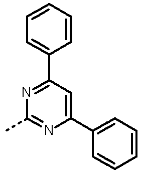 |
| **D-33** | 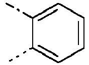 | 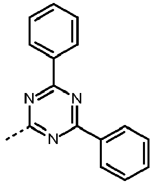 |
| **D-34** | 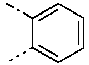 | 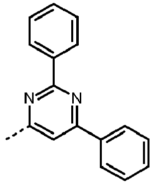 |
| **D-35** | 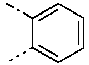 | 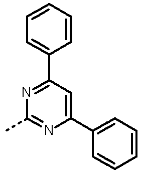 |
| **D-36** | 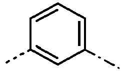 | 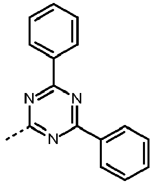 |
| **D-37** | 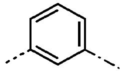 | 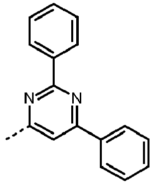 |
| **D-38** | 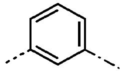 | 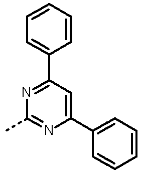 |
| **D-39** | 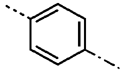 | 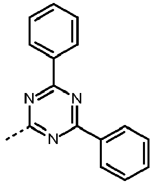 |
| **D-40** | 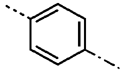 | 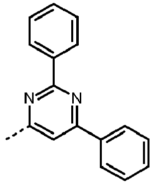 |
| **D-41** | 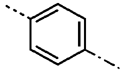 | 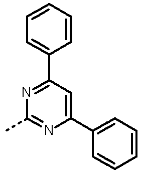 |
| **D-42** | - | 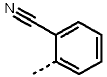 |
| **D-43** | - | 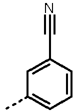 |
| **D-44** | - | 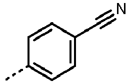 |
| **D-45** | - | 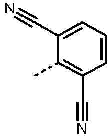 |
| **D-46** | - | 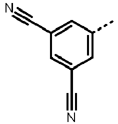 |

Examples for particularly preferred compounds are:

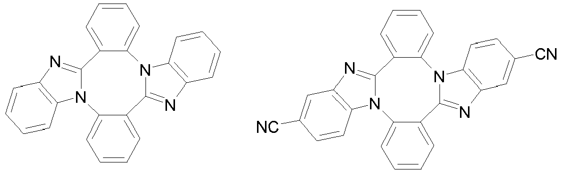

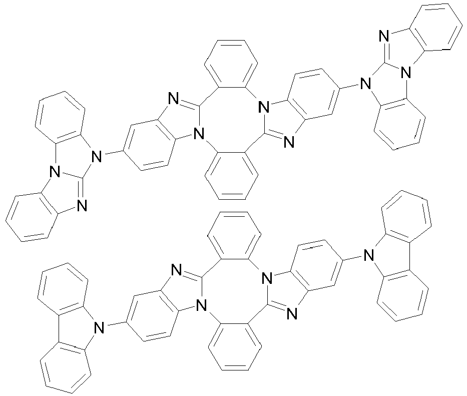

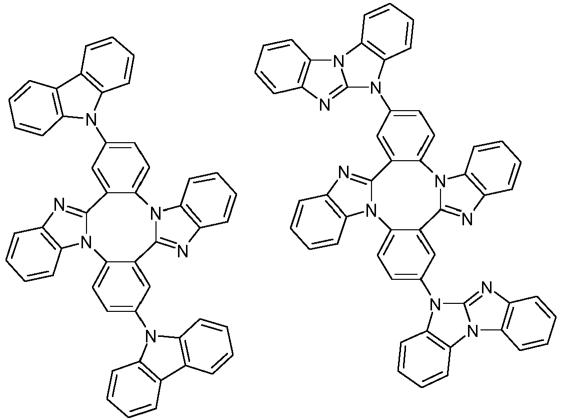

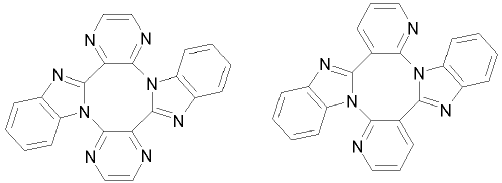

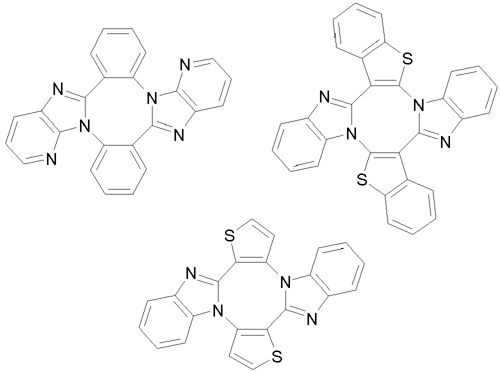

### Process for the preparation of the compounds of formula (I)

The compounds of formula (I) 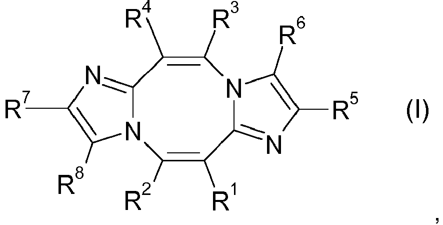 wherein
R¹, R², R³, R⁴, R⁵, R⁶, R⁷ and R⁸ are defined above,
can for example prepared by the following process:
   a) reacting a compound of formula 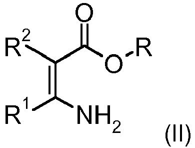 and a compound of formula 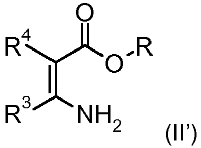 with a base in a solvent to obtain a compound of formula 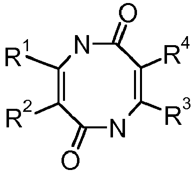 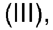 wherein R is C₁-C₁₈alkyl; C₁-C₁₈alkyl which is interrupted by -O-; C₃-C₂₅cycloalkyl; C₆-C₁₈ aryl group; or a heteroaryl group having 5 to 22 ring atoms;
   b) reacting the compound of formula (III) with a base and a compound of formula 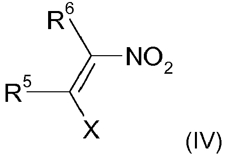 and a compound of formula 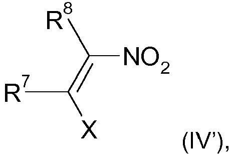 wherein X is F, Cl, Br or I, in a solvent to obtain a compound of formula 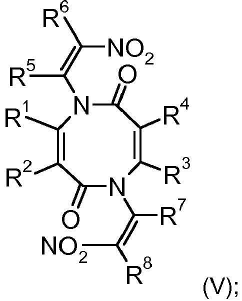
   c1) reacting the compound of formula (V) with a reducing agent in a solvent to obtain a compound of formula (I); or
   c2) reducing the compound of formula (V) with H₂ in the presence of Pd/C in a solvent to obtain a compound of formula 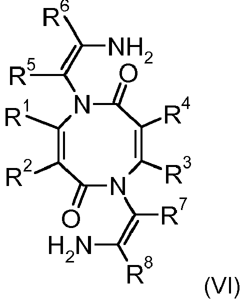 and
   d) reacting the compound of formula (VI) with an catalyst to obtain a compound of formula (I).

### Step a)

The reaction conditions, bases and solvents suitable are known by a person skilled in the art.

Suitable bases are preferably selected from the group consisting of alkali metal and alkaline earth metal hydroxides such as NaOH, KOH, Ca(OH)₂, alkali metal hydrides such as NaH, KH, alkali metal amides such as NaNH₂, alkali metal or alkaline earth metal carbonates such as K₂CO₃ or Cs₂CO₃, alkali metal phosphates, such as, for example, K₃PO₄, and alkali metal alkoxides such as NaOMe, NaOEt. In addition, mixtures of the aforementioned bases are suitable.

More preferred bases are NaH, KOH, NaOH, K₃PO₄, K₂CO₃, Cs₂CO₃. Particular preference is given to K₃PO₄ and K₂CO₃.

Suitable solvents are, for example, (polar) aprotic solvents such asTHF, dioxane, dimethyl sulfoxide (DMSO), dimethylformamide (DMF), N-methyl-2-pyrrolidone (NMP), tridecane or alcohols.

More preferred solvents are THF, dioxane, DMFand DMSO.

The temperature is preferably in the range from room temperature (r.t.) (i.e. 20°C) to 100°C.

The reaction time is preferably from 4 to 42 hours.

### Step b)

The reaction conditions, bases and solvents suitable are known by a person skilled in the art.

Suitable bases are preferably selected from the group consisting of alkali metal and alkaline earth metal hydroxides such as NaOH, KOH, Ca(OH)₂, alkali metal hydrides such as NaH, KH, alkali metal amides such as NaNH₂, alkali metal or alkaline earth metal carbonates such as K₂CO₃ or Cs₂CO₃, alkali metal phosphates, such as, for example, K₃PO₄, and alkali metal alkoxides such as NaOMe, NaOEt. In addition, mixtures of the aforementioned bases are suitable.

More preferred bases are NaH, KOH, NaOH, K₃PO₄, K₂CO₃ and Cs₂CO₃. Particular preference is given to K₃PO₄ and K₂CO₃.

Suitable solvents are, for example, (polar) aprotic solvents such asTHF, dioxane, dimethyl sulfoxide (DMSO), dimethylformamide (DMF), N-methyl-2-pyrrolidone (NMP), tridecane or alcohols such as ethanol.

More preferred solvents are THF, dioxane, DMFand DMSO.

The temperature is preferably in the range from room temperature (i.e. 20°C) to 100°C.

The reaction time is preferably from 4 to 12 hours.

### Step c1)

The reaction conditions and solvents suitable are known by a person skilled in the art. Preferred reducing agents are for example Sn, Zn and/or Fe

Preferred solvents are alcohols such as ethanol, isopropanol, acetic acid; and/or THF. Additionally, HCl (concentrated or diluted with water) may be added.

The temperature ranges from r.t. to 130°C and the reaction last between 4 to 12h

### Step c2)

The reaction conditions and solvents suitable are known by a person skilled in the art.

Suitable solvents are, for example, (polar) aprotic solvents such asTHF, dioxane, dimethyl sulfoxide (DMSO), dimethylformamide (DMF), N-methyl-2-pyrrolidone (NMP), tridecane or alcohols such as ethanol.

Preferred solvents are THF, ethanol and/or DMF.

The temperature is preferably in the range from room temperature (i.e. 20°C) to 50°C.

The reaction time is preferably from 1 to 5 hours.

The pressure of hydrogen is preferably from 1 to 5 bars.

### Step d)

The reaction conditions, acids and solvents suitable are known by a person skilled in the art.

Preferred catalysts are p-touenesulfonic acid, polyphosphoric acid and/or trifluoroacetic acid. Step d) may be carried out in the presence or in absence of a solvent.

In the case that a solvent is used, preferred solvents are aromatic solvents such as toluene and xylene.

The temperature is preferably in the range from room temperature (i.e. 20°C) to 150°C.

The reaction time is preferably from 5 to 12 hours.

### Compounds of the general formula (I)

The present invention further relates to compounds of formula (I), 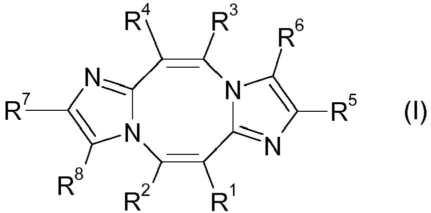 wherein
R¹, R², R³, R⁴, R⁵, R⁶, R⁷ and R⁸
are independently hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
   or
R¹ and R² and/or R³ and R⁴ an/or R⁵ and R⁶ and/or R⁷ and R⁸
independently of each other form together with the carbon atoms to which they are bonded a 5-or 6-membered (hetero)aromatic ring, which can optionally be part of an annelated ring system, wherein the (hetero)aromatic ring and/or annelated ring system can optionally be substituted by G, or by a group of formula -(Ar¹)ₒ-(Ar²)ₚ-(Ar³)_{q}-A; and the hetero atoms of the 5- or 6-membered heteroaromatic ring are selected from N, S and O;
   preferably,
R¹ and R² and/or R³ and R⁴,
independently of each other form a 5- or 6-membered (hetero)aromatic ring selected from 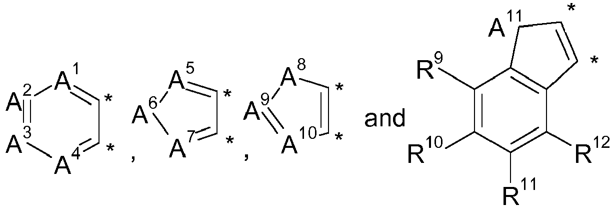 and/or
R⁵ and R⁶ and/or R⁷ and R⁸
independently of each other form a 5- or 6-membered (hetero)aromatic ring selected from 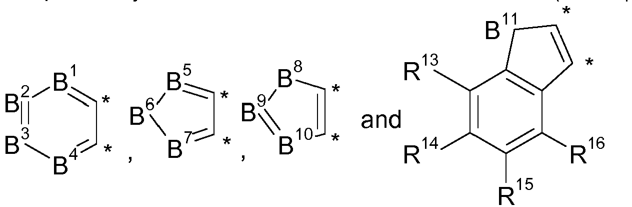 wherein
   A¹ and B¹ are independently N, or CR¹⁷,
   A² and B² are independently N, or CR¹⁸,
   A³ and B³ are independently N, or CR¹⁹,
   A⁴ and B⁴ are independently N, or CR²⁰,
   A⁵ and B⁵ are independently N, or CR²¹,
   A⁶ and B⁶ are independently O, S, NR²⁵ or CR²⁶R²⁷,
   A⁷ and B⁷ are independently N, or CR²²,
   A⁸ and B⁸ are independently O, S, NR²⁵ or CR²⁶R²⁷,
   A⁹ and B⁹ are independently N, or CR²³,
   A¹⁰ and B¹⁰ are independently N, or CR²⁴,
   A¹¹ and B¹¹ are independently O, S, NR²⁵ or CR²⁶R²⁷;
   R⁹, R¹⁰, R¹¹, R¹², R¹³, R¹⁴, R¹⁵, R¹⁶, R¹⁷, R¹⁸, R¹⁹, R²⁰, R²¹, R²², R²³ and R²⁴ are independently hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G, or a group of formula -(Ar¹)ₒ₋(Ar²)ₚ₋(Ar³)_{q}-A;
   R²⁵, R²⁶, R²⁷
   are independently hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
   * represents the carbon atoms of the basic sceleton;
   D is -CO-, -COO-, -S-, -SO-, -SO₂-, -O-, -NR⁶⁵-, -SiR⁷⁰R⁷¹-, -POR⁷²-, -CR⁶³=CR⁶⁴-, or -C≡C-,
   E is F; Cl; Br; CN; an unsubstituted C₆-C₂₂aryl group, a C₆-C₂₂aryl group, which is substituted by F, C₁-C₁₈alkyl, C₁-C₁₈alkyl which is interrupted by O; an unsubstituted heteroaryl group having 5 to 22 ring atoms, a heteroaryl group having 5 to 22 ring atoms, which is substituted by F, -OR⁶⁹, -SR⁶⁹, -NR⁶⁵R⁶⁶, -COR⁶⁸, -COOR⁶⁷, -CONR⁶⁵R⁶⁶, -CN, or F;
   G is E, -OR⁶⁹; -SR69; -NR⁶⁵R⁶⁶;
   R⁶³ and R⁶⁴ are independently of each other H, C₆-C₁₈aryl; C₆-₁₈aryl which is substituted by C₁-C₁₈alkyl, or C₁-C₁₈alkoxy; C₁-C₁₈alkyl; or C₁-C₁₈alkyl which is interrupted by -O-;
   R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
   R⁶⁵ and R⁶⁶ together form a five or six membered ring,
   R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
   R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
   R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
   R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
   R⁷² is a C₁-C₁₈alkyl group, a C₆-C₁₈aryl group, or a C₆-C₁₈aryl group, which is substituted by C₁-C₁₈alkyl;
   Ar¹, Ar², Ar³ are are independently of each other a C₆-C₂₄arylen group, which can optionally be substituted by G, or a C₂-C₃₀heteroarylen group, which can optionally be substituted by G;
   A is hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
   o is 0, or 1;
   p is 0, or 1; and
   q is 0, or 1;
   with the proviso that the following compounds are excluded:

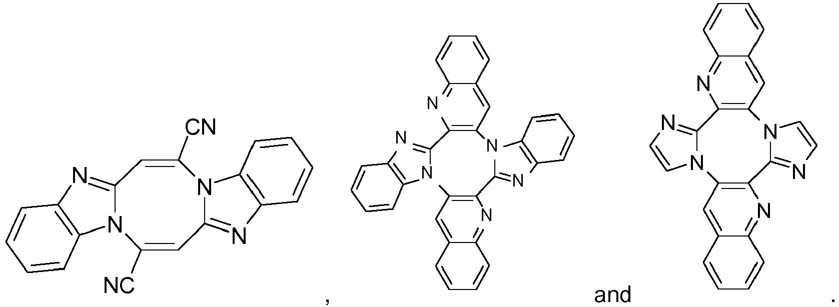

Preferably, the present invention relates to compounds of formula (I), wherein
R¹, R², R³, R⁴, R⁵, R⁶, R⁷ and R⁸
are independently hydrogen, halogen, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
or
R¹ and R² and/or R³ and R⁴ an/or R⁵ and R⁶ and/or R⁷ and R⁸
independently of each other form together with the carbon atoms to which they are bonded a 5-or 6-membered (hetero)aromatic ring, which can optionally be part of an annelated ring system, wherein the (hetero)aromatic ring and/or annelated ring system can optionally be substituted by G, or by a group of formula -(Ar¹)ₒ-(Ar²)ₚ-(Ar³)_{q}-A; and the hetero atoms of the 5- or 6-membered heteroaromatic ring are selected from N, S and O.

Preferred residues and groups in the compounds of formula (I) as well as preferred compounds of formula (I) are mentioned above.

### Organic Electronic Devices

It has been found that the compounds of the formula (I) are particularly suitable for use in applications in which charge carrier conductivity is required, especially for use in organic electronics applications (organic electronic devices), for example selected from switching elements such as organic transistors, e.g. organic FETs and organic TFTs, organic solar cells and organic light-emitting diodes (OLEDs), the compounds of the formula (I) being particularly suitable in OLEDs for use as matrix material in a light-emitting layer and/or as electron transport material and/or as hole transport material (i.e. as charge transport material), and/or as charge/exciton blocker material, especially in combination with a phosphorescence emitter. In the case of use of the compounds of the formula (I) in OLEDs, OLEDs which have good efficiencies and a long lifetime and which can be operated especially at a low use and operating voltage are obtained. The compounds of the formula (I) are suitable especially for use as matrix and/or charge/exciton transport materials for blue and green emitters, for example light blue or deep blue emitters, these being especially phosphorescence emitters. Furthermore, the compounds of the formula (I) can be used as conductor/complementary materials in organic electronics applications selected from switching elements and organic solar cells.

The compounds of the formula (I) can be used as matrix material and/or charge transport material (charge conductor material) and/or charge/exciton blocker material. The present invention therefore preferably concerns organic electronic devices, especially OLEDS, comprising a compound of the formula (I) as matrix material and/or charge transport material and/or charge/exciton blocker material.

In the emission layer or one of the emission layers of an OLED, it is also possible to combine an emitter material with a matrix material of the compound of the formula (I) and a further matrix material (second host). This may achieve a high quantum efficiency of this emission layer.

It is likewise possible that the compounds of the formula (I) are present in the light-emitting layer (preferably as matrix material) and in the charge transport layers (as charge transport material) and/or additionally in the charge/exciton blocker layer (charge/exciton blocker material) of an OLED.

Suitable structures of organic electronic devices are known to those skilled in the art and are specified below.

The organic transistor generally includes a semiconductor layer formed from an organic layer with charge transport capacity; a gate electrode formed from a conductive layer; and an insulating layer introduced between the semiconductor layer and the conductive layer. A source electrode and a drain electrode are mounted on this arrangement in order thus to produce the transistor element. In addition, further layers known to those skilled in the art may be present in the organic transistor.

The organic solar cell (photoelectric conversion element) generally comprises an organic layer present between two plate-type electrodes arranged in parallel. The organic layer may be configured on a comb-type electrode. There is no particular restriction regarding the site of the organic layer and there is no particular restriction regarding the material of the electrodes. When, however, plate-type electrodes arranged in parallel are used, at least one electrode is preferably formed from a transparent electrode, for example an ITO electrode or a fluorine-doped tin oxide electrode. The organic layer is formed from two sublayers, i.e. a layer with p-type semiconductor properties or hole transport capacity, and a layer formed with n-type semiconductor properties or charge transport capacity. In addition, it is possible for further layers known to those skilled in the art to be present in the organic solar cell. The layers with charge transport capacity may comprise the compounds of formula (I).

The present invention further provides an organic light-emitting diode (OLED) comprising an anode (a) and a cathode (i) and a light-emitting layer (e) arranged between the anode (a) and the cathode (i), and if appropriate at least one further layer selected from the group consisting of at least one blocking layer for holes/excitons, at least one blocking layer for electrons/excitons, at least one hole injection layer, at least one hole transport layer, at least one electron injection layer and at least one electron transport layer, wherein the at least one compound of the formula (I) is present in the light-emitting layer (e) and/or in at least one of the further layers. The at least one compound of the formula (I) is preferably present in the light-emitting layer and/or the charge transport layers and/or charge/exciton blocker layer.

The present invention further relates to a charge transport layer, a charge/exciton blocker layer, or an emitting layer comprising a compound of formula (I). Preferred compounds of formula (I) are mentioned above.

The present application further relates to a light-emitting layer comprising a compound of formula (I) as host material, preferably in combination with a phosphorescent emitter. Preferred compounds of formula (I) are mentioned above and preferred phosphorescent emitter are mentioned below.

### Structure of the inventive OLED

The inventive organic light-emitting diode (OLED) thus generally has the following structure: an anode (a) and a cathode (i) and a light-emitting layer (e) arranged between the anode (a) and the cathode (i).

The inventive OLED may, for example - in a preferred embodiment - be formed from the following layers:
1. Anode (a)
2. Hole transport layer (c)
3. Light-emitting layer (e)
4. Blocking layer for holes/excitons (f)
5. Electron transport layer (g)
6. Cathode (i)

Layer sequences different than the aforementioned structure are also possible, and are known to those skilled in the art. For example, it is possible that the OLED does not have all of the layers mentioned; for example, an OLED with layers (a) (anode), (e) (light-emitting layer) and (i) (cathode) is likewise suitable, in which case the functions of the layers (c) (hole transport layer) and (f) (blocking layer for holes/excitons) and (g) (electron transport layer) are assumed by the adjacent layers. OLEDs which have layers (a), (c), (e) and (i), or layers (a), (e), (f), (g) and (i), are likewise suitable. In addition, the OLEDs may have a blocking layer for electrons/excitons (d) between the hole transport layer (c) and the Light-emitting layer (e).

It is additionally possible that a plurality of the aforementioned functions (electron/exciton blocker, hole/exciton blocker, hole injection, hole conduction, electron injection, electron conduction) are combined in one layer and are assumed, for example, by a single material present in this layer. For example, a material used in the hole transport layer, in one embodiment, may simultaneously block excitons and/or electrons.

Furthermore, the individual layers of the OLED among those specified above may in turn be formed from two or more layers. For example, the hole transport layer may be formed from a layer into which holes are injected from the electrode, and a layer which transports the holes away from the hole-injecting layer into the light-emitting layer. The electron conduction layer may likewise consist of a plurality of layers, for example a layer in which electrons are injected by the electrode, and a layer which receives electrons from the electron injection layer and transports them into the light-emitting layer. These layers mentioned are each selected according to factors such as energy level, thermal resistance and charge carrier mobility, and also energy difference of the layers specified with the organic layers or the metal electrodes. The person skilled in the art is capable of selecting the structure of the OLEDs such that it is matched optimally to the organic compounds used in accordance with the invention.

In a preferred embodiment the OLED according to the present invention comprises in this order:
(a) an anode,
(b) optionally a hole injection layer,
(c) optionally a hole transport layer,
(d) optionally an exciton blocking layer
(e) an emitting layer,
(f) optionally a hole/ exciton blocking layer
(g) optionally an electron transport layer,
(h) optionally an electron injection layer, and
(i) a cathode.

In a particularly preferred embodiment the OLED according to the present invention comprises in this order:
(a) an anode,
(b) optionally a hole injection layer,
(c) a hole transport layer,
(d) an exciton blocking layer
(e) an emitting layer,
(f) a hole/ exciton blocking layer
(g) an electron transport layer, and
(h) optionally an electron injection layer, and
(i) a cathode.

The properties and functions of these various layers, as well as example materials are known from the prior art and are described in more detail below on basis of preferred embodiments.

### Anode (a):

The anode is an electrode which provides positive charge carriers. It may be composed, for example, of materials which comprise a metal, a mixture of different metals, a metal alloy, a metal oxide or a mixture of different metal oxides. Alternatively, the anode may be a conductive polymer. Suitable metals comprise the metals of groups 11, 4, 5 and 6 of the Periodic Table of the Elements, and also the transition metals of groups 8 to 10. When the anode is to be transparent, mixed metal oxides of groups 12, 13 and 14 of the Periodic Table of the Elements are generally used, for example indium tin oxide (ITO). It is likewise possible that the anode (a) comprises an organic material, for example polyaniline, as described, for example, in Nature, Vol. 357, pages 477 to 479 (June 11, 1992). Preferred anode materials include conductive metal oxides, such as indium tin oxide (ITO) and indium zinc oxide (IZO), aluminum zinc oxide (AlZnO), and metals. Anode (and substrate) may be sufficiently transparent to create a bottom-emitting device. A preferred transparent substrate and anode combination is commercially available ITO (anode) deposited on glass or plastic (substrate). A reflective anode may be preferred for some top-emitting devices, to increase the amount of light emitted from the top of the device. At least either the anode or the cathode should be at least partly transparent in order to be able to emit the light formed. Other anode materials and structures may be used.

### Hole injection layer (b):

Generally, injection layers are comprised of a material that may improve the injection of charge carriers from one layer, such as an electrode or a charge generating layer, into an adjacent organic layer. Injection layers may also perform a charge transport function. The hole injection layer may be any layer that improves the injection of holes from anode into an adjacent organic layer. A hole injection layer may comprise a solution deposited material, such as a spin-coated polymer, or it may be a vapor deposited small molecule material, such as, for example, CuPc or MTDATA. Polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore^{®} OC Conducting Inks commercially available from Plextronics), and copolymers such as poly(3,4-ethylenedioxythiophene)/poly(4-styrenesulfonate) also called PEDOT/PSS.

### Hole transport layer (c):

Suitable hole transport materials are the compounds of formula (I). However, in the case that further hole transport materials are used in the hole transport layer of the inventive OLED or in the case that the compounds of formula (I) are not used as hole transport materials in the hole transport layer, the following hole transport materials are for example suitable as hole transport materials in the inventive OLED.

Either hole-transporting molecules or polymers may be used as the hole transport material. Suitable hole transport materials for layer (c) of the inventive OLED are disclosed, for example, in Kirk-Othmer Encyclopedia of Chemical Technology, 4th Edition, Vol. 18, pages 837 to 860, 1996, US20070278938, US2008/0106190, US2011/0163302 (triarylamines with (di)benzothiophen/(di)benzofuran; Nan-Xing Hu et al. Synth. Met. 111 (2000) 421 (indolocarbazoles), WO2010002850 (substituted phenylamine compounds) and WO2012/16601 (in particular the hole transport materials mentioned on pages 16 and 17 of WO2012/16601). Combination of different hole transport material may be used. Reference is made, for example, to WO2013/022419, wherein 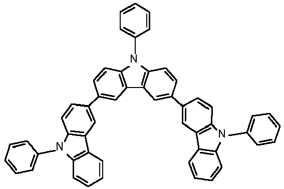 (HTL1-1) and 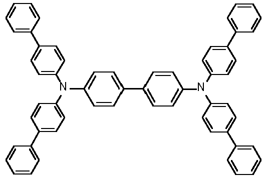 (HTL2-1) constitute the hole transport layer.

Customarily used hole-transporting molecules are selected from the group consisting of 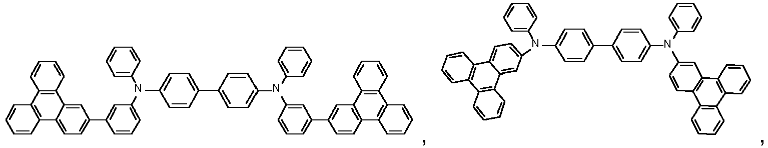 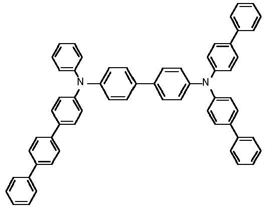 (4-phenyl-N-(4-phenylphenyl)-N-[4-[4-(N-[4-(4-phenyl-phenyl)phenyl]anilino)phenyl]phenyl]aniline), 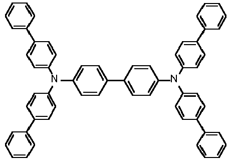 (4-phenyl-N-(4-phenylphenyl)-N-[4-[4-(4-phenyl-N-(4-phenylphenyl)anilino)phenyl]phenyl]aniline), 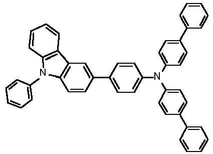 (4-phenyl-N-[4-(9-phenylcarbazol-3-yl)phenyl]-N-(4-phenylphenyl)aniline), 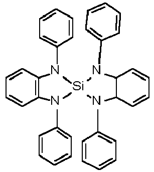 (1,1',3,3'-tetraphenylspiro[1,3,2-benzodiazasilole-2,2'-3a,7a-dihydro-1,3,2-benzodiazasilole]), 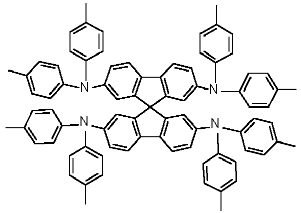 (N2,N2,N2',N2',N7,N7,N7',N7'-octakis(p-tolyl)-9,9'-spirobi[fluorene]-2,2',7,7'-tetramine), 4,4'-bis[N-(1-naphthyl)-N-phenylamino]biphenyl (α-NPD), N,N'-diphenyl-N,N'-bis(3-methylphenyl)-[1,1'-biphenyl]-4,4'-diamine (TPD), 1,1-bis[(di-4-tolylamino)phenyl]cyclohexane (TAPC), N,N'-bis(4-methylphenyl)-N,N'-bis(4-ethylphenyl)-[1,1'-(3,3'-dimethyl)biphenyl]-4,4'-diamine (ETPD), tetrakis(3-methylphenyl)-N,N,N',N'-2,5-phenylenediamine (PDA), α-phenyl-4-N,N-diphenylaminostyrene (TPS), p-(diethylamino)benzaldehyde diphenylhydrazone (DEH), triphenylamine (TPA), bis[4-(N,N-diethylamino)2-methylphenyl](4-methylphenyl)methane (MPMP), 1-phenyl-3-[p-(diethylamino)styryl]5-[p-(diethylamino)phenyl]pyrazoline (PPR or DEASP), 1,2-trans-bis(9H-carbazol9-yl)-cyclobutane (DCZB), N,N,N',N'-tetrakis(4-methylphenyl)-(1,1'-biphenyl)-4,4'-diamine (TTB), fluorine compounds such as 2,2',7,7'-tetra(N,N-di-tolyl)amino9,9-spirobifluorene (spiro-TTB), N,N'-bis(naphthalen-1-yl)-N,N'-bis(phenyl)9,9-spirobifluorene (spiro-NPB) and 9,9-bis(4-(N,N-bis-biphenyl-4-yl-amino)phenyl-9Hfluorene, benzidine compounds such as N,N'-bis(naphthalen-1-yl)-N,N'-bis(phenyl)benzidine and porphyrin compounds such as copper phthalocyanines. In addition, polymeric hole-injection materials can be used such as poly(N-vinylcarbazole) (PVK), polythiophenes, polypyrrole, polyaniline, self-doping polymers, such as, for example, sulfonated poly(thiophene-3-[2[(2-methoxyethoxy)ethoxy]-2,5-diyl) (Plexcore® OC Conducting Inks commercially available from Plextronics), and copolymers such as poly(3,4-ethylenedioxythiophene)/poly(4-styrenesulfonate) also called PEDOT/PSS. Preferred examples of a material of the hole transport layer are a porphyrin compound, an aromatic tertiary amine compound, or a styrylamine compound. Particularly preferable examples include an aromatic tertiary amine compound such as hexacyanohexaazatriphenylene (HAT).

In a preferred embodiment it is possible to use metal carbene complexes as hole transport materials. Suitable carbene complexes are, for example, carbene complexes as described in WO2005/019373A2, WO2006/056418 A2, WO2005/113704, WO2007/115970, WO2007/115981, WO2008/000727 and PCT/EP2014/055520. One example of a suitable carbene complex is lr(DPBIC)₃ with the formula: 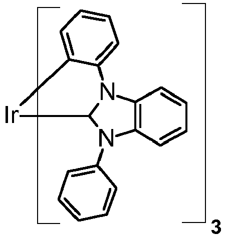 (HTM-1). Another example of a suitable carbene complex is lr(ABIC)₃ with the formula: 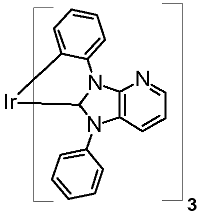 (HTM-2).

The hole-transporting layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, 2003, 359 (p-doped organic layers); A. G. Werner, F. Li, K. Harada, M. Pfeiffer, T. Fritz, K. Leo, Appl. Phys. Lett., Vol. 82, No. 25, 2003, 4495 and Pfeiffer et al., Organic Electronics 2003, 4, 89 - 103 and K. Walzer, B. Maennig, M. Pfeiffer, K. Leo, Chem. Soc. Rev. 2007, 107, 1233. For example it is possible to use mixtures in the hole-transporting layer, in particular mixtures which lead to electrical p-doping of the hole-transporting layer. p-Doping is achieved by the addition of oxidizing materials. These mixtures may, for example, be the following mixtures: mixtures of the abovementioned hole transport materials with at least one metal oxide, for example MoO₂ MoO₃, WOₓ, ReO₃ and/or V₂O₅, preferably MoO₃ and/or ReO₃, more preferably MoO₃, or mixtures comprising the aforementioned hole transport materials and one or more compounds selected from 7,7,8,8-tetracyanoquinodimethane (TCNQ), 2,3,5,6-tetrafluoro-7,7,8,8-tetracyanoquinodimethane (F₄-TCNQ), 2,5-bis(2-hydroxyethoxy)-7,7,8,8-tetracyanoquinodimethane, bis(tetra-n-butylammonium)tetracyanodiphenoquinodimethane, 2,5-dimethyl-7,7,8,8-tetracyanoquinodimethane, tetracyanoethylene, 11,11,12,12-tetracyanonaphtho2,6-quinodimethane, 2-fluoro-7,7,8,8-tetracyanoquino-dimethane, 2,5-difluoro-7,7,8,8etracyanoquinodimethane, dicyanomethylene-1,3,4,5,7,8-hexafluoro-6Hnaphthalen-2-ylidene)malononitrile (F₆-TNAP), Mo(tfd)₃ (from Kahn et al., J. Am. Chem. Soc. 2009, 131 (35), 12530-12531), compounds as described in EP1988587, US2008265216, EP2180029, US20100102709, WO2010132236, EP2180029 and quinone compounds as mentioned in EP2401254. Preferred mixtures comprise the aforementioned carbene complexes, such as, for example, the carbene complexes **HTM-1** and **HTM-2,** and MoO₃ and/or ReO₃, especially MoO₃. In a particularly preferred embodiment the hole transport layer comprises from 0.1 to 10 wt % of MoO₃ and 90 to 99.9 wt % carbene complex, especially of the carbene complex **HTM-1** and **HTM-2,** wherein the total amount of the MoO₃ and the carbene complex is 100 wt %.

### Electron/exciton blocking layer (d):

Blocking layers may be used to reduce the number of charge carriers (electrons or holes) and/or excitons that leave the emissive layer. An electron/exciton blocking layer (d) may be disposed between the first emitting layer (e) and the hole transport layer (c), to block electrons from emitting layer (e) in the direction of hole transport layer (c). Blocking layers may also be used to block excitons from diffusing out of the emissive layer.

Suitable electron blocker materials are the compounds of formula (I). However, in the case that further electron blocker materials are used in the electron blocking layer of the inventive OLED or in the case that the compounds of formula (I) are not used as electron blocker materials in the electron blocking layer, the following electron blocker materials are for example suitable as electron blocker materials in the inventive OLED.

Suitable metal complexes for use as electron/exciton blocker material are, for example, carbene complexes as described in WO2005/019373A2, WO2006/056418A2, WO2005/113704, WO2007/115970, WO2007/115981, WO2008/000727 and PCT/EP2014/055520. Explicit reference is made here to the disclosure of the WO applications cited, and these disclosures shall be considered to be incorporated into the content of the present application. Examples of a suitable carbene complexes are compounds **HTM-1** and **HTM-2.**

### Emitting layer (e)

The light-emitting layer (e) comprises at least one emitter material. In principle, it may be a fluorescence or phosphorescence emitter, suitable emitter materials being known to those skilled in the art. The at least one emitter material is preferably a phosphorescence emitter. The phosphorescence emitter compounds used with preference are based on metal complexes, and especially the complexes of the metals Ru, Rh, Ir, Pd and Pt, in particular the complexes of Ir, have gained significance. The compounds of the formula (I) can be used as matrix material (host material) in the light-emitting layer.

Suitable metal complexes for use in the inventive OLEDs are described, for example, in documents WO 02/60910 A1, US 2001/0015432 A1, US 2001/0019782 A1, US 2002/0055014 A1, US 2002/0024293 A1, US 2002/0048689 A1, EP 1 191 612 A2, EP 1 191 613 A2, EP 1 211 257 A2, US 2002/0094453 A1, WO 02/02714 A2, WO 00/70655 A2, WO 01/41512 A1, WO 02/15645 A1, WO 2005/019373 A2, WO 2005/113704 A2, WO 2006/115301 A1, WO 2006/067074 A1, WO 2006/056418, WO 2006121811 A1, WO 2007095118 A2, WO 2007/115970, WO 2007/115981, WO 2008/000727, WO2010129323, WO2010056669, WO10086089, US2011/0057559, WO2011/106344, US2011/0233528, WO2012/048266 and WO2012/172482.

Further suitable metal complexes are the commercially available metal complexes tris(2-phenylpyridine)iridium(III), iridium(III) tris(2-(4-tolyl)pyridinato-N,C^{2'}), bis(2-phenylpyridine)(acetylacetonato)iridium(III), iridium(III) tris(1-phenylisoquinoline), iridium(III) bis(2,2'-benzothienyl)pyridinato-N,C^{3'})(acetylacetonate), tris(2-phenylquinoline)iridium(III), iridium(III) bis(2-(4,6-difluorophenyl)pyridinato-N,C²)picolinate, iridium(III) bis(1-phenylisoquinoline)(acetylacetonate), bis(2-phenylquinoline)(acetylacetonato)iridium(III), iridium(III) bis(di-benzo[f,h]quinoxaline)(acetylacetonate), iridium(III) bis(2-methyldibenzo[f,h]quinoxaline)(acetylacetonate) and tris(3-methyl-1-phenyl-4-trimethylacetyl-5-pyrazolino)terbium(III), bis[1-(9,9-dimethyl-9H-fluoren-2-yl)isoquinoline](acetyl-acetonato)iridium(III), bis(2-phenylbenzothiazolato)(acetylacetonato)iridium(III), bis(2-(9,9-dihexylfluorenyl)-1-pyridine)(acetylacetonato)iridium(III), bis(2-benzo[b]thiophen-2-yl-pyridine)(acetylacetonato)iridium(III).

In addition, the following commercially available materials are suitable:
tris(dibenzoylacetonato)mono(phenanthroline)europium(III), tris(dibenzoylmethane)-mono(phenanthroline)europium(III), tris(dibenzoylmethane)mono(5-aminophenanthroline)-europium(III), tris(di-2-naphthoylmethane)mono(phenanthroline)europium(III), tris(4-bromobenzoylmethane)mono(phenanthroline)europium(III), tris(di(biphenyl)methane)-mono(phenanthroline)europium(III), tris(dibenzoylmethane)mono(4,7-diphenyl-phenanthroline)europium(III), tris(dibenzoylmethane)mono(4,7-di-methyl-phenanthroline)europium(III), tris(dibenzoylmethane)mono(4,7-dimethylphenanthrolinedisulfonic acid)europium(III) disodium salt, tris[di(4-(2-(2-ethoxyethoxy)ethoxy)benzoylmethane)]mono-(phenanthroline)europium(III) and tris[di[4-(2-(2-ethoxyethoxy)ethoxy)benzoylmethane)]mono(5-aminophenanthroline)europium(III), osmium(II) bis(3-(trifluoromethyl)-5-(4-tert-butylpyridyl)-1,2,4-triazolato)diphenylmethylphosphine, osmium(II) bis(3-(trifluoromethyl)-5-(2-pyridyl)-1,2,4-triazole)dimethylphenylphosphine, osmium(II) bis(3-(trifluoromethyl)-5-(4-tert-butylpyridyl)-1,2,4-triazolato)dimethylphenylphosphine, osmium(II) bis(3-(trifluoromethyl)-5-(2-pyridyl)-pyrazolato)dimethylphenylphosphine, tris[4,4'-di-tert-butyl(2,2')-bipyridine]ruthenium(III), osmium(II) bis(2-(9,9-dibutylfluorenyl)-1-isoquinoline(acetylacetonate).

Preferred phosphorescence emitters are carbene complexes. Suitable phosphorescent blue emitters are specified in the following publications: WO2006/056418A2, WO2005/113704, WO2007/115970, WO2007/115981, WO2008/000727, WO2009050281, WO2009050290, WO2011051404, US2011/057559 WO2011/073149, WO2012/121936A2, US2012/0305894A1, WO2012/170571, WO2012/170461, WO2012/170463, WO2006/121811, WO2007/095118, WO2008/156879, WO2008/156879, WO2010/068876, US2011/0057559, WO2011/106344, US2011/0233528, WO2012/048266, WO2012/172482, PCT/EP2014/064054 and PCT/EP2014/066272.

Preferably, the light emitting layer (e) comprises at least one carbene complex as phosphorescence emitter. Suitable carbene complexes are, for example, compounds of the formula 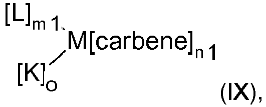 which are described in WO 2005/019373 A2, wherein the symbols have the following meanings:
M is a metal atom selected from the group consisting of Co, Rh, Ir, Nb, Pd, Pt, Fe, Ru, Os, Cr, Mo, W, Mn, Tc, Re, Cu, Ag and Au in any oxidation state possible for the respective metal atom; Carbene is a carbene ligand which may be uncharged or monoanionic and monodentate, bidentate or tridentate, with the carbene ligand also being able to be a biscarbene or triscarbene ligand;
L is a monoanionic or dianionic ligand, which may be monodentate or bidentate;
K is an uncharged monodentate or bidentate ligand selected from the group consisting of phosphines; phosphonates and derivatives thereof, arsenates and derivatives thereof; phosphites; CO; pyridines; nitriles and conjugated dienes which form a π complex with M¹;
n1 is the number of carbene ligands, where n1 is at least 1 and when n1 > 1 the carbene ligands in the complex of the formula I can be identical or different;
m1 is the number of ligands L, where m1 can be 0 or ≥ 1 and when m1 > 1 the ligands L can be identical or different;
o is the number of ligands K, where o can be 0 or ≥ 1 and when o > 1 the ligands K can be identical or different;
where the sum n1 + m1 + o is dependent on the oxidation state and coordination number of the metal atom and on the denticity of the ligands carbene, L and K and also on the charge on the ligands, carbene and L, with the proviso that n1 is at least 1.

More preferred are metal-carbene complexes of the general formula 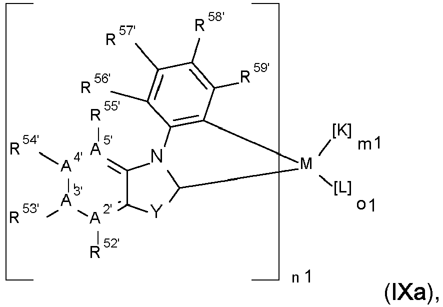 which are described in WO2011/073149, where M is Ir, or Pt,
n1 is an integer selected from 1, 2 and 3,
Y is NR^{51'}, O, S or C(R^{25'})₂,
A^{2'}, A^{3'}, A^{4'}, and A^{5'} are each independently N or C, where 2 A' = nitrogen atoms and at least one carbon atom is present between two nitrogen atoms in the ring,
R^{51'} is a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
R^{52'}, R^{53'}, R^{54'} and R^{55'} are each, if A^{2'}, A^{3'}, A^{4'} and/or A^{5'} is N, a free electron pair, or, if A^{2'}, A^{3'}, A^{4'} and/or A^{5'} is C, each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R^{53'} and R^{54'} together with A^{3'} and A^{4'} form an optionally substituted, unsaturated ring optionally interrupted by at least one further heteroatom and having a total of 5 to 18 carbon atoms and/or heteroatoms,
R^{56'}, R^{57'}, R^{58'} and R^{59'} are each independently hydrogen, linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, cyclo-heteroalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms, group with donor or acceptor action, or
R^{56'} and R^{57'}, R^{57'} and R^{58'} or R^{58'} and R^{59'}, together with the carbon atoms to which they are bonded, form a saturated, unsaturated or aromatic, optionally substituted ring optionally interrupted by at least one heteroatom and having a total of 5 to 18 carbon atoms and/or heteroatoms, and/or
if A^{5'} is C, R^{55'} and R^{56'} together form a saturated or unsaturated, linear or branched bridge optionally comprising heteroatoms, an aromatic unit, heteroaromatic unit and/or functional groups and having a total of 1 to 30 carbon atoms and/or heteroatoms, to which is optionally fused a substituted or unsubstituted, five- to eight-membered ring comprising carbon atoms and/or heteroatoms,
R^{25'} is independently a linear or branched alkyl radical optionally interrupted by at least one heteroatom, optionally bearing at least one functional group and having 1 to 20 carbon atoms, cycloalkyl radical optionally interrupted by at least one heteroatom, optionally bearing at least one functional group and having 3 to 20 carbon atoms, substituted or unsubstituted aryl radical optionally interrupted by at least one heteroatom, optionally bearing at least one functional group and having 6 to 30 carbon atoms, substituted or unsubstituted heteroaryl radical optionally interrupted by at least one heteroatom, optionally bearing at least one functional group and having a total of 5 to 18 carbon atoms and/or heteroatoms,
K is an uncharged mono- or bidentate ligand,
L is a mono- or dianionic ligand, preferably monoanionic ligand, which may be mono- or bidentate,
m1 is 0, 1 or 2, where, when m1 is 2, the K ligands may be the same or different,
o1 is 0, 1 or 2, where, when o1 is 2, the L ligands may be the same or different.

The compound of formula IX is preferably a compound of the formula:

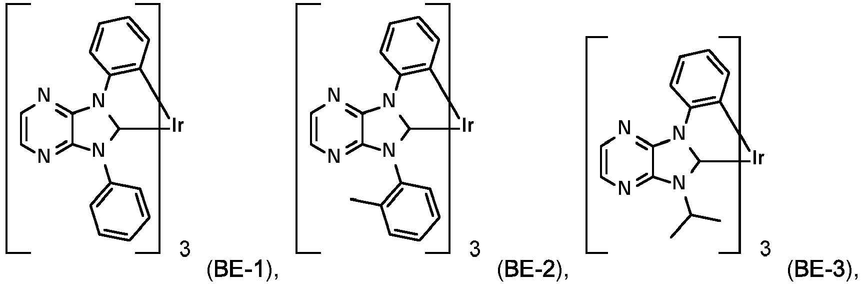

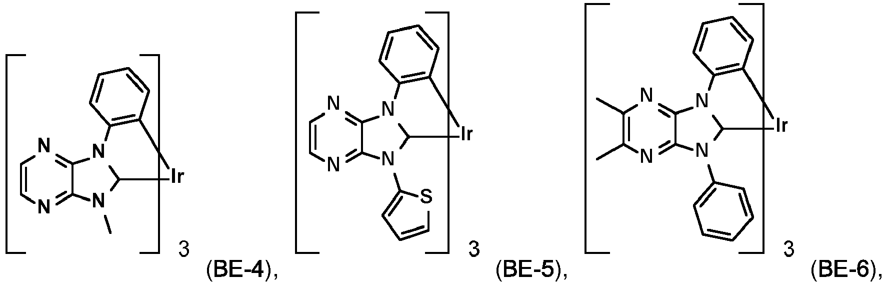

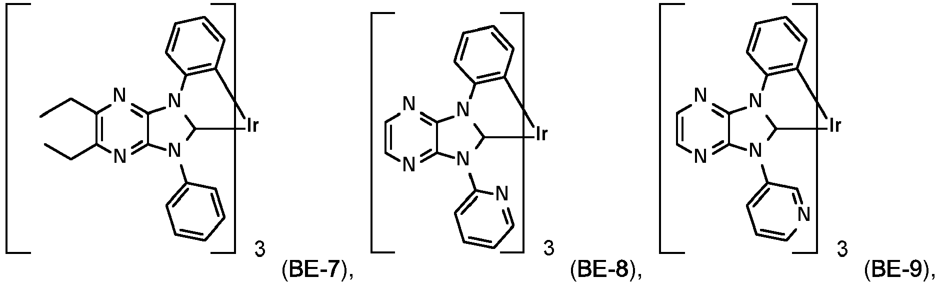

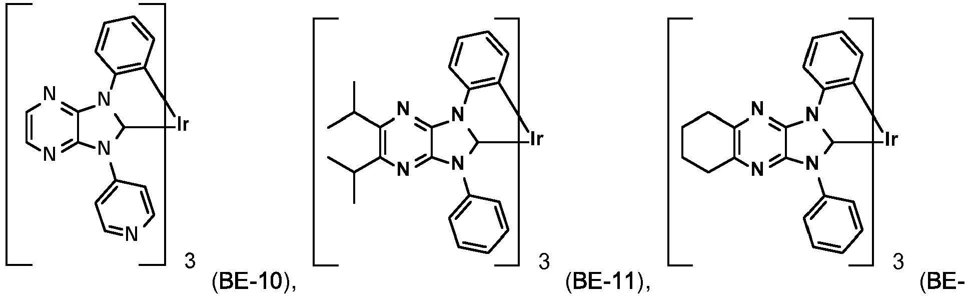

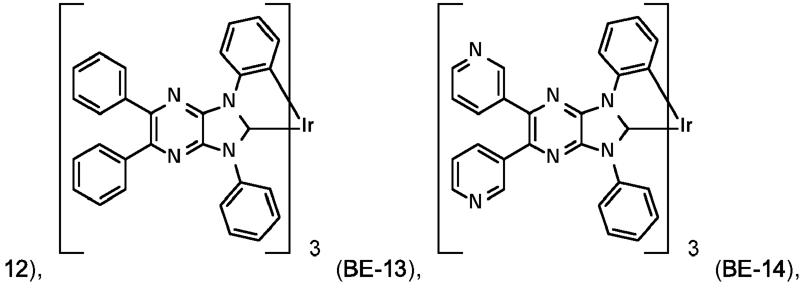

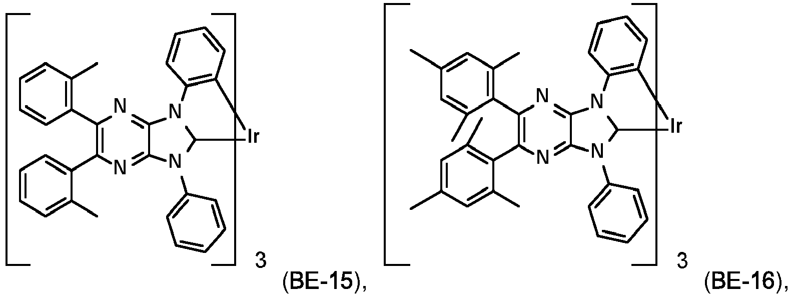

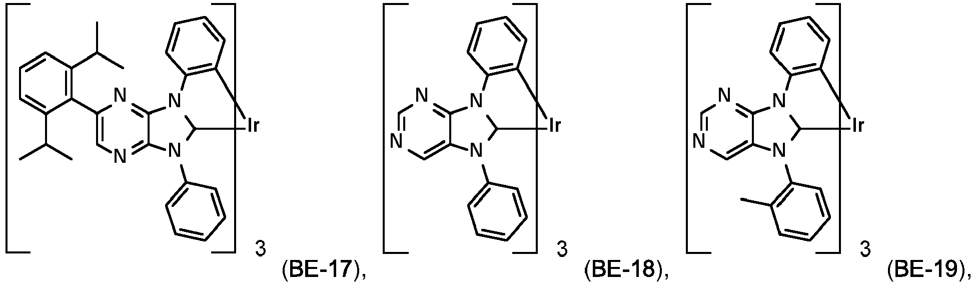

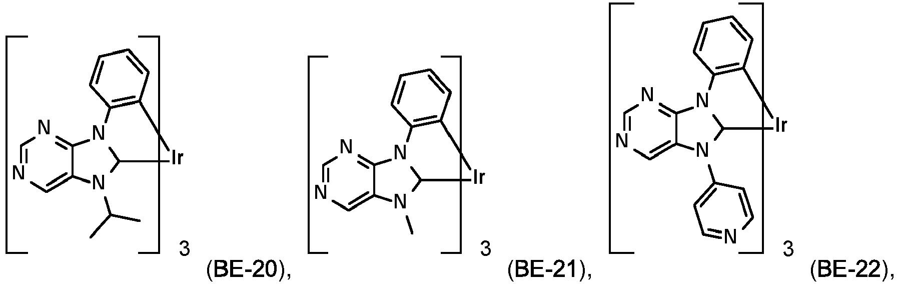

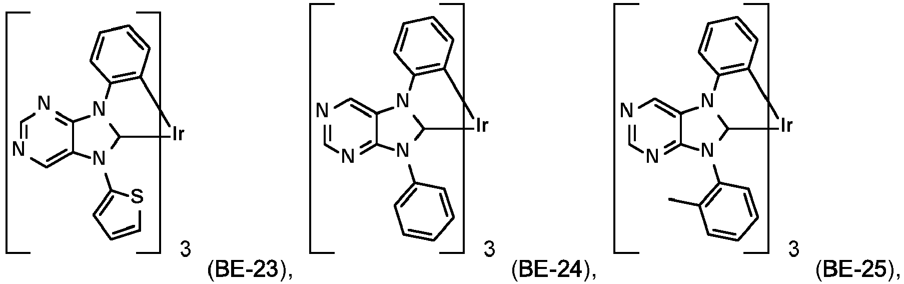

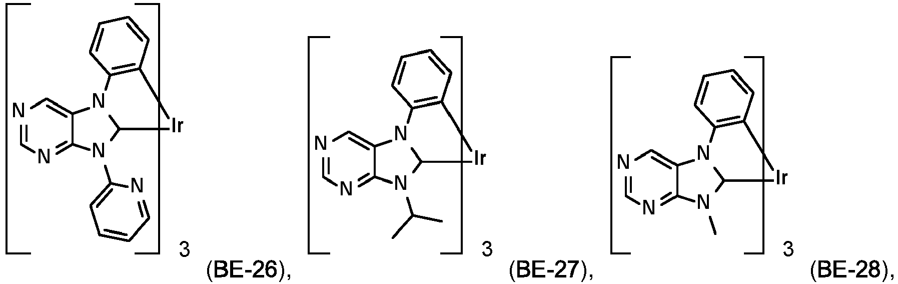

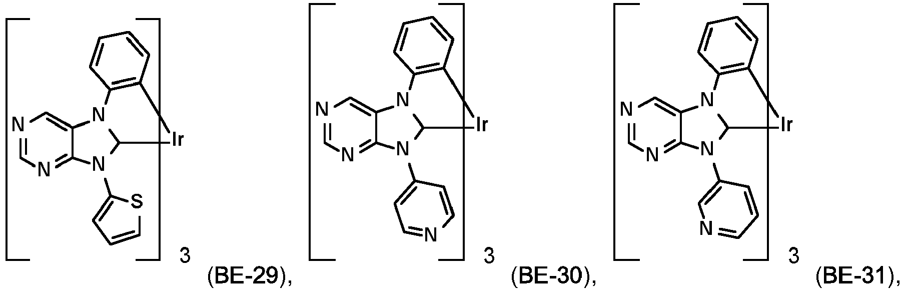

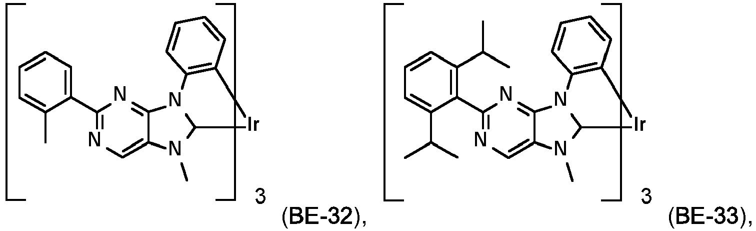

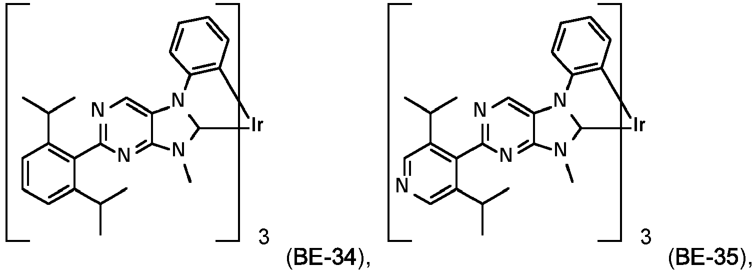

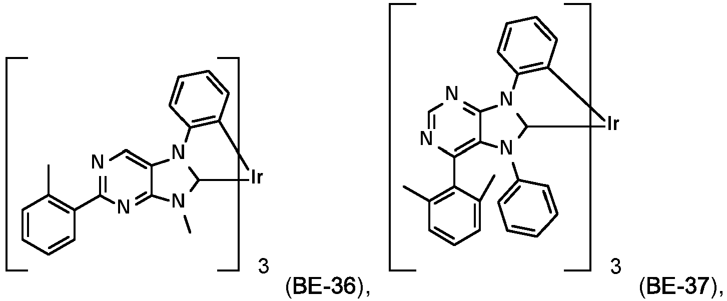

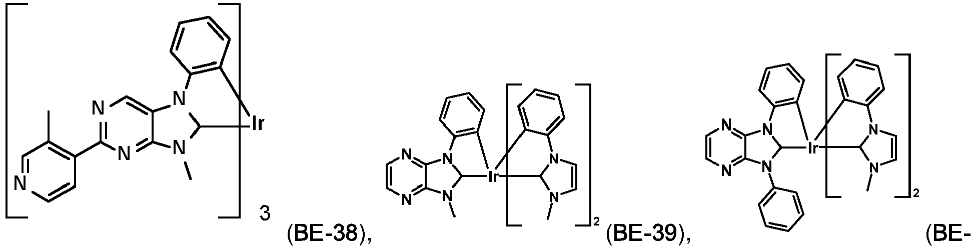

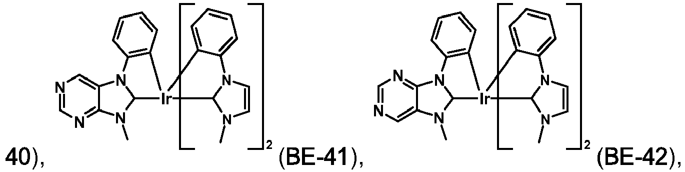

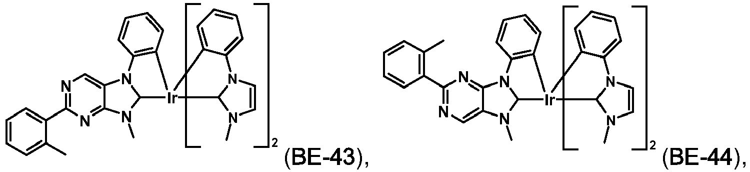

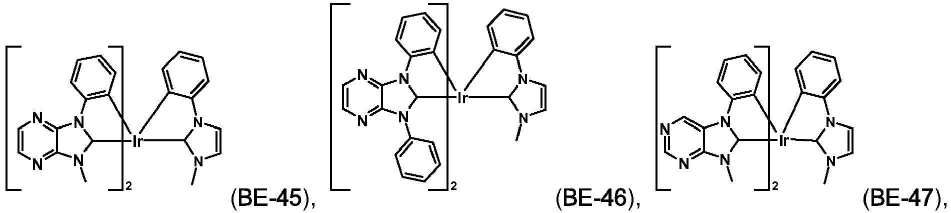

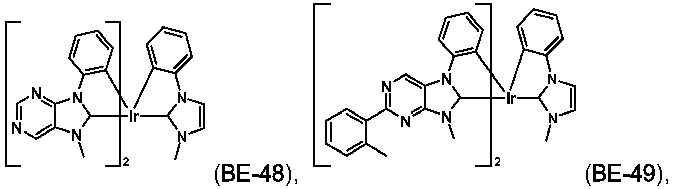

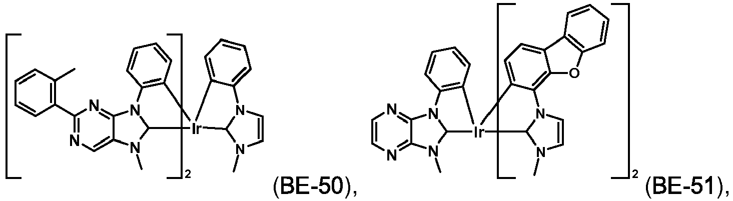

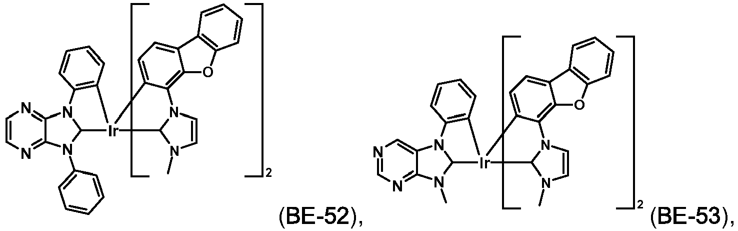

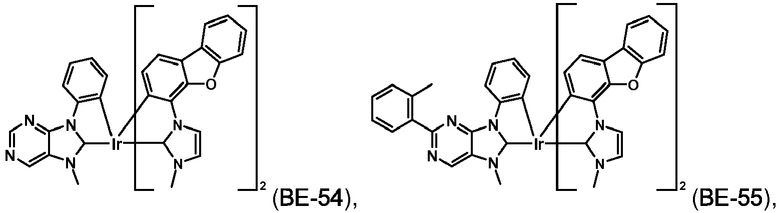

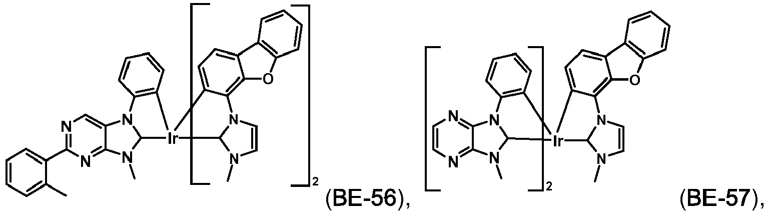

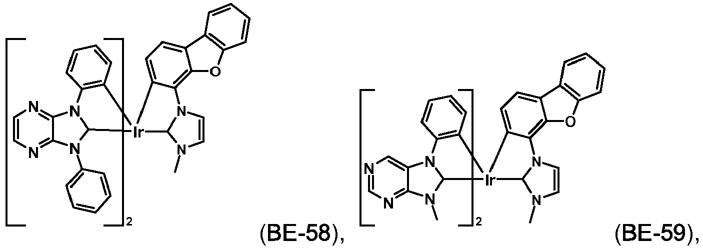

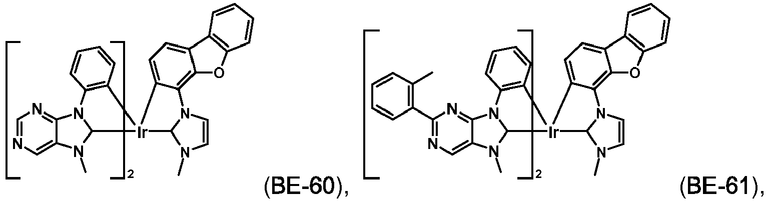

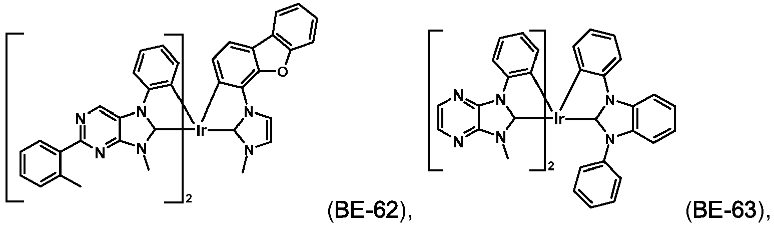

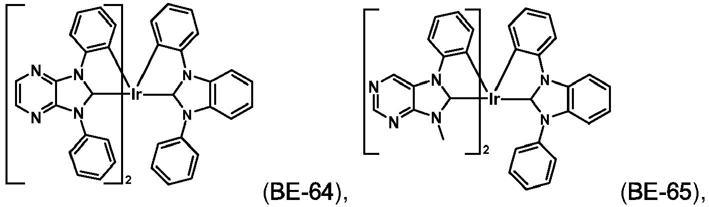

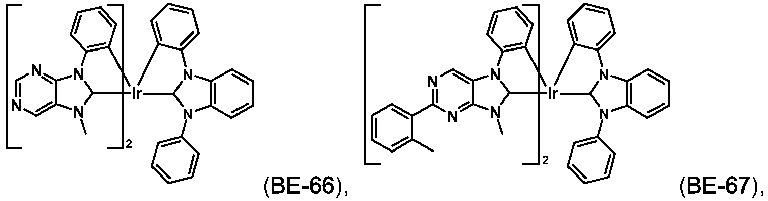

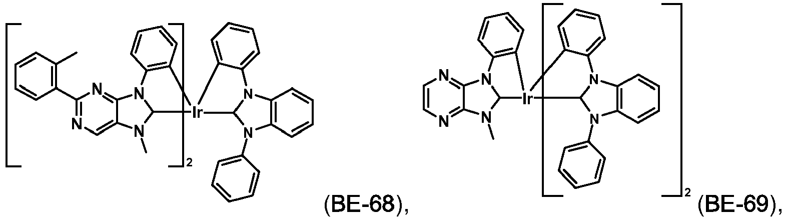

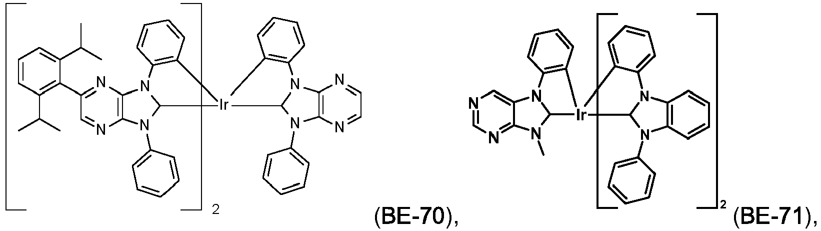

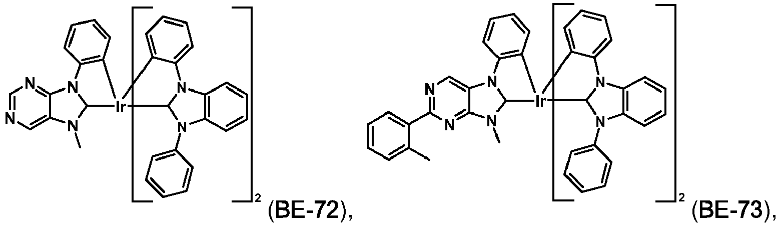

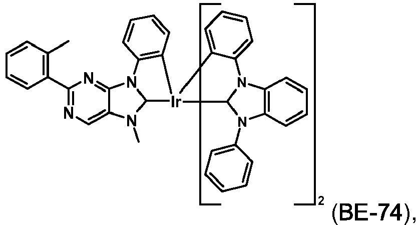

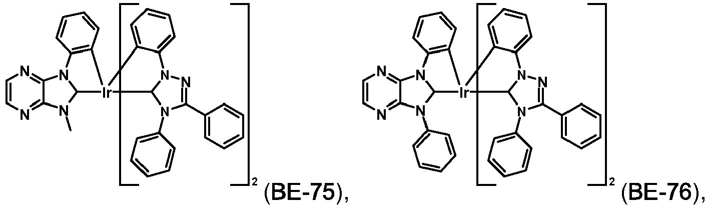

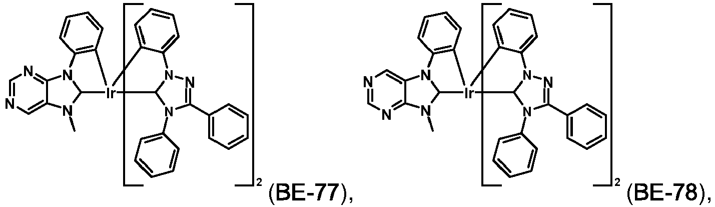

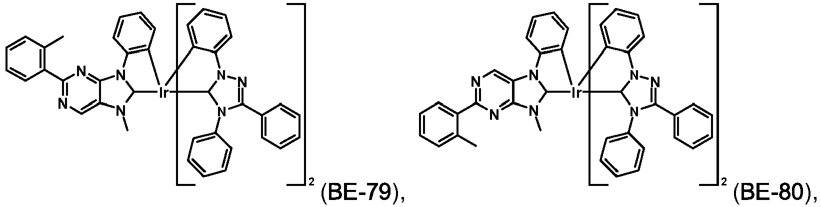

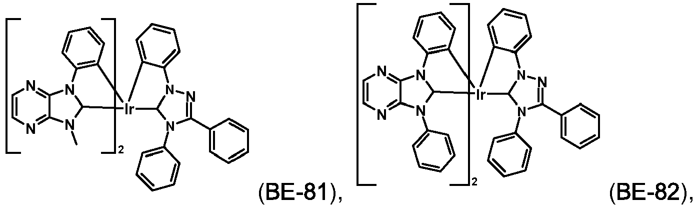

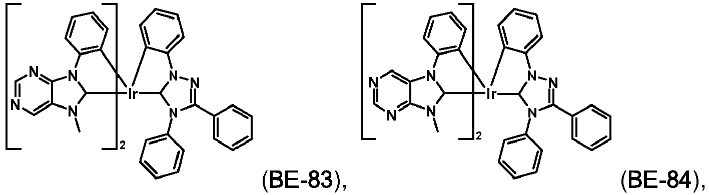

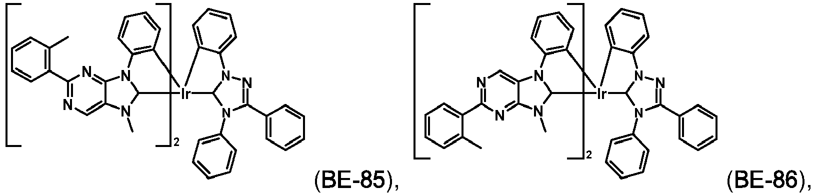

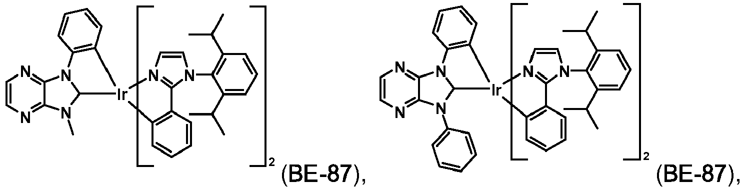

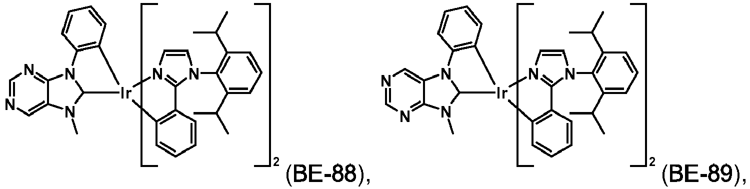

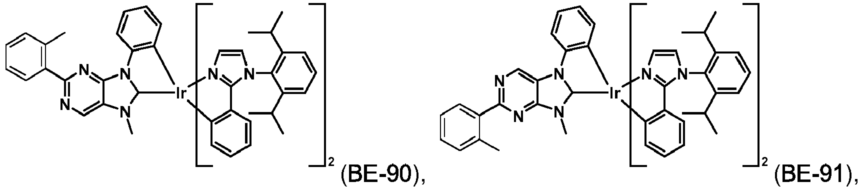

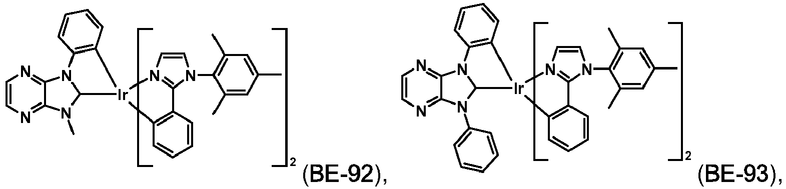

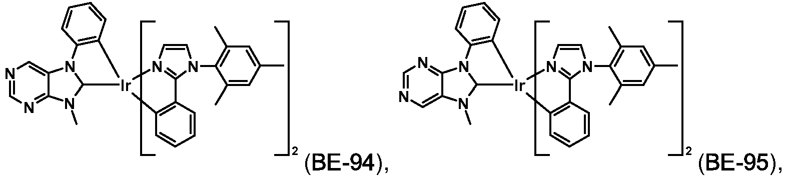

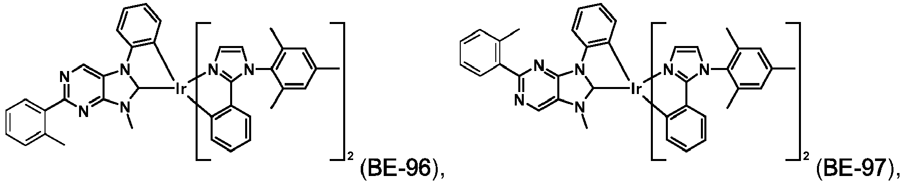

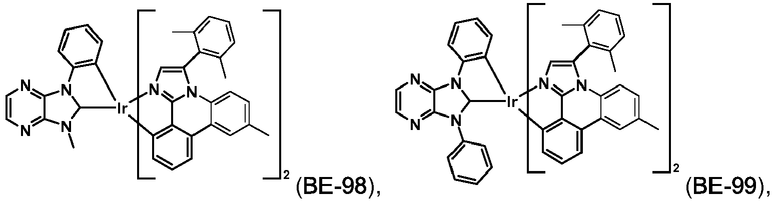

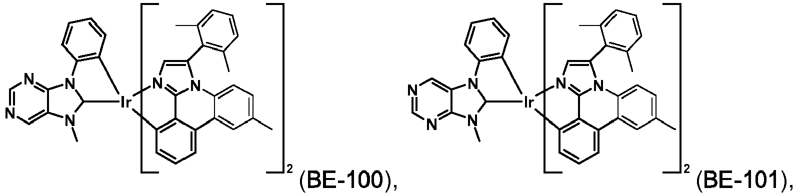

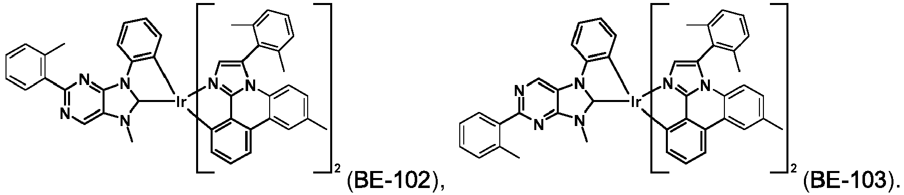

Further suitable non-carbene emitter materials are mentioned below:

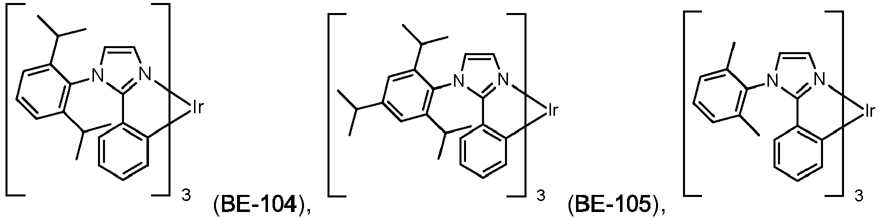

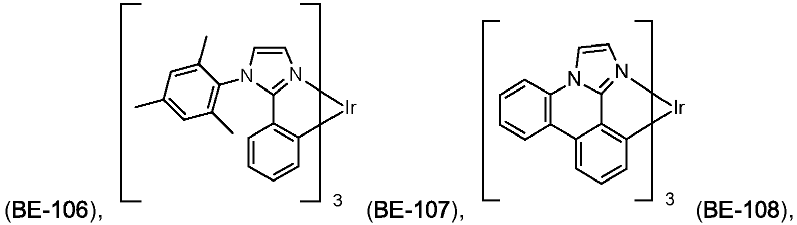

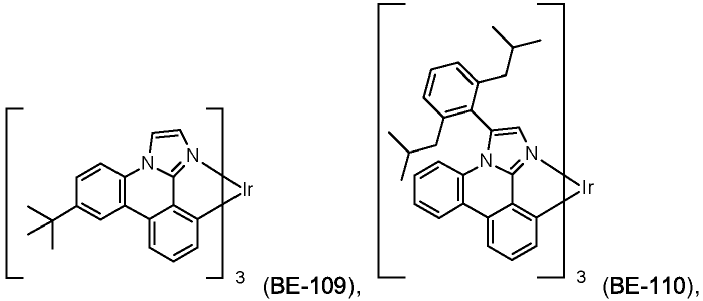

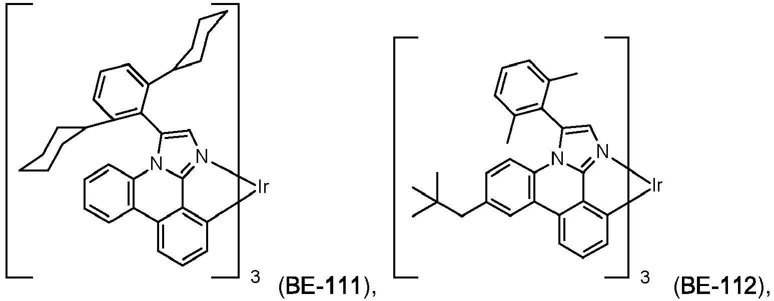

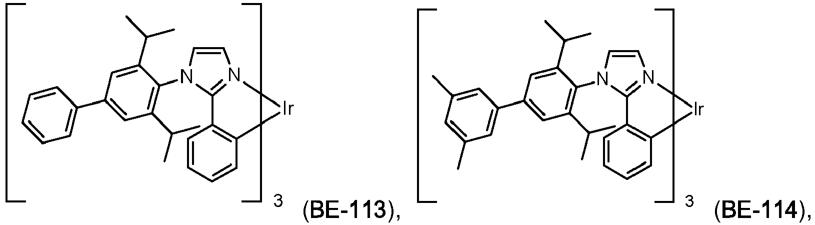

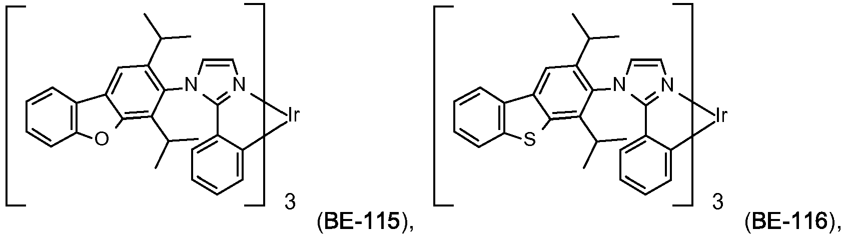

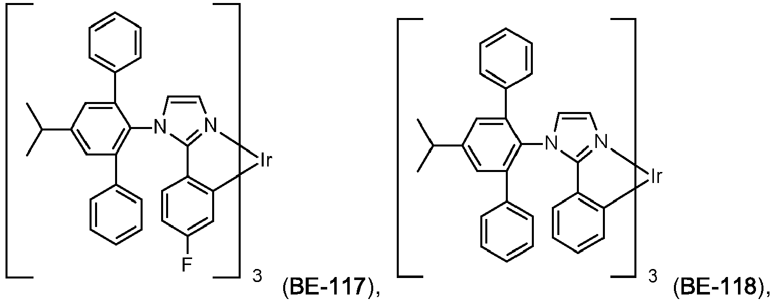

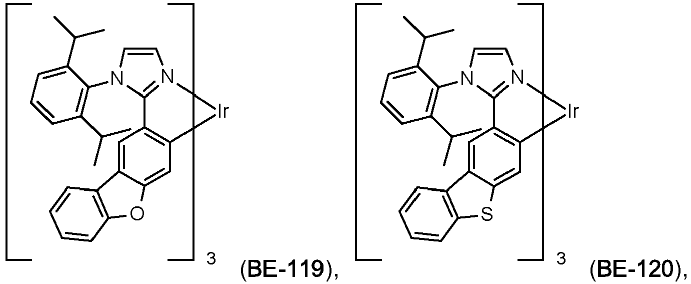

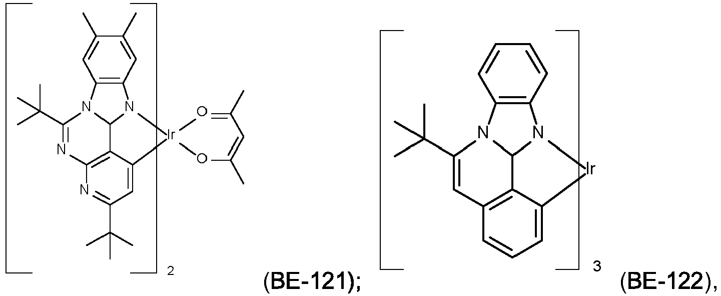

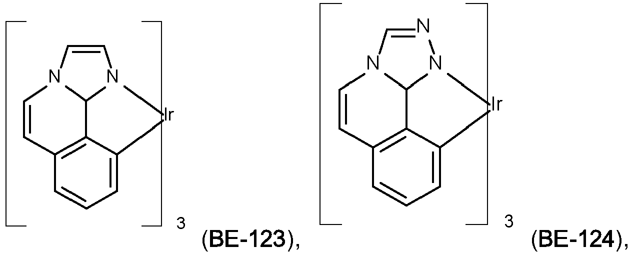

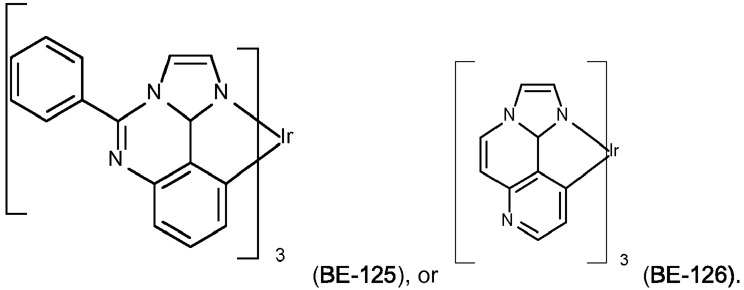

The compound of formula **IX** is more preferably a compound (**BE-1**), (**BE-2**), (**BE-7**), (**BE-12**), (**BE-16**), (**BE-64**), or (**BE-70**). The most preferred phosphorescent blue emitters are compounds (**BE-1**) and (**BE-12**).

The homoleptic metal-carbene complexes mentioned above may be present in the form of facial or meridional isomers, preference being given to the facial isomers.

Suitable carbene complexes of formula (**IX**) and their preparation process are, for example, described in WO2011/073149.

The compounds of the present invention can also be used as host (matrix material) for phosphorescent green emitters. Suitable phosphorescent green emitters are, for example, specified in the following publications: WO2006014599, WO20080220265, WO2009073245, WO2010027583, WO2010028151, US20110227049, WO2011090535, WO2012/08881, WO20100056669, WO20100118029, WO20100244004, WO2011109042, WO2012166608, US20120292600, EP2551933A1; US6687266, US20070190359, US20070190359, US20060008670; WO2006098460, US20110210316, WO2012053627; US6921915, US20090039776; JP2007123392 and European patent application no. 14180422.9.

Examples of suitable phosphorescent green emitters are shown below:

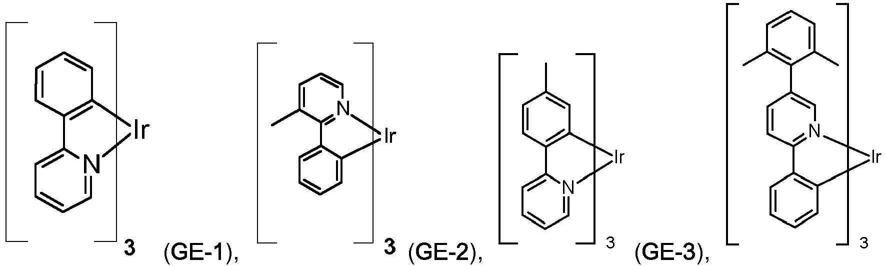

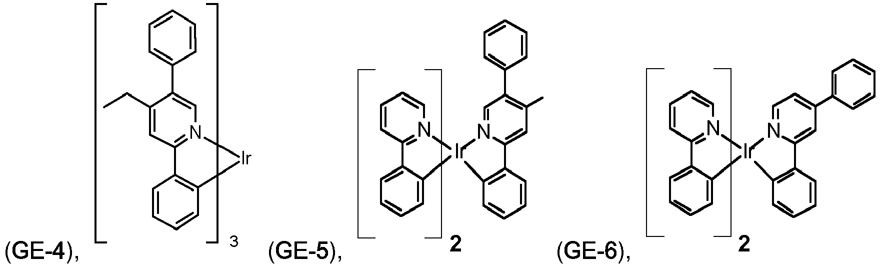

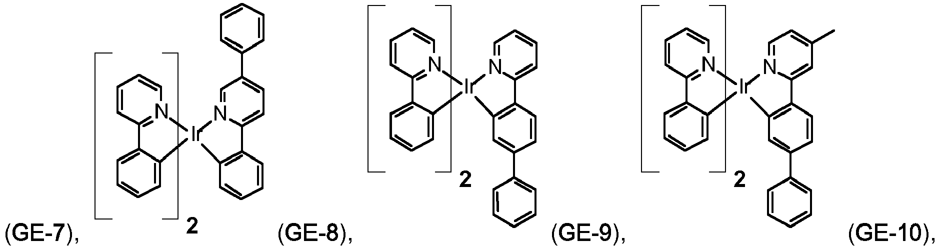

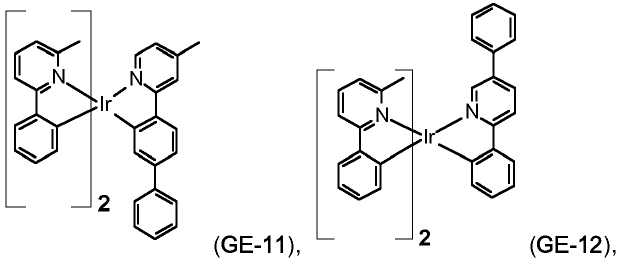

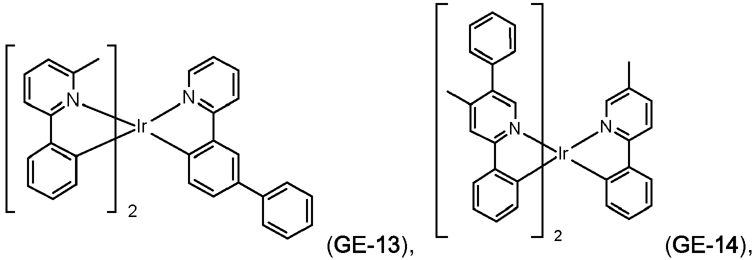

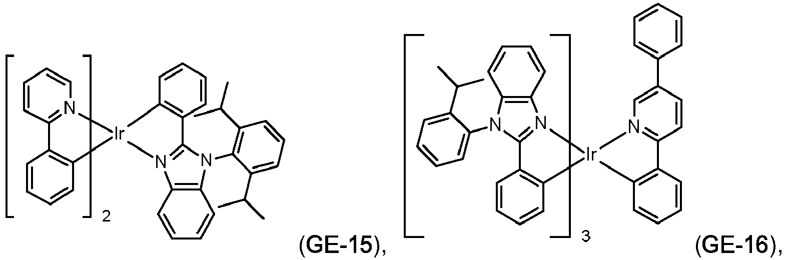

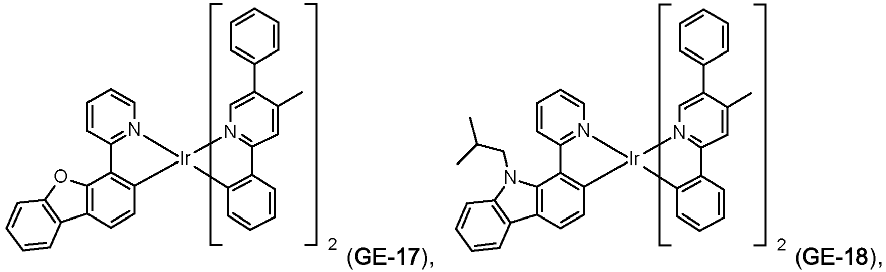

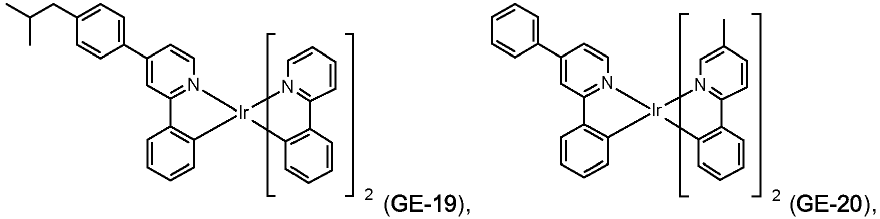

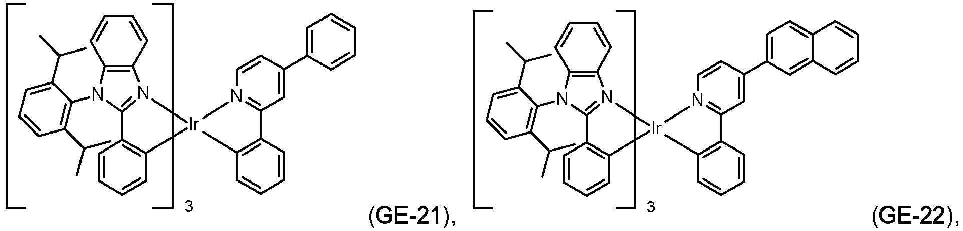

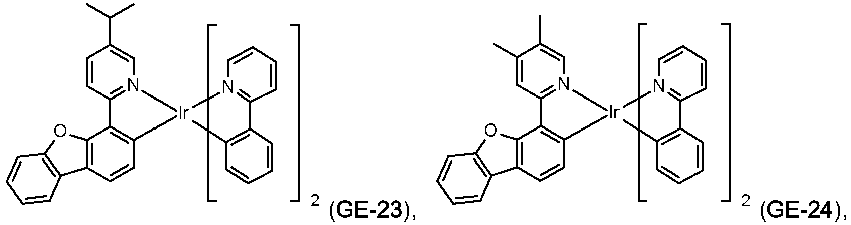

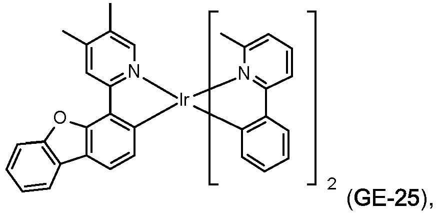

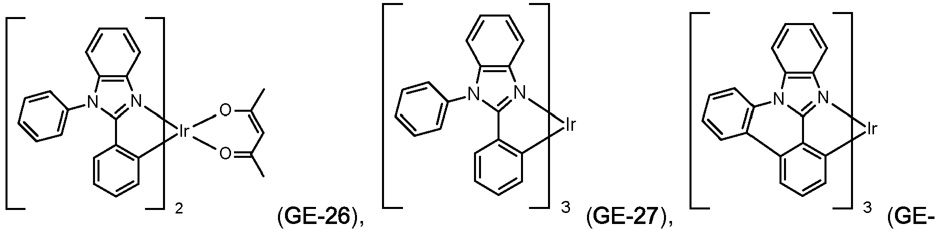

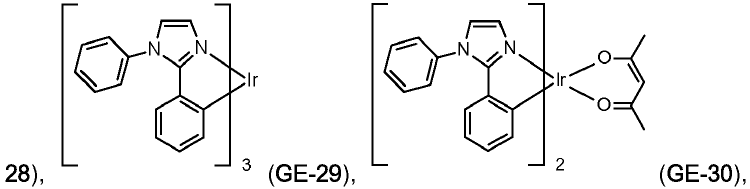

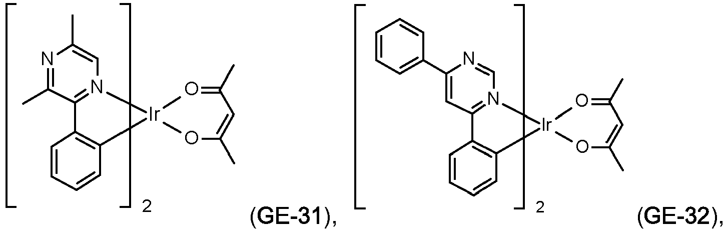

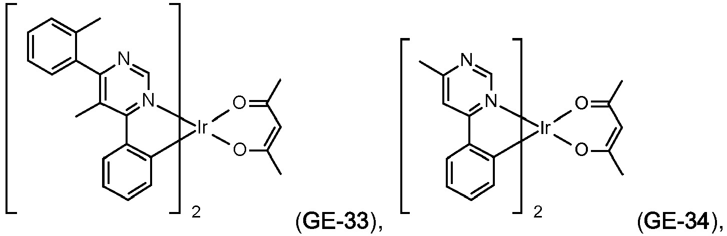

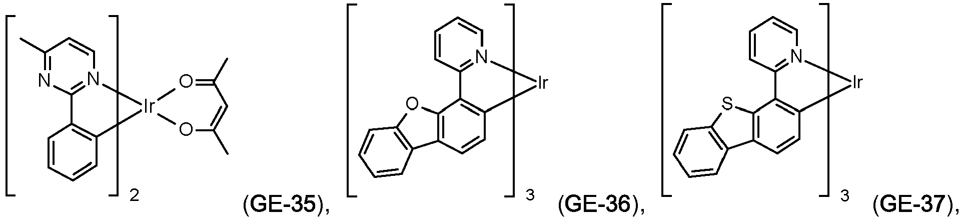

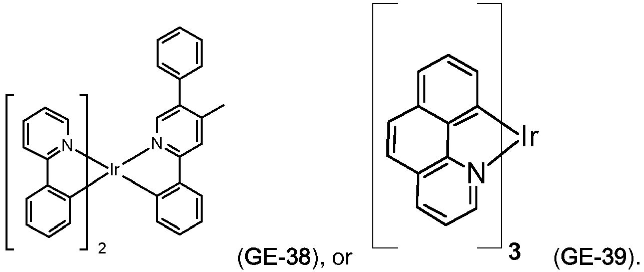

### Host (matrix) materials

The light-emitting layer may comprise further components in addition to the emitter material. For example, a fluroescent dye may be present in the light-emitting layer in order to alter the emission color of the emitter material. In addition - in a preferred embodiment - a matrix material can be used. This matrix material may be a polymer, for example poly(N-vinylcarbazole) or polysilane. The matrix material may, however, be a small molecule, for example 4,4'-N,N'-dicarbazolebiphenyl (CDP=CBP) or tertiary aromatic amines, for example TCTA.

In another preferred embodiment of the present invention, at least one compound of formula (I) is used as matrix material. Preferred compounds of formula (I) are mentioned above.

In a preferred embodiment, the light-emitting layer is formed from 2 to 40% by weight, preferably 5 to 35% by weight, of at least one of the aforementioned emitter materials and 60 to 98% by weight, preferably 75 to 95% by weight, of at least one of the aforementioned matrix materials - in one embodiment at least one compound of the formula (I) - where the sum total of the emitter material and of the matrix material adds up to 100% by weight. Preferred compounds of formula (I) are mentioned above.

In one embodiment of the present invention, at least one carbene complex is used - preferably beside the compound of formula (I) - as second host material in the light emitting layer of an OLED. Suitable carbene complexes are mentioned above and below. Particularly preferably, the light-emitting layer comprises a compound of formula (I), and two carbene complexes, wherein one carbene complex is the emitter and one carbene complex is a second host material, preferably **BE-1** and **HTM-1,** or **HTM-2.** In said embodiment, the light-emitting layer is formed from 2 to 40% by weight, preferably 5 to 35% by weight, of a carbene complex, **BE-1,** as emitter and 60 to 98% by weight, preferably 65 to 95% by weight, of a compound of the formula (I) and a second carbene complex, preferably **HTM-1,** or **HTM-2,** where the sum total of the carbene complexes and of the compound of formula (I) adds up to 100% by weight. Preferred compounds of formula (I) are mentioned above.

Suitable metal complexes for use together with the compounds of the formula (I) as matrix material in OLEDs (second host materials) are, for example, also carbene complexes as described in WO 2005/019373 A2, WO 2006/056418 A2, WO 2005/113704, WO 2007/115970, WO 2007/115981 and WO 2008/000727.

The at least one compound of formula (I) may be used as single host material or in combination with a second host material. Suitable second host materials are the host materials mentioned below. Further, in the case that the at least one compound of formula (I) is not used as host material, the host materials mentioned below are used as single host materials.

Further suitable host materials, which may be small molecules or (co)polymers of the small molecules mentioned, are specified in the following publications: WO2007108459 (H-1 to H-37), preferably H-20 to H-22 and H-32 to H-37, most preferably H-20, H-32, H-36, H-37, WO2008035571 A1 (Host 1 to Host 6), JP2010135467 (compounds 1 to 46 and Host-1 to Host-39 and Host-43), WO2009008100 compounds No.1 to No.67, preferably No.3, No.4, No.7 to No. 12, No.55, No.59, No. 63 to No.67, more preferably No. 4, No. 8 to No. 12, No. 55, No. 59, No.64, No.65, and No. 67, WO2009008099 compounds No. 1 to No. 110, WO2008140114 compounds 1-1 to 1-50, WO2008090912 compounds OC-7 to OC-36 and the polymers of Mo-42 to Mo-51, JP2008084913 H-1 to H-70, WO2007077810 compounds 1 to 44, preferably 1, 2, 4-6, 8, 19-22, 26, 28-30, 32, 36, 39-44, WO201001830 the polymers of monomers 1-1 to 1-9, preferably of 1-3, 1-7, and 1-9, WO2008029729 the (polymers of) compounds 1-1 to 1-36, WO20100443342 HS-1 to HS-101 and BH-1 to BH-17, preferably BH-1 to BH-17, JP2009182298 the (co)polymers based on the monomers 1 to 75, JP2009170764, JP2009135183 the (co)polymers based on the monomers 1-14, WO2009063757 preferably the (co)polymers based on the monomers 1-1 to 1-26, WO2008146838 the compounds a-1 to a-43 and 1-1 to 1-46, JP2008207520 the (co)polymers based on the monomers 1-1 to 1-26, JP2008066569 the (co)polymers based on the monomers 1-1 to 1-16, WO2008029652 the (co)polymers based on the monomers 1-1 to 1-52, WO2007114244 the (co)polymers based on the monomers 1-1 to 1-18, JP2010040830 the compounds HA-1 to HA-20, HB-1 to HB-16, HC-1 to HC-23 and the (co)polymers based on the monomers HD-1 to HD-12, JP2009021336, WO2010090077 the compounds 1 to 55, WO2010079678 the compounds H1 to H42, WO2010067746, WO2010044342 the compounds HS-1 to HS-101 and Poly-1 to Poly-4, JP2010114180 the compounds PH-1 to PH-36, US2009284138 the compounds 1 to 111 and H1 to H71, WO2008072596 the compounds 1 to 45, JP2010021336 the compounds H-1 to H-38, preferably H-1, WO2010004877 the compounds H-1 to H-60, JP2009267255 the compounds 1-1 to 1-105, WO2009104488 the compounds 1-1 to 1-38, WO2009086028, US2009153034, US2009134784, WO2009084413 the compounds 2-1 to 2-56, JP2009114369 the compounds 2-1 to 2-40, JP2009114370 the compounds 1 to 67, WO2009060742 the compounds 2-1 to 2-56, WO2009060757 the compounds 1-1 to 1-76, WO2009060780 the compounds 1-1 to 1-70, WO2009060779 the compounds 1-1 to 1-42, WO2008156105 the compounds 1 to 54, JP2009059767 the compounds 1 to 20, JP2008074939 the compounds 1 to 256, JP2008021687 the compounds 1 to 50, WO2007119816 the compounds 1 to 37, WO2010087222 the compounds H-1 to H-31, WO2010095564 the compounds HOST-1 to HOST-61, WO2007108362, WO2009003898, WO2009003919, WO2010040777, US2007224446, WO06128800, WO2012014621, WO2012105310, WO2012/130709 and European patent applications EP12175635.7 and EP12185230.5 and EP12191408.9 (in particular page 25 to 29 of EP12191408.9).

The above-mentioned small molecules are more preferred than the above-mentioned (co)polymers of the small molecules.

Further suitable host materials, are described in WO2011137072 (for example, 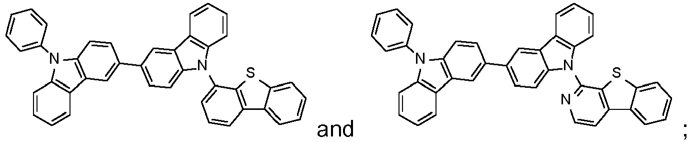 best results are achieved if said compounds are combined with 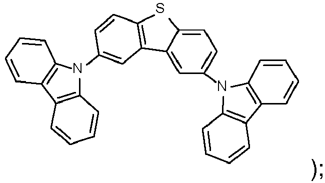 WO2012048266 (for example, 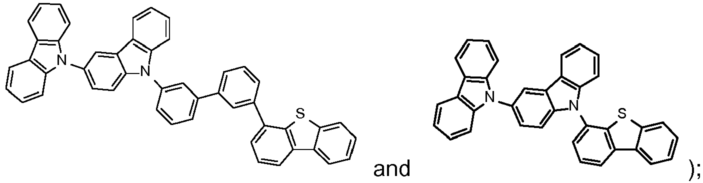 WO2012162325 (for example, 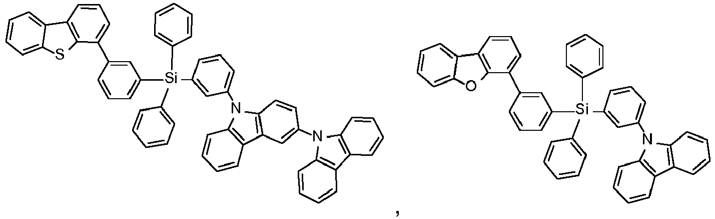 and 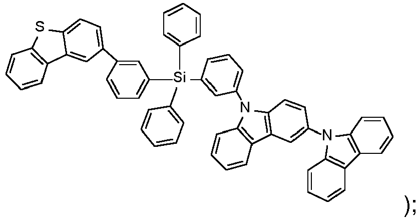 and EP2551932 (for example,

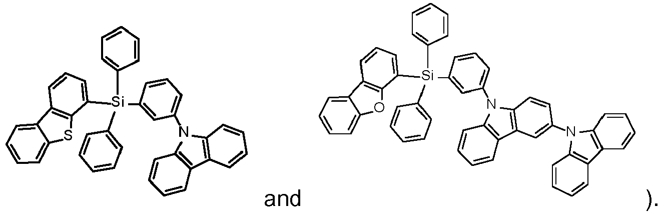

In a particularly preferred embodiment, one or more compounds of the general formula (X) specified hereinafter are used as host material. 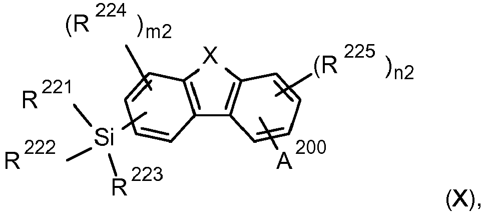 wherein
X is NR, S, O or PR;
R is aryl, heteroaryl, alkyl, cycloalkyl, or heterocycloalkyl;
A²⁰⁰ is -NR²⁰⁶R²⁰⁷, -P(O)R²⁰⁸R²⁰⁹, -PR²¹⁰R²¹¹, -S(O)₂R²¹², -S(O)R²¹³, -SR²¹⁴, or -OR²¹⁵;
R²²¹, R²²² and R²²³ are independently of each other aryl, heteroaryl, alkyl, cycloalkyl, or heterocycloalkyl, wherein at least on of the groups R²²¹, R²²², or R²²³ is aryl, or heteroaryl;
R²²⁴ and R²²⁵ are independently of each other alkyl, cycloalkyl, heterocycloalkyl, aryl, heteroaryl, a group A²⁰⁰, or a group having donor, or acceptor characteristics;
n2 and m2 are independently of each other 0, 1, 2, or 3;
R²⁰⁶ and R²⁰⁷ form together with the nitrogen atom a cyclic residue having 3 to 10 ring atoms, which can be unsubstituted, or which can be substituted with one, or more substituents selected from alkyl, cycloalkyl, heterocycloalkyl, aryl, heteroaryl and a group having donor, or acceptor characteristics; and/or which can be annulated with one, or more further cyclic residues having 3 to 10 ring atoms, wherein the annulated residues can be unsubstituted, or can be substituted with one, or more substituents selected from alkyl, cycloalkyl, heterocycloalkyl, aryl, heteroaryl and a group having donor, or acceptor characteristics; and
R²⁰⁸, R²⁰⁹, R²¹⁰, R²¹¹, R²¹² R²¹³ R²¹⁴ und R²¹⁵ are independently of each other aryl, heteroaryl, alkyl, cycloalkyl, or heterocycloalkyl. Compounds of formula X, such as, for example, 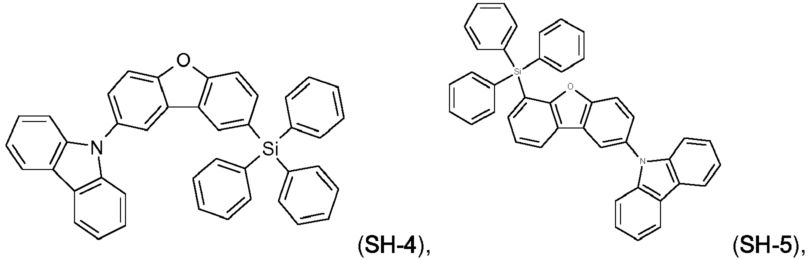 or 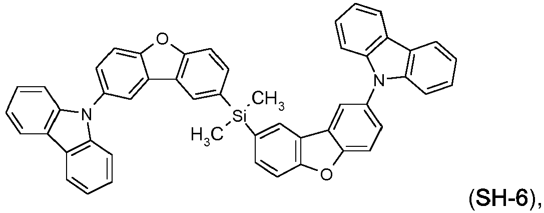 are described in WO2010079051 (in particular pages on 19 to 26 and in tables on pages 27 to 34, pages 35 to 37 and pages 42 to 43).

Additional host materials on basis of dibenzofurane are, for example, described in US2009066226, EP1885818B1, EP1970976, EP1998388, EP2034538 and European patent application no. 14160197.1. Examples of particularly preferred host materials are shown below:

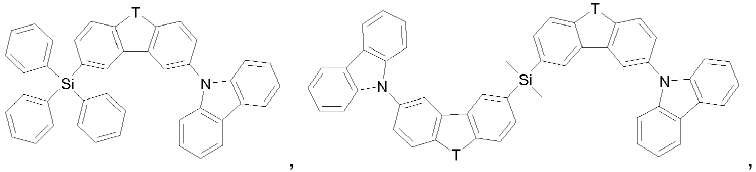

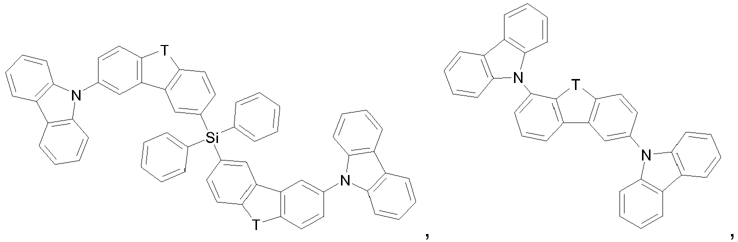

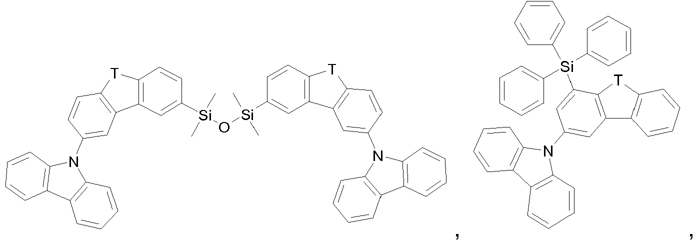

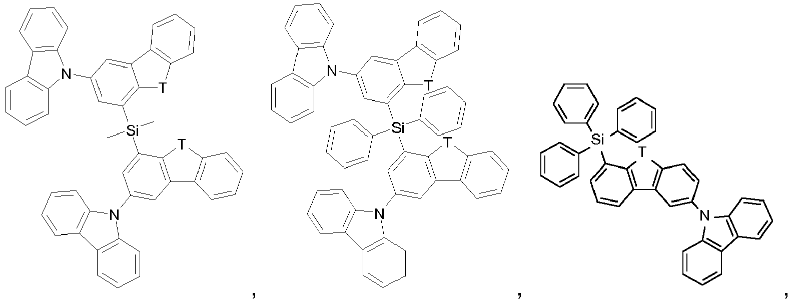

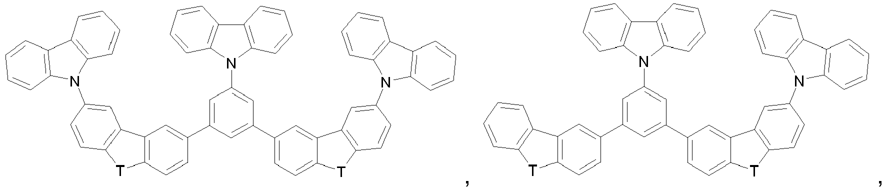

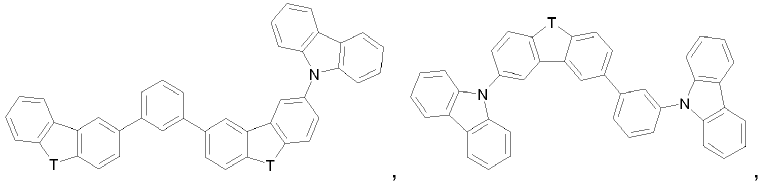

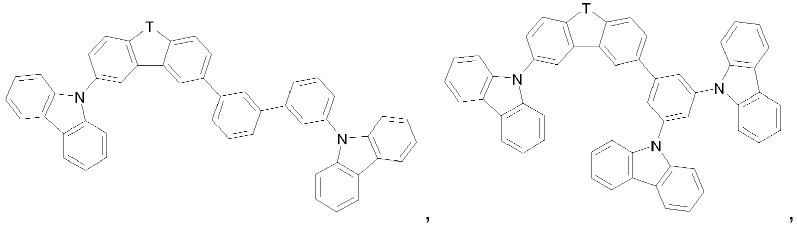

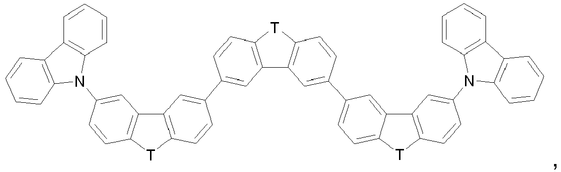

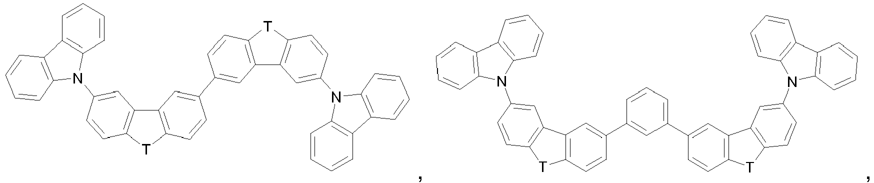

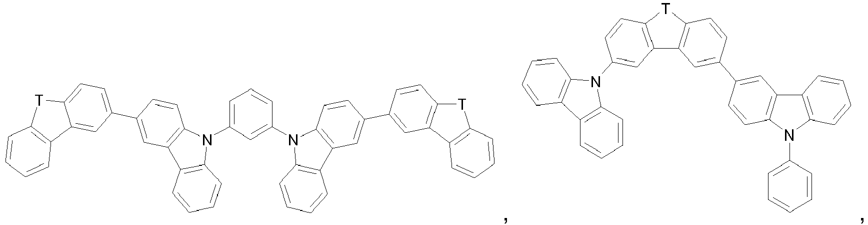

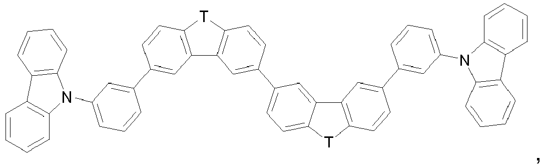

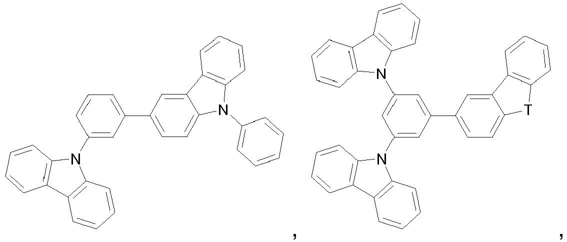

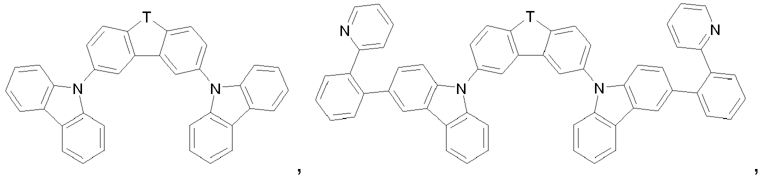

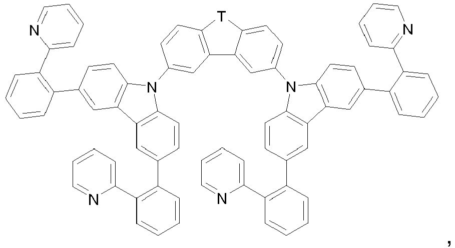

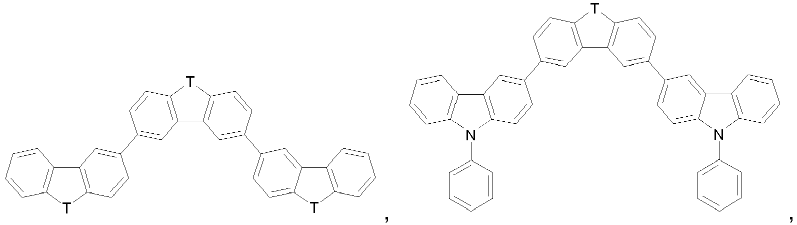

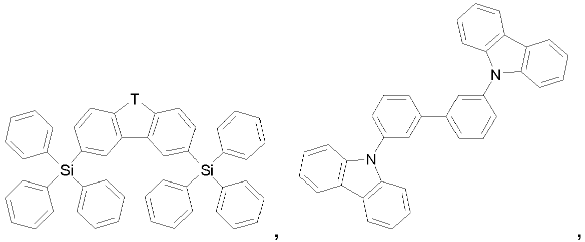

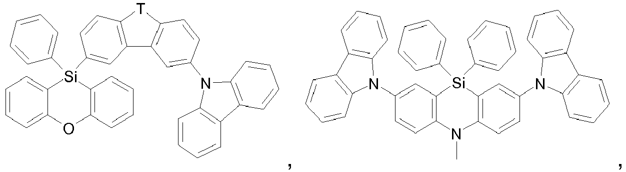

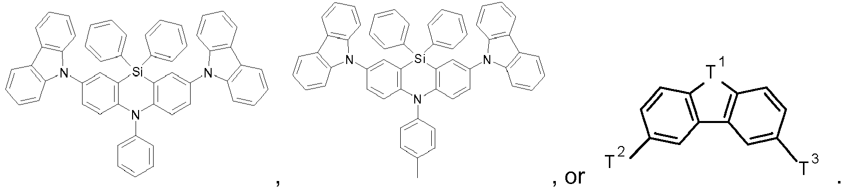

In the above-mentioned compounds T is O, or S, preferably O. If T occurs more than one time in a molecule, all groups T have the same meaning. T¹ is O, or S, preferably O. T¹ and T² are independently of each other 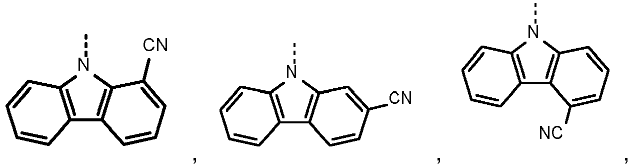 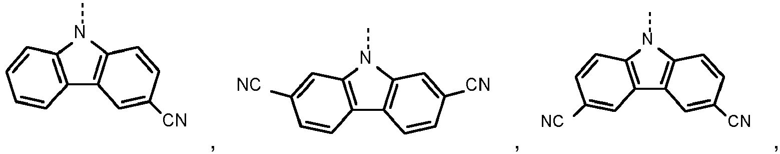 or 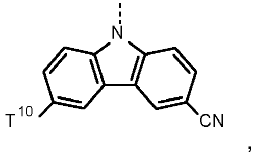 wherein T¹⁰ is a C₁-C₂₅alkyl group.

Compounds 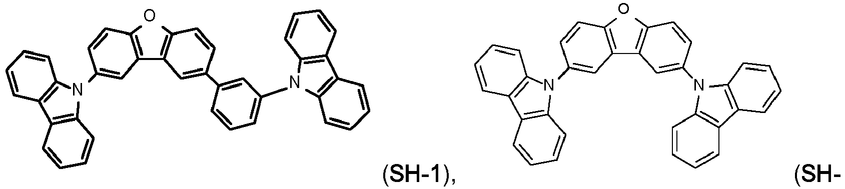 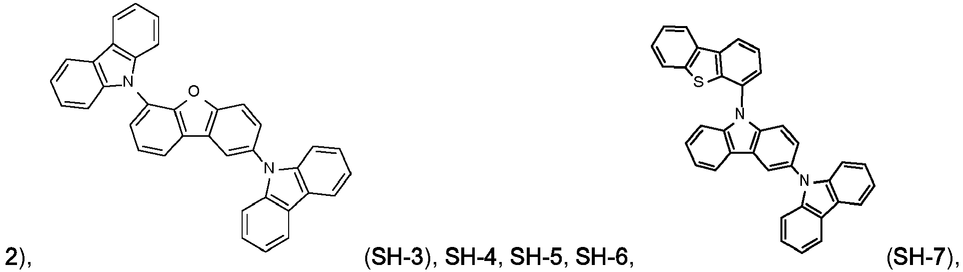 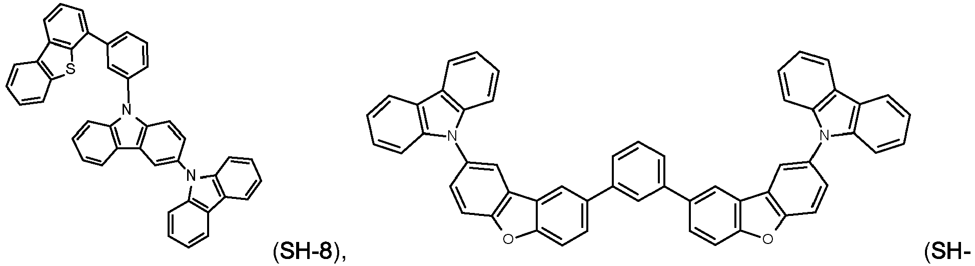 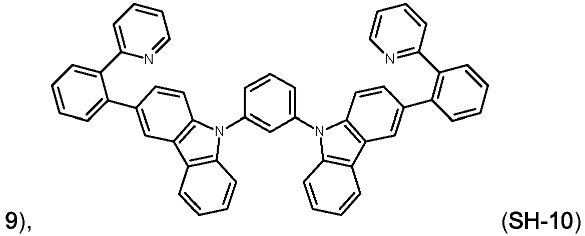 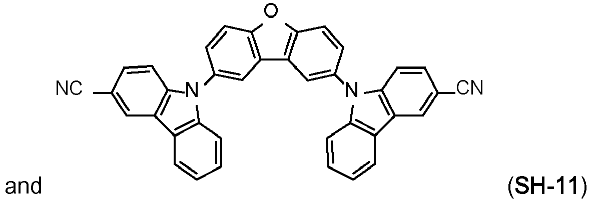 are most preferred.

### Hole/exciton blocking layer (f):

Blocking layers may be used to reduce the number of charge carriers (electrons or holes) and/or excitons that leave the emissive layer. The hole blocking layer may be disposed between the emitting layer (e) and electron transport layer (g), to block holes from leaving layer (e) in the direction of electron transport layer (g). Blocking layers may also be used to block excitons from diffusing out of the emissive layer.

Suitable hole blocker materials are the compounds of formula (I). However, in the case that further hole blocker materials are used in the hole blocking layer of the inventive OLED or in the case that the compounds of formula (I) are not used as hole blocker materials in the hole blocking layer, the following hole blocker materials are for example suitable as hole blocker materials in the inventive OLED.

Additional hole blocker materials typically used in OLEDs are 2,6-bis(N-carbazolyl)pyridine (mCPy), 2,9-dimethyl-4,7-diphenyl-1,10-phenanthroline (bathocuproin, (BCP)), bis(2-methyl-8-quinolinato)-4-phenylphenylato)aluminum(III) (BAIq), phenothiazine S,S-dioxide derivates and 1,3,5-tris(N-phenyl-2-benzylimidazolyl)benzene) (TPBI), TPBI also being suitable as electron-transport material. Further suitable hole blockers and/or electron conductor materials are 2,2',2"-(1,3,5-benzenetriyl)tris(1-phenyl-1-H-benzimidazole), 2-(4-biphenylyl)-5-(4-tert-butylphenyl)-1,3,4-oxadiazole, 8-hydroxyquinolinolatolithium, 4-(naphthalen-1-yl)-3,5-diphenyl-4H-1,2,4-triazole, 1,3-bis[2-(2,2'-bipyridin-6-yl)-1,3,4-oxadiazo-5-yl]benzene, 4,7-diphenyl-1,10-phenanthroline, 3-(4-biphenylyl)-4-phenyl-5-tert-butylphenyl-1,2,4-triazole, 6,6'-bis[5-(biphenyl-4-yl)-1,3,4-oxadiazo-2-yl]-2,2'-bipyridyl, 2-phenyl-9,10-di(naphthalene-2-yl)anthracene, 2,7-bis[2-(2,2'-bipyridin-6-yl)-1,3,4-oxadiazo-5-yl]-9,9-dimethylfluorene, 1,3-bis[2-(4-tert-butylphenyl)-1,3,4-oxadiazo-5-yl]benzene, 2-(naphthalene-2-yl)-4,7-diphenyl-1,10-phenanthroline, tris(2,4,6-trimethyl-3-(pyridin-3-yl)phenyl)borane, 2,9-bis(naphthalene-2-yl)-4,7-diphenyl-1,10-phenanthroline, 1-methyl-2-(4-(naphthalene-2-yl)phenyl)-1H-imidazo[4,5-f][1,10]-phenanthroline. In a further embodiment, it is possible to use compounds which comprise aromatic or heteroaromatic rings joined via groups comprising carbonyl groups, as disclosed in WO2006/100298, disilyl compounds selected from the group consisting of disilylcarbazoles, disilylbenzofurans, disilylbenzothiophenes, disilylbenzophospholes, disilylbenzothiophene S-oxides and disilylbenzothiophene S,S-dioxides, as specified, for example, in PCT applications WO2009/003919 and WO2009003898 and disilyl compounds as disclosed in WO2008/034758, as a blocking layer for holes/excitons (f).

In another preferred embodiment compounds (**SH-1**), (**SH-2**), (**SH-3**), **SH-4, SH-5, SH-6,** (**SH-7**), (**SH-8**), (**SH-9**), (**SH-10**) and (**SH-11**) may be used as hole/exciton blocking materials.

### Electron transport layer (g):

Electron transport layer may include a material capable of transporting electrons. Electron transport layer may be intrinsic (undoped), or doped. Doping may be used to enhance conductivity.

Suitable electron transport materials are the compounds of formula (I). However, in the case that further electron transport materials are used in the electron transport layer of the inventive OLED or in the case that the compounds of formula (I) are not used as electron transport materials in the electron transport layer, the following electron transport materials are for example suitable as electron transport materials in the inventive OLED.

Suitable electron-transporting materials for layer (g) of the inventive OLEDs comprise metals chelated with oxinoid compounds, such as tris(8-hydroxyquinolato)aluminum (Alq₃), compounds based on phenanthroline such as 2,9-dimethyl-4,7-diphenyl-1,10-phenanthroline (DDPA = BCP), 4,7-diphenyl-1,10-phenanthroline (Bphen), 2,4,7,9-tetraphenyl-1,10-phenanthroline, 4,7-diphenyl-1,10-phenanthroline (DPA) or phenanthroline derivatives disclosed in EP1786050, in EP1970371, or in EP1097981, and azole compounds such as 2-(4-biphenylyl)-5-(4-t-butylphenyl)-1,3,4-oxadiazole (PBD) and 3-(4-biphenylyl)-4phenyl-5-(4-t-butylphenyl)-1,2,4-triazole (TAZ).

It is likewise possible to use mixtures of at least two materials in the electron-transporting layer, in which case at least one material is electron-conducting. Preferably, in such mixed electron-transport layers, at least one phenanthroline compound is used, preferably BCP, or at least one pyridine compound according to the formula (**VIII**) below, preferably a compound of the formula (**VIIIaa**) below. More preferably, in mixed electron-transport layers, in addition to at least one phenanthroline compound, alkaline earth metal or alkali metal hydroxyquinolate complexes, for example Liq, are used. Suitable alkaline earth metal or alkali metal hydroxyquinolate complexes are specified below (formula **VII**). Reference is made to WO2011/157779.

The electron-transport layer may also be electronically doped in order to improve the transport properties of the materials used, in order firstly to make the layer thicknesses more generous (avoidance of pinholes/short circuits) and in order secondly to minimize the operating voltage of the device. Electronic doping is known to those skilled in the art and is disclosed, for example, in W. Gao, A. Kahn, J. Appl. Phys., Vol. 94, No. 1, 1 July 2003 (p-doped organic layers); A. G. Werner, F. Li, K. Harada, M. Pfeiffer, T. Fritz, K. Leo, Appl. Phys. Lett., Vol. 82, No. 25, 23 June 2003 and Pfeiffer et al., Organic Electronics 2003, 4, 89 - 103 and K. Walzer, B. Maennig, M. Pfeiffer, K. Leo, Chem. Soc. Rev. 2007, 107, 1233. For example, it is possible to use mixtures which lead to electrical n-doping of the electron-transport layer. n-Doping is achieved by the addition of reducing materials. These mixtures may, for example, be mixtures of the abovementioned electron transport materials with alkali/alkaline earth metals or alkali/alkaline earth metal salts, for example Li, Cs, Ca, Sr, Cs₂CO₃, with alkali metal complexes, for example 8-hydroxyquinolatolithium (Liq), and with Y, Ce, Sm, Gd, Tb, Er, Tm, Yb, Li₃N, Rb₂CO₃, dipotassium phthalate, W(hpp)₄ from EP1786050, or with compounds described in EP1837926B1, EP1837927, EP2246862 and WO2010132236.

In a preferred embodiment, the electron-transport layer comprises at least one compound of the general formula (**VII**) 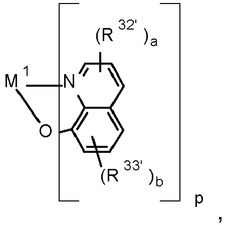 in which
R^{32'} and R^{33'} are each independently F, C₁-C₈-alkyl, or C₆-C₁₄-aryl, which is optionally substituted by one or more C₁-C₈-alkyl groups, or
two R^{32'} and/or R^{33'} substituents together form a fused benzene ring which is optionally substituted by one or more C₁-C₈-alkyl groups;
a and b are each independently 0, or 1, 2 or 3,
M¹ is an alkaline metal atom or alkaline earth metal atom,
p is 1 when M¹ is an alkali metal atom, p is 2 when M¹ is an earth alkali metal atom.

A very particularly preferred compound of the formula (**VII**) is 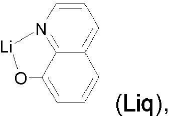 which may be present as a single species, or in other forms such as Li_{g}Q_{g} in which g is an integer, for example Li₆Q₆. Q is an 8-hydroxyquinolate ligand or an 8-hydroxyquinolate derivative.

In a further preferred embodiment, the electron-transport layer comprises at least one compound of the formula (**VIII**), 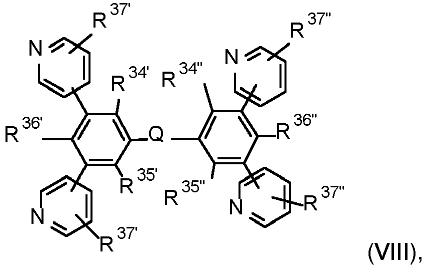 in which
R^{34"} R^{35"} R^{36"} R^{37"} R^{34'} R^{35'}, R^{36'} and R^{37'} are each independently H, C₁-C₁₈-alkyl, C₁-C₁₈-alkyl which is substituted by E' and/or interrupted by D', C₆-C₂₄-aryl, C₆-C₂₄-aryl which is substituted by G', C₂-C₂₀-heteroaryl or C₂-C₂₀-heteroaryl which is substituted by G',
Q is an arylene or heteroarylene group, each of which is optionally substituted by G';
D' is -CO-; -COO-; -S-; -SO-; -SO₂-; -O-; -NR^{40'}-; -SiR^{45'}R^{46'}-; -POR^{47'}-; -CR^{38'}=CR^{39'}-; or -C≡C-; E' is -OR^{44'}; -SR^{44'}; -NR^{40'}R^{41'}; -COR^{43'}; -COOR^{42'}; -CONR^{40'}R^{41'}; -CN; or F;
G' is E', C₁-C₁₈-alkyl, C₁-C₁₈-alkyl which is interrupted by D', C₁-C₁₈-perfluoroalkyl, C₁-C₁₈-alkoxy, or C₁-C₁₈-alkoxy which is substituted by E' and/or interrupted by D', in which R^{38'} and R^{39'} are each independently H, C₆-C₁₈-aryl; C₆-C₁₈-aryl which is substituted by C₁-C₁₈-alkyl or C₁-C₁₋alkoxy C₁-C₁₈-alkyl; or C₁-C₁₈-alkyl which is interrupted by -O-;
R^{40'} and R^{41'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-; or R^{40'} and R^{41'} together form a 6-membered ring;
R^{42'} and R^{43'} are each independently C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R^{44'} is C₆-C₁₈-aryl; C₆-C₁₈-aryl which is substituted by C₁-C₁₈-alkyl or C₁-C₁₈-alkoxy; C₁-C₁₈-alkyl; or C₁-C₁₈-alkyl which is interrupted by -O-,
R^{45'} and R^{46'} are each independently C₁-C₁₈-alkyl, C₆-C₁₈-aryl or C₆-C₁₈-aryl which is substituted by C₁-C₁₈-alkyl,
R^{47'} is C₁-C₁₈-alkyl, C₆-C₁₈-aryl or C₆-C₁₈-aryl which is substituted by C₁-C₁₈-alkyl.

Preferred compounds of the formula (**VIII**) are compounds of the formula (**VIIIa**) 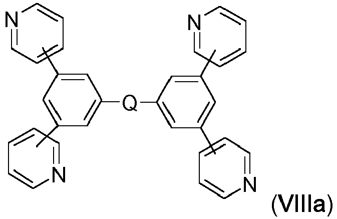 in which Q is: 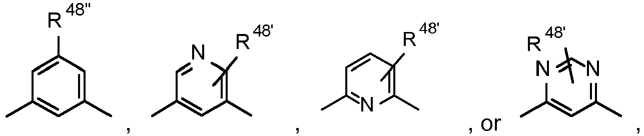 R^{48'} is H or C₁-C₁₈-alkyl and
R^{48"} is H, C₁-C₁₈-alkyl or

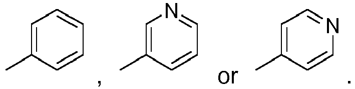

Particular preference is given to a compound of the formula 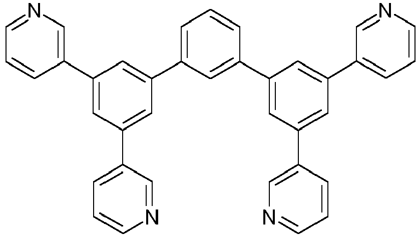 (ETM-2).

In a further, very particularly preferred embodiment, the electron-transport layer comprises a compound **Liq** and a compound **ETM-2.**

In a preferred embodiment, the electron-transport layer comprises the compound of the formula (**VII**) in an amount of 99 to 1 % by weight, preferably 75 to 25% by weight, more preferably about 50% by weight, where the amount of the compounds of the formulae (**VII**) and the amount of the compounds of the formulae (**VIII**) adds up to a total of 100% by weight.

The preparation of the compounds of the formula (**VIII**) is described in J. Kido et al., Chem. Commun. (2008) 5821-5823, J. Kido et al., Chem. Mater. 20 (2008) 5951-5953 and JP2008/127326, or the compounds can be prepared analogously to the processes disclosed in the aforementioned documents.

It is likewise possible to use mixtures of alkali metal hydroxyquinolate complexes, preferably Liq, and dibenzofuran compounds in the electron-transport layer. Reference is made to WO2011/157790. Dibenzofuran compounds **A-1 to A-36** and **B-1** to **B-22** described in WO2011/157790 are preferred, wherein dibenzofuran compound 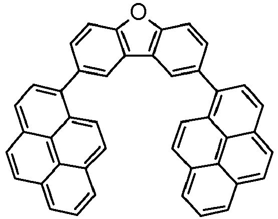 (**A-10; = ETM-1**) is most preferred.

In a preferred embodiment, the electron-transport layer comprises **Liq** in an amount of 99 to 1 % by weight, preferably 75 to 25% by weight, more preferably about 50% by weight, where the amount of **Liq** and the amount of the dibenzofuran compound(s), especially **ETM-1,** adds up to a total of 100% by weight.

In a preferred embodiment, the electron-transport layer comprises at least one phenanthroline derivative and/or pyridine derivative.

In a further preferred embodiment, the electron-transport layer comprises at least one phenanthroline derivative and/or pyridine derivative and at least one alkali metal hydroxyquinolate complex.

In a further preferred embodiment, the electron-transport layer comprises at least one of the dibenzofuran compounds **A-1** to **A-36** and **B-1** to **B-22** described in WO2011/157790, especially **ETM-1.**

In a further preferred embodiment, the electron-transport layer comprises a compound described in WO2012/111462, WO2012/147397, WO2012014621, such as, for example, a compound of formula 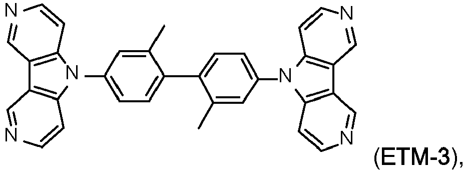 US2012/0261654 , such as, for example, a compound of formula 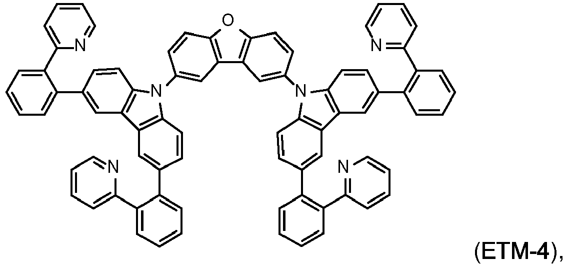 and WO2012/115034, such as for example, such as, for example, a compound of formula

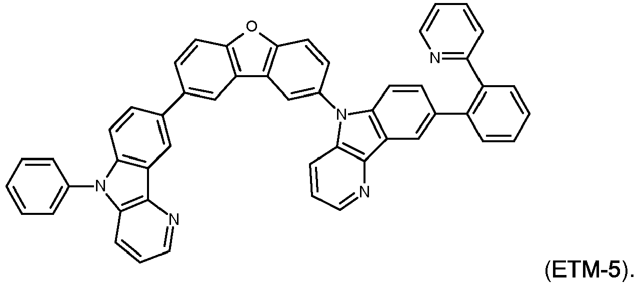

### Electron injection layer (h):

The electron injection layer may be any layer that improves the injection of electrons into an adjacent organic layer. Lithium-comprising organometallic compounds such as 8-hydroxyquinolatolithium (Liq), CsF, NaF, KF, Cs₂CO₃ or LiF may be applied between the electron transport layer (g) and the cathode (i) as an electron injection layer (h) in order to reduce the operating voltage.

### Cathode (i):

The cathode (i) is an electrode which serves to introduce electrons or negative charge carriers. The cathode may be any metal or nonmetal which has a lower work function than the anode. Suitable materials for the cathode are selected from the group consisting of alkali metals of group 1, for example Li, Cs, alkaline earth metals of group 2, metals of group 12 of the Periodic Table of the Elements, comprising the rare earth metals and the lanthanides and actinides. In addition, metals such as aluminum, indium, calcium, barium, samarium and magnesium, and combinations thereof, may be used.

In general, the different layers, if present, have the following thicknesses:
anode (a): 500 to 5000 Å (ångström), preferably 1000 to 2000 Å;
hole injection layer (b): 50 to 1000 Å, preferably 200 to 800 Å,
hole-transport layer (c): 50 to 1000 Å, preferably 100 to 800 Å,
exciton blocking layer (d): 10 to 500 Å, preferably 50 to 100 Å,
light-emitting layer (e): 10 to 1000 Å, preferably 50 to 600 Å,
hole/ exciton blocking layer (f): 10 to 500 Å, preferably 50 to 100 Å,
electron-transport layer (g): 50 to 1000 Å, preferably 200 to 800 Å,
electron injection layer (h): 10 to 500 Å, preferably 20 to 100 Å,
cathode (i): 200 to 10 000 Å, preferably 300 to 5000 Å.

The person skilled in the art is aware (for example on the basis of electrochemical studies) of how suitable materials have to be selected. Suitable materials for the individual layers are known to those skilled in the art and are disclosed, for example, in WO 00/70655.

In addition, it is possible that some of the layers used in the inventive OLED have been surface-treated in order to increase the efficiency of charge carrier transport. The selection of the materials for each of the layers mentioned is preferably determined by obtaining an OLED with a high efficiency and lifetime.

The inventive OLED can be produced by methods known to those skilled in the art. In general, the inventive OLED is produced by successive vapor deposition of the individual layers onto a suitable substrate. Suitable substrates are, for example, glass, inorganic semiconductors or polymer films. For vapor deposition, it is possible to use customary techniques, such as thermal evaporation, chemical vapor deposition (CVD), physical vapor deposition (PVD) and others. In an alternative process, the organic layers of the OLED can be applied from solutions or dispersions in suitable solvents, employing coating techniques known to those skilled in the art.

Use of the compounds of the formula (I) in at least one layer of the OLED, preferably in the light-emitting layer (preferably as a matrix material), charge transport layer and/or in the charge/exciton blocking layer makes it possible to obtain OLEDs with high efficiency and with low use and operating voltage. Frequently, the OLEDs obtained by the use of the compounds of the formula (I) additionally have long lifetimes. The efficiency of the OLEDs can additionally be improved by optimizing the other layers of the OLEDs. For example, high-efficiency cathodes such as Ca or Ba, if appropriate in combination with an intermediate layer of LiF, can be used. Moreover, additional layers may be present in the OLEDs in order to adjust the energy level of the different layers and to facilitate electroluminescence.

The OLEDs may further comprise at least one second light-emitting layer. The overall emission of the OLEDs may be composed of the emission of the at least two light-emitting layers and may also comprise white light.

The OLEDs can be used in all apparatus in which electroluminescence is useful. Suitable devices are preferably selected from stationary and mobile visual display units and illumination units. Stationary visual display units are, for example, visual display units of computers, televisions, visual display units in printers, kitchen appliances and advertising panels, illuminations and information panels. Mobile visual display units are, for example, visual display units in cellphones, tablet PCs, laptops, digital cameras, MP3 players, vehicles and destination displays on buses and trains. Further devices in which the inventive OLEDs can be used are, for example, keyboards; items of clothing; furniture; wallpaper. In addition, the present invention relates to a device selected from the group consisting of stationary visual display units such as visual display units of computers, televisions, visual display units in printers, kitchen appliances and advertising panels, illuminations, information panels, and mobile visual display units such as visual display units in cellphones, tablet PCs, laptops, digital cameras, MP3 players, vehicles and destination displays on buses and trains; illumination units; keyboards; items of clothing; furniture; wallpaper, comprising at least one inventive organic light-emitting diode or at least one inventive light-emitting layer.

The following examples are included for illustrative purposes only and do not limit the scope of the claims. Unless otherwise stated, all parts and percentages are by weight.

### Examples

### Example 1: Synthesis of compound 3

The synthesis of 1 is done according to the procedure describe in Tetrahedron 69 (2013) 2647-2654. 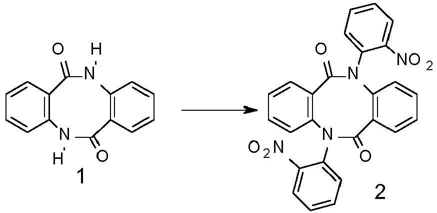
a) 2.00 g of 1 (8.395 mmol), 2.49 g of 2-fluoronitrobenzene (17.63 mmol) and 2.90 g of K₂CO₃ (20.99 mmol) are stirred together in 15 mL of DMSO at 100°C for 4h. The mixture is then poured into 100 mL of water and the beige powder is washed with 2x50 mL of water and 2x50 mL of methanol. It is purified by chromatography (silica gel, heptane/dichloromethane) to yield 3.3g of the desired product (yield = 81%).
   ¹H NMR (400 MHz, CDCl₃); δ = 7.22 (dd, *J*₁= 8.1 Hz, *J*₂= 1.5 Hz, 2H); 7.36 (m, 6H); 7.52 (m, 6H); 8.11 (dd, *J*₁= 8.0 Hz, *J*₂= 1.4 Hz, 2H). 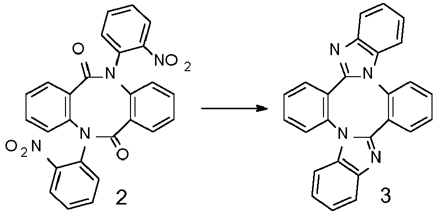
b) 3.24 g of **2** (6.744 mmol), 4.80 g of tin (40.435 mmol) and 13 mL of concentrated HCl (37%) are stirred together in 80 mL of ethanol at reflux for 12h. After removal of the solvent under vacuum, chloroform is added and the organic phase is washed several times with water. The product is then purified by chromatography (silica gel, heptane/chloroform), recrystallization (DMF) and sublimation (Yield = 41%).
   ¹H NMR (400 MHz, CDCl₃); δ = 7.25 (m, 6H); 7.44 (m, 2H); 7.60 (m, 4H); 7.78 (m, 2H); 7.93 (m, 2H). Purity _{(HPLC)} = 99.97%

### Example 2: Synthesis of compound 5

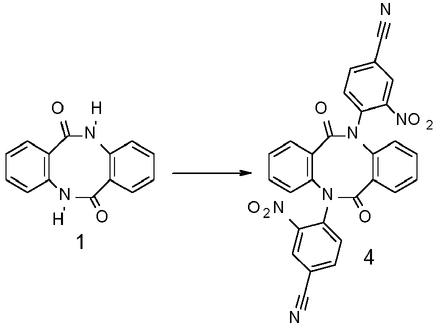

a) It is done according the same procedure described in Example 1 from **1** to **2** with 4-fluoro-3-nitrobenzonitrile instead of 2-fluoronitrobenzene (yield = 64%).
   ¹H NMR (400 MHz, CDCl₃); δ = 7.32 (dd, *J*₁= 8.5 Hz, *J*₂= 0.6 Hz, 2H); 7.44 (m, 2H); 7.56 (d, *J*₁= 8.5 Hz, *J*₂= 1.4 Hz, 2H), 7.72 (m, 4H); 7.45 (m, 2H); 7.97 (m, 2H); 8.14 (dd, *J*₁= 1.4 Hz, *J*₂= 0.7 Hz, 2H). 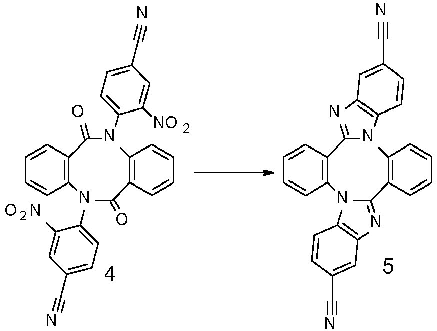
b) It is done according the same procedure described in Example 1 from **2** to **3** (yield = 38%).
   ¹H NMR (400 MHz, CDCl₃); δ = 7.32 (d, *J=* 8.8 Hz, 2H); 7.44 (dd, *J*₁= 7.3 Hz, *J*₂= 1.6 Hz, 2H); 7.55 (dd, *J*₁= 8.6 Hz, *J*₂= 1.4 Hz, 2H); 7.72 (m, 4H); 7.97 (dd, *J*₁= 7.3 Hz, *J*₂= 1.9 Hz , 2H), 8.14 (d, *J=* 1.4 Hz, 2H). Purity _{(HPLC)} = 99.80%

### Example 3: Synthesis of compound 8

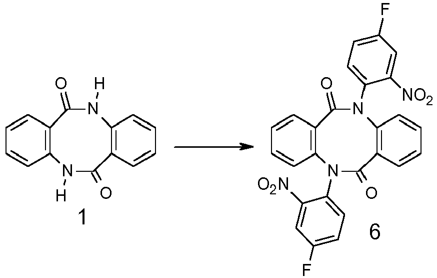

a) It is done according the same procedure described in Example 1 from **1** to **2** with 2,5-difluoronitrobenzene instead of 2-fluoronitrobenzene (yield = 73%).
   ¹H NMR (400 MHz, CDCl₃); δ = 7.17-7.30 (m, 4H); 7.33-7.40 (m, 6H); 7.50 (m, 2H), 7.84 (dd, *J*₁= 7.8 Hz, *J*₂= 2.8 Hz, 2H). 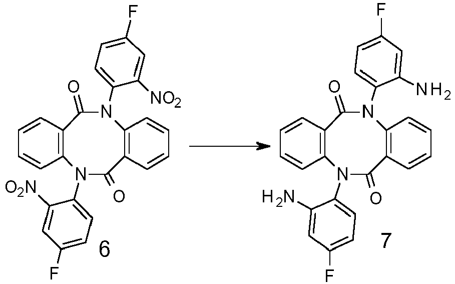
b) 6.31 g of **6** (12.2 mmol) and 0.52 g of Pd/C (10%) in 110 mL of a solution of THF/EtOH are stirred in an autoclave at 50°C under a pressure of 5 bar of hydrogen for 2 hours. The mixture is then filtrated and the solvent evaporated to yield 7 as a white powder that is immediatly used in the next step (yield = quantitative). 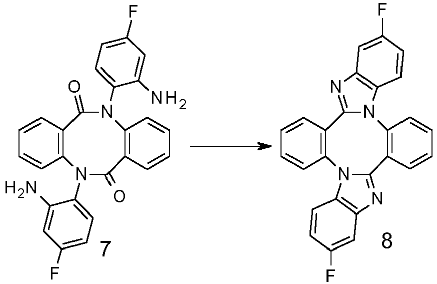
c) 15.9 g of **7** (30.8 mmol), 350 mg of *p*-toluenesulfonic acid monohydrate (1.84 mmol) and 300 mL of o-xylene are mixed together at reflux for 7 hours. The mixture is cooled down, the solid is filtrated and then purified by chromatography (silica gel, toluene/chloroform) (yield = 68%).
   ¹H NMR (400 MHz, CDCl₃); δ = 7.04 (td, *J*₁= 9.1 Hz, *J*₂= 2.4 Hz, 2H); 7.16 (dd, *J*₁= 8.9 Hz, *J*₂= 4.5 Hz, 2H); 7.45 (m, 4H); 7.64 (m, 4H); 7.94 (dd, *J*₁= 7.4 Hz, *J*₂= 1.6 Hz , 2H). Purity _{(HPLC)} = 99.85%

### Example 4: Synthesis of compound 12

The synthesis of **9** is done according to the procedure describe in Tetrahedron 69 (2013) 2647-2654 while using methyl 2-amino-5-fluorobenzoate instead of methyl 2-aminobenzoate. 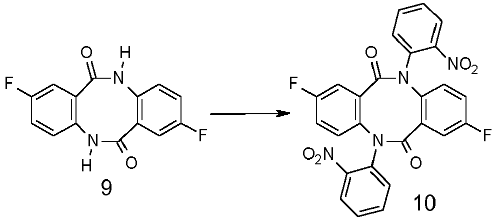
a) It is done according the same procedure described in Example 1 from 1 to **2** with **9** instead of **1** (yield = 73%).
   ¹H NMR (400 MHz, CDCl₃); δ = 7.06 (m, 2H); 7.21 (m, 4H); 7.41 (dd, *J*₁= 8.9 Hz, *J*₂= 4.6 Hz, 2H), 7.49 (td, *J*₁= 7.6 Hz, *J*₂= 1.5 Hz, 2H); 7.56 (td, *J*₁= 7.7 Hz, *J*₂= 1.6 Hz, 2H); 8.11 (dd, *J*₁= 8.1 Hz, *J*₂= 1.6 Hz, 2H). 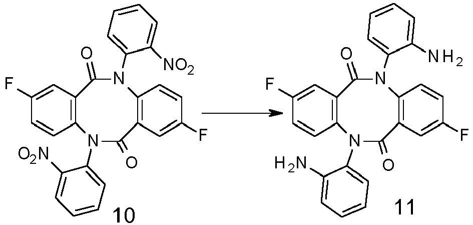
b) It is done according the same procedure described in Example 3 from **6** to **7** (yield = quantitative). 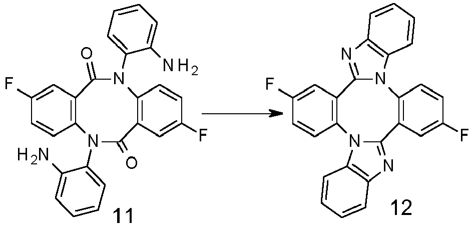
c) It is done according the same procedure described in Example 3 from **7** to **8** (yield = 89%).
   ¹H NMR (400 MHz, DMSO); δ = 7.23-7.34 (m, 6H); 7.60-7.80 (m, 8H). Purity _{(HPLC)} = 99.65%

### Example 5: Synthesis of compound 14

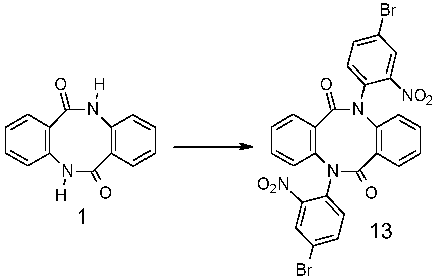

a) It is done according the same procedure described in Example 1 from **1** to **2** with 4-bromo-1-fluoro-2-nitrobenzene instead of 2-fluoronitrobenzene (yield = 78%).
   ¹H NMR (400 MHz, CDCl₃); δ = 7.07 (d, *J=* 8.6 Hz, 2H), 7.30-7.40 (m, 6H); 7.48 (m, 2H); 7.64 (dd, *J*₁= 8.6 Hz, *J*₂= 2.2 Hz, 2H); 8.23 (d, *J=* 2.2 Hz, 2H). 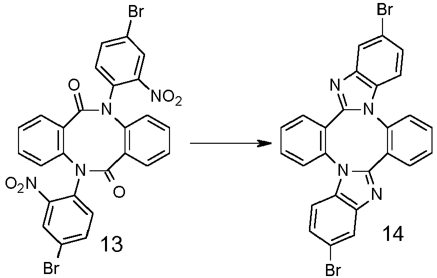
b) It is done according the same procedure described in Example 1 from **2** to **3** (yield = 27%).
   ¹H NMR (400 MHz, CDCl₃); δ = 7.32 (d, *J=* 8.8 Hz, 2H); 7.44 (dd, *J*₁= 7.3 Hz, *J*₂= 1.6 Hz, 2H); 7.55 (dd, *J*₁= 8.6 Hz, *J*₂= 1.4 Hz, 2H); 7.72 (m, 4H); 7.97 (dd, *J*₁= 7.3 Hz, *J*₂= 1.9 Hz , 2H), 8.14 (d, *J=* 1.4 Hz, 2H). Purity _{(HPLC)} = 99.80%

### Example 6: Synthesis of compound 15

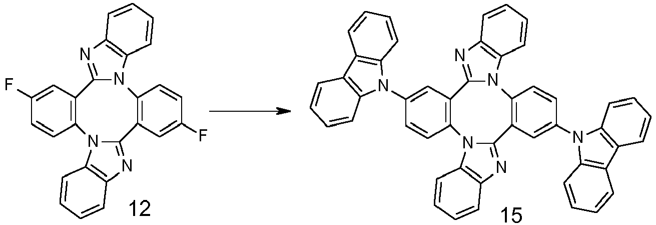

2.29 g of **12** (5.00 mmol), 1.84 g of carbazol (11.00 mmol) and 3.18 g of K₃PO₄ (15.00 mmol) are stirred together in 30 mL of NMP at 190°C for 10h. The mixture is then poured into 200 mL of water and the beige powder is washed with 3x10 mL of water and 130 mL of ethanol. It is purified by chromatography (silica gel, toluol/ethyl acetate), recrystallization (DMF) to yield 2.9g of the desired product **15** that is sublimed (yield = 81%).
¹H NMR (400 MHz, CDCl₃); δ = 7.33-7.46 (m, 10H); 7.50 (m, 4H); 7.68 (d, *J=* 8.2 Hz, 4H); 7.79 (d, *J=* 8.5 Hz, 2H); 7.86 (m, 2H); 7.99 (dd, *J*₁= 8.5 Hz, *J*₂= 2.4 Hz, 2H); 8.19 (d, *J=* 7.6 Hz, 4H); 8.33 (d, *J=* 2.4 Hz, 2H). Purity _{(HPLC)} = 99.98%

### Example 7: Synthesis of compound 16

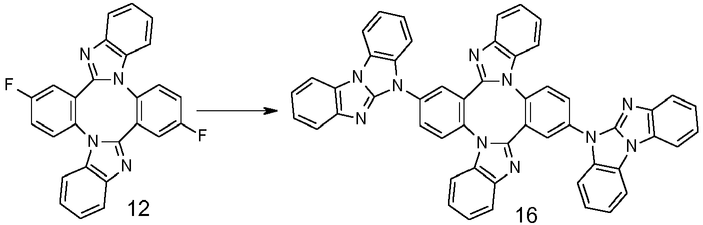

It is done according the same procedure described in Example 7 from **12** to **15** (yield = 27%) MS (MH⁺) = 795.5 g.mol⁻¹. Purity _{(HPLC)} = 98.05%

### Example 8: Synthesis of compound 17

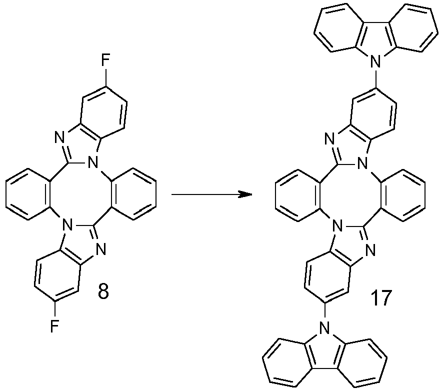

It is done according the same procedure described in Example 7 from **12** to **15** (yield = 21 %) ¹H NMR (400 MHz, CDCl₃); δ = 7.28-7.35 (m, 4H); 7.38-7.46 (m, 8H); 7.49 (m, 4H); 7.68 (dd, *J*₁= 7.9 Hz, *J*₂= 1.4 Hz, 2H); 7.74 (m, 8H); 8.07 (dd, *J*₁= 7.8 Hz, *J*₂= 1.7 Hz, 4H); 8.18 (d, *J*= 7.9 Hz, 4H). Purity _{(HPLC)} = 99.90%

### Device Examples

### Comparative Application Example 1

A glass substrate with 120 nm-thick indium-tin-oxide (ITO) transparent electrode used as an anode is first cleaned with isopropanol in an ultrasonic bath for 10 min. To eliminate any possible organic residues, the substrate is exposed to an ultraviolet light and ozone for further 30 min. This treatment also improves the hole injection properties of the ITO. The cleaned substrate is mounted on a substrate holder and loaded into a vacuum chamber. Thereafter, the organic materials specified below are applied by vapor deposition to the ITO substrate at a rate of approx. 0.2-1 Å/sec at about 10⁻⁶ -10⁻⁸ mbar. As a hole injection layer, compound 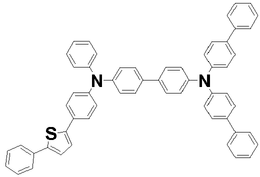 with 30 nm thickness is applied. Then compound 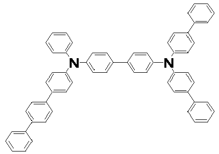 with 60 nm thickness is applied as a hole transporting layer. As an exciton and electron blocker, compound 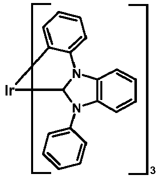 (**HTM-1;** for preparation, see Ir complex (7) in the application WO2005/019373) is then applied with a thickness of 10 nm. Subsequently, a mixture of 20% by weight of emitter compound, 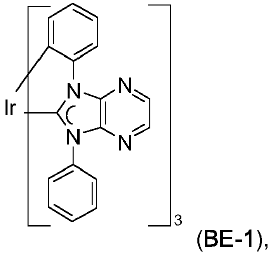 15% by weight of compound (**HTM-1**) and 65% by weight of host 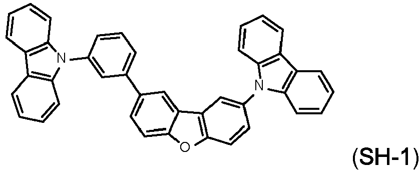 are applied to form a 40 nm-thick emitting layer. On the emitting layer, 5 nm-thick material (**SH-1**) is applied as an exciton blocker. Thereafter, compound 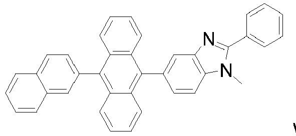 with 20 nm thickness is deposited as an electron transport layer. Finally, 1 nm-thick LiF is deposited as an electron injection layer and 80 nm-thick Al is then deposited as a cathode to complete the device. The device is sealed with a glass lid and a getter in an inert nitrogen atmosphere with less than 1 ppm of water and oxygen.

### OLED characterization

To characterize the OLED, electroluminescence spectra are recorded at various currents and voltages. In addition, the current-voltage characteristic is measured in combination with the luminance to determine luminous efficiency and external quantum efficiency (EQE). Driving voltage U and EQE are given at luminance (L) = 1000 cd/m² and Commission Internationale de l'Éclairage (CIE) coordinate are given at 5mA/cm² except otherwise stated.

### Application Example 1

Comparative Application Example 1 is repeated except that the exciton blocker (SH-1) is replaced by compound 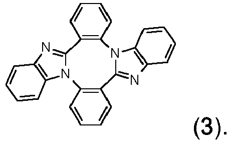 The device results are shown in Table 1.

### Application Example 2

Comparative Application Example 1 is repeated except that the exciton blocker (**SH-1**) is replaced by compound 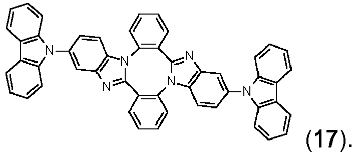 The device results are shown in Table 1.

### Application Example 3

Comparative Application Example 1 is repeated except that the exciton blocker (**SH-1**) is replaced by compound 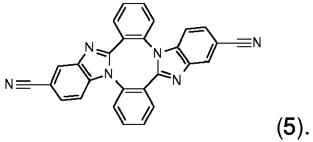 The device results are shown in Table 1.

### Application Example 4

Comparative Application Example 1 is repeated except that the exciton blocker (**SH-1**) is replaced by compound 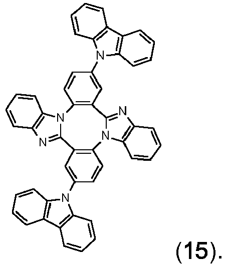 The device results are shown in Table 1.

**Table 1**

| **Appl. Ex.** | **Exciton blocker** | **U [V]** | **EQE [%]** | **CIEx** | **CIEy** |
|---|---|---|---|---|---|
| **Comp. Appl. Ex. 1** | **(SH-1)** | **5.42** | **17.1** | **0.149** | **0.275** |
| **Appl. Ex. 1** | **(3)** | **4.69** | **17.8** | **0.151** | **0.282** |
| **Appl. Ex. 2** | **(17)** | **4.50** | **17.8** | **0.151** | **0.285** |
| **Appl. Ex. 3** | **(5)** | **4.88** | **16.3** | **0.149** | **0.281** |
| **Appl. Ex. 4** | **(15)** | **4.38** | **18.0** | **0.151** | **0.285** |

The results shown in Table 1 demonstrate that the driving voltage U are significantly reduced when compounds (**3**), (**17**), (**5**) and (**15**) are used as exciton blocker instead of reference compound (**SH-1**).

### Application Example 5

Comparative Application Example 1 is repeated except that both host (**SH-1**) and exciton blocker (**SH-1**) are replaced by compound 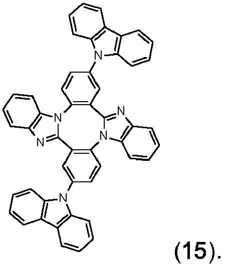 The device results are shown in table 2.

**Table 2**

| **Appl. Ex.** | **Host** | **Exciton blocker** | **U [V]** | **EQE [%]** | **CIEx** | **CIEy** |
|---|---|---|---|---|---|---|
| **Comp. Appl. Ex. 1** | **(SH-1)** | **(SH-1)** | **5.42** | **17.1** | **0.149** | **0.275** |
| **Appl. Ex. 4** | **(SH-1)** | **(15)** | **4.38** | **18.0** | **0.151** | **0.285** |
| **Appl. Ex. 5** | **(15)** | **(15)** | **4.05** | **16.9** | **0.159** | **0.319** |

The results shown in Table 2 demonstrate that the driving voltage U is even more reduced when compound (**15**) is used as both host and exciton blocker at the same time with maintaining the EQE in the same range.

## Claims

1. An organic electronic device comprising a compound of formula (I) 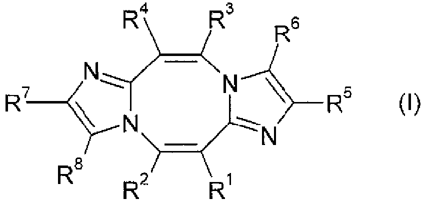 wherein
R¹, R², R³, R⁴, R⁵, R⁶, R⁷ and R⁸
are independently hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
or
R¹ and R² and/or R³ and R⁴ and/or R⁵ and R⁶ and/or R⁷ and R⁸
R¹ and R² and/or R³ and R⁴ and/or R⁵ and R⁶ and/or R⁷ and R⁸
independently of each other form together with the carbon atoms to which they are bonded a 5- or 6-membered (hetero)aromatic ring, which can optionally be part of an annelated ring system, wherein the (hetero)aromatic ring and/or annelated ring system can optionally be substituted by G or by a group of formula -(Ar¹)ₒ-(Ar²)ₚ-(Ar³)_{q}-A; and the hetero atoms of the 5- or 6-membered heteroaromatic ring are selected from N, S and O; preferably,
R¹ and R² and/or R³ and R⁴,
independently of each other form a 5- or 6-membered (hetero)aromatic ring selected from 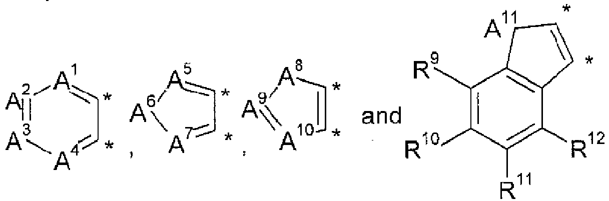 and/or
R⁵ and R⁶ and/or R⁷ and R⁸
independently of each other form a 5- or 6-membered (hetero)aromatic ring selected from 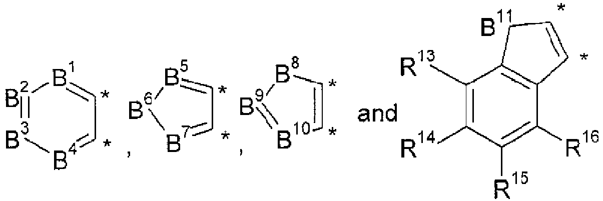 wherein
A¹ and B¹ are independently N, or CR¹⁷,
A² and B² are independently N, or CR¹⁸,
A³ and B³ are independently N, or CR¹⁹,
A⁴ and B⁴ are independently N, or CR²⁰,
A⁵ and B⁵ are independently N, or CR²¹,
A⁶ and B⁶ are independently O, S, NR²⁵ or CR²⁶R²⁷,
A⁷ and B⁷ are independently N, or CR²²,
A⁸ and B⁸ are independently O, S, NR²⁵ or CR²⁶R²⁷,
A⁹ and B⁹ are independently N, or CR²³,
A¹⁰ and B¹⁰ are independently N, or CR²⁴,
A¹¹ and B¹¹ are independently O, S, NR²⁵ or CR²⁶R²⁷,
R⁹, R¹⁰, R¹¹, R¹², R¹³, R¹⁴, R¹⁵, R¹⁶, R¹⁷, R¹⁸, R¹⁹, R²⁰, R²¹, R²², R²³ and R²⁴ are independently hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G, or a group of formula-(Ar¹)ₒ-(Ar²)ₚ-(Ar³)_{q}-A;
R²⁵, R²⁶, R²⁷
are independently hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
* represents the carbon atoms of the basic sceleton;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or-C≡C-,
E is F; Cl; Br; CN; an unsubstituted C₆-C₂₂aryl group, a C₆-C₂₂aryl group, which is substituted by F, C₁-C₁₈alkyl, C₁-C₁₈alkyl which is interrupted by O; an unsubstituted heteroaryl group having 5 to 22 ring atoms, a heteroaryl group having 5 to 22 ring atoms, which is substituted by F, -OR⁶⁹, -SR⁶⁹, -NR⁶⁵R⁶⁶, -COR⁶⁸, -COOR⁶⁷, -CONR⁶⁵R⁶⁶, -CN, or F;
G is E, -OR⁶⁹; -SR⁶⁹; -NR⁶⁵R⁶⁶;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-; R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group: or a C₁-C₁₈alkyl group, which is interrupted by -0-; or
R⁶⁵ and R⁶⁶ together form a five or six membered ring,
R⁶⁷ is a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -0-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -0-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl;
Ar¹, Ar², Ar³ are are independently of each other a C₆-C₂₄arylen group, which can optionally be substituted by G, or a C₂-C₃₀heteroarylen group, which can optionally be substituted by G;
A is hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
o is 0, or 1;
p is 0, or 1; and
q is 0, or 1.

2. The organic electronic device according to claim 1, wherein
R¹ and R² and/or R³ and R⁴
independently of each other form a 5- or 6-membered (hetero)aromatic ring selected from

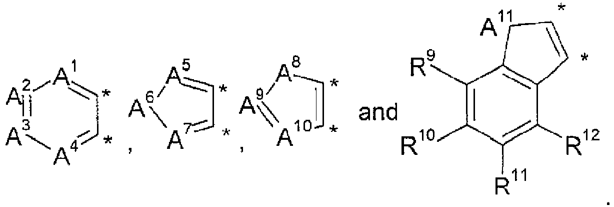

3. The organic electronic device according to claim 1 or 2, wherein
R⁵ and R⁶ and/or R⁷ and R⁸
independently of each other form a 5- or 6-membered (hetero)aromatic ring selected from

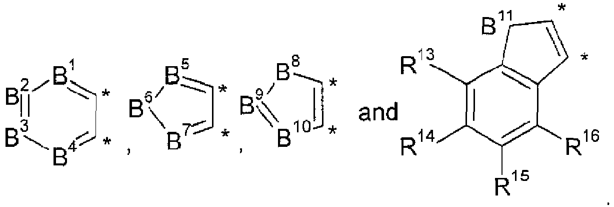

4. The organic electronic device according to any one of claims 1 to 3, wherein 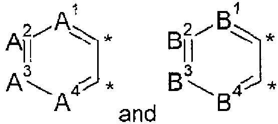 are independently selected from 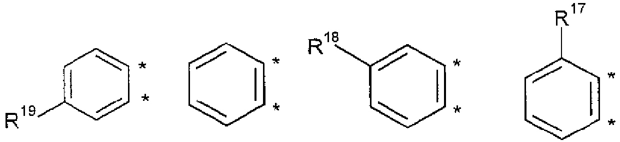 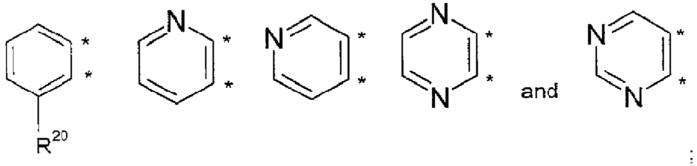 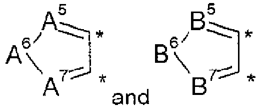 are independently selected from 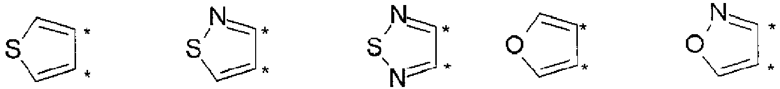 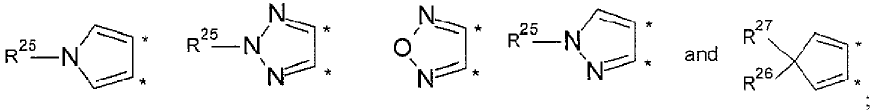 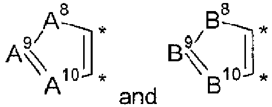 are independently selected from 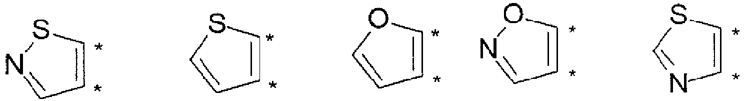 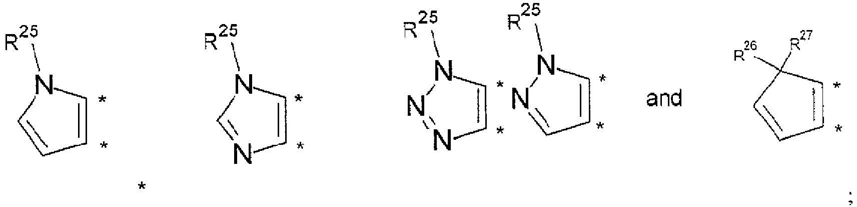 and 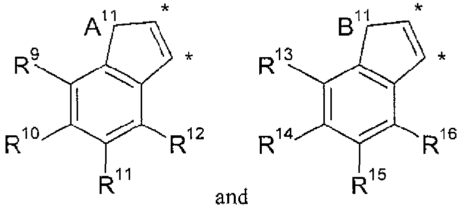 are independently selected from 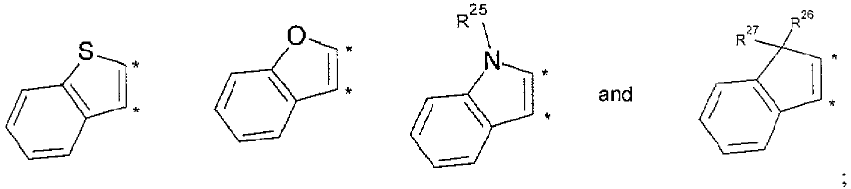 wherein
* represents the carbon atoms of the basic skeleton.

5. The organic electronic device according to any one of claims 1 to 4, wherein the compound of formula (I) is a compound of formula Ia 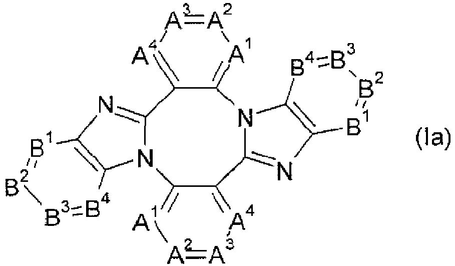 wherein each group A¹, A², A³, A⁴, B¹, B², B³ and B⁴ is mentioned more than one time in formula (Ia) and may be the same or different.

6. The organic electronic device according to claim 5 wherein the compound of formula (Ia) is selected from the following compounds 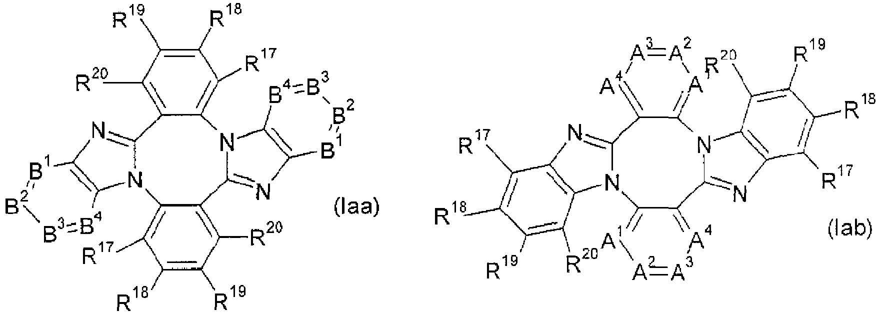 wherein each group A¹, A², A³, A⁴, B¹, B², B³ and B⁴ and each residue R¹⁷, R¹⁸, R¹⁹, R²⁰ is mentioned more than one time in formulae (Iaa) and (lab) and may be the same or different.

7. The organic electronic device according to claim 6, wherein the compound of formula (I) is the following compound 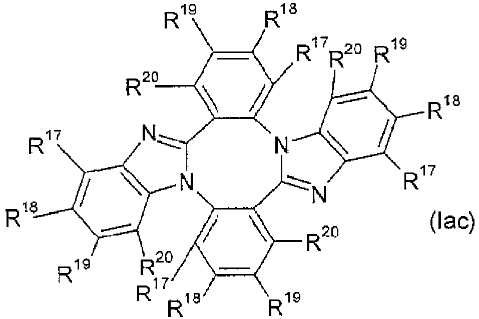 wherein each residue R¹⁷, R¹⁸, R¹⁹, R²⁰ is mentioned more than one time in formula (Iac) and may be the same or different.

8. The organic electronic device according to claim 7, wherein the compound of formula (I) is selected from the following compounds 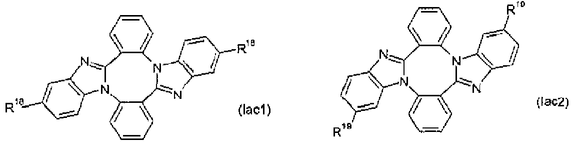 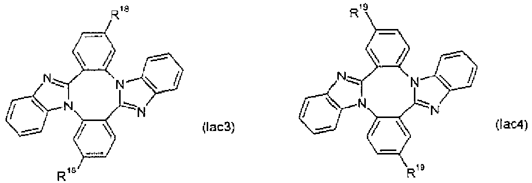 wherein each residue R¹⁸ and R¹⁹ is mentioned more than one time in formulae (Iac1), (Iac2), (Iac3), (Iac4) may be the same or different.

9. The organic electronic device according to any one of claims 1 to 8, wherein
R⁹, R¹⁰, R¹¹, R¹², R¹³, R¹⁴, R¹⁵, R¹⁶, R¹⁷, R¹⁸, R¹⁹, R²⁰, R²¹, R²², R²³ and R²⁴ are independently hydrogen, F, CN, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -0-; or a group of formula -(Ar¹)ₒ-(Ar²)ₚ-(Ar³)_{q}-A, wherein
A is selected from the group consisting of 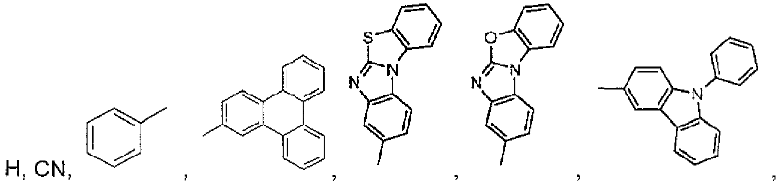 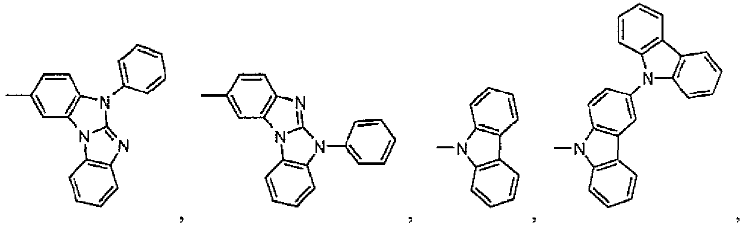 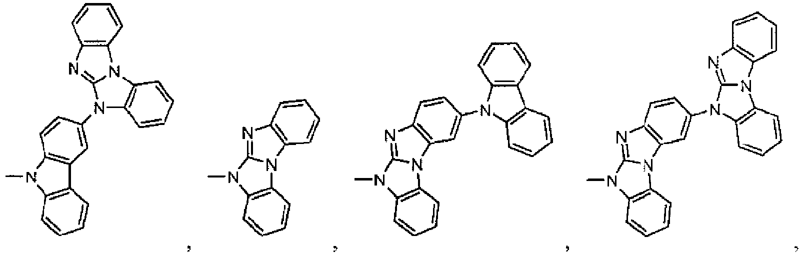 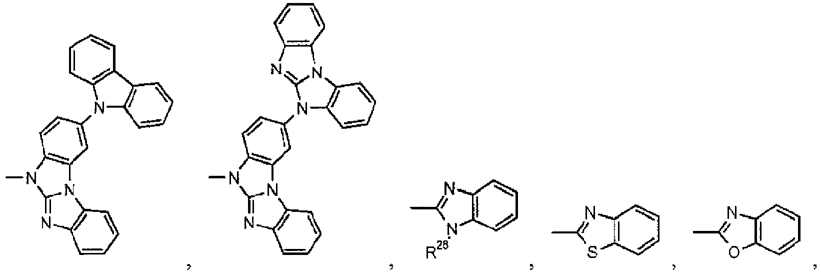 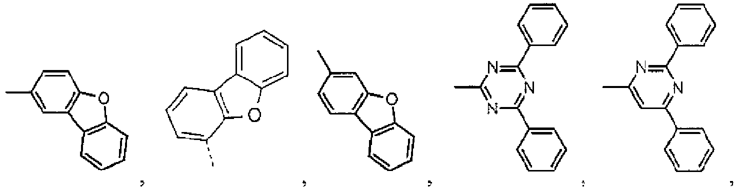 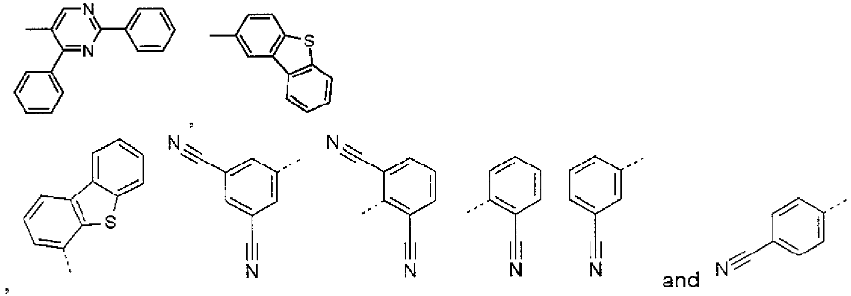 wherein
R²⁸ is hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G; preferably, A is selected from the group consisting of H, CN, 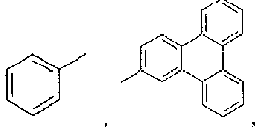 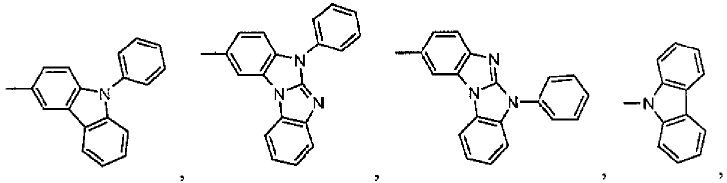 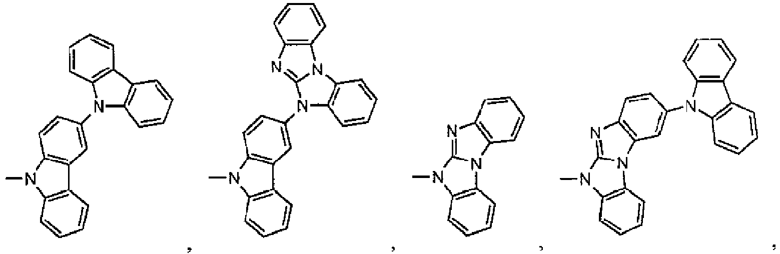 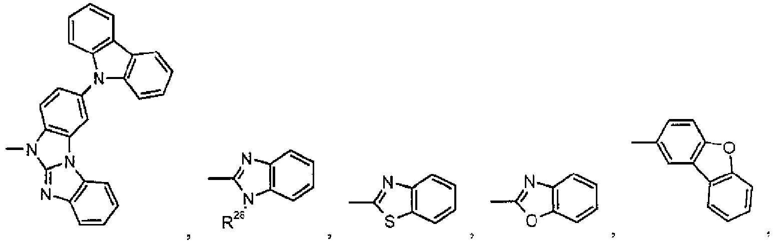 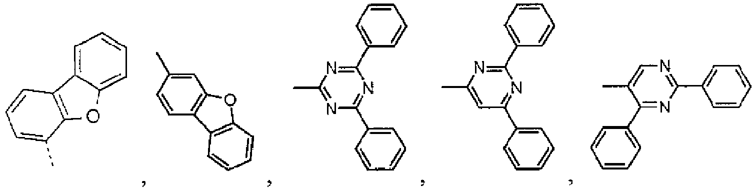 or 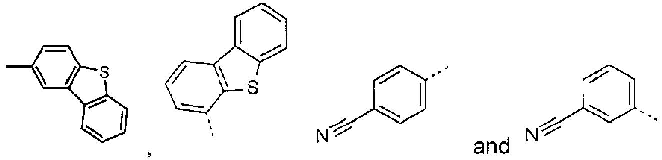 wherein
R²⁸ is hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;Ar¹, Ar² and Ar³ are independently of each other a group of formula 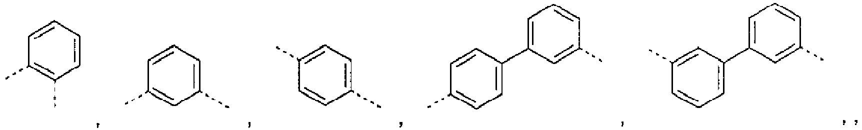 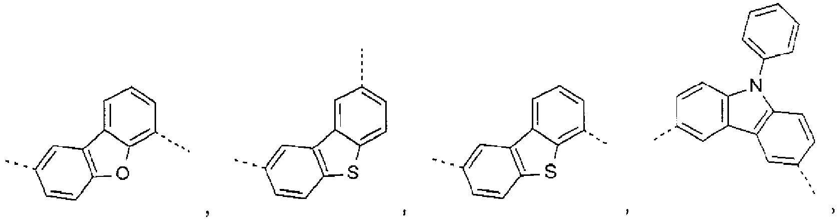 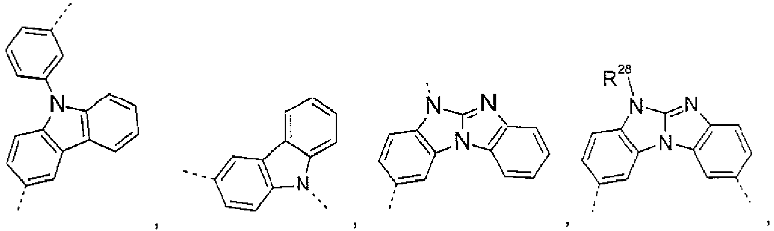 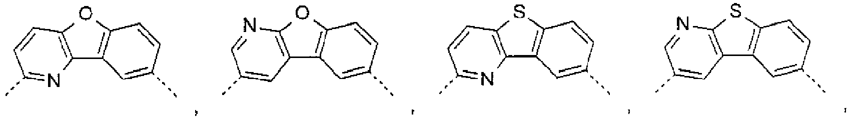 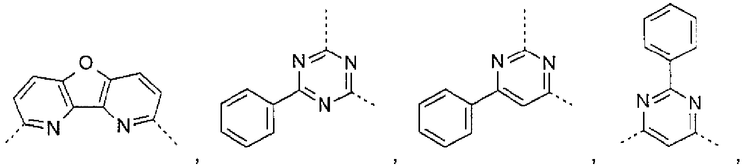 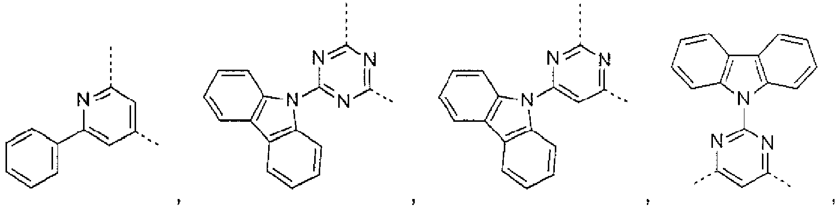 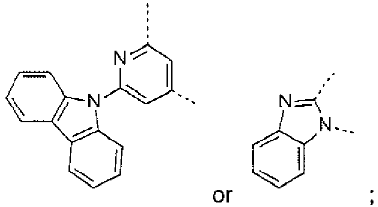 wherein
R²⁸ is hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
preferably, Ar¹, Ar² and Ar³ are independently of each other a group of formula 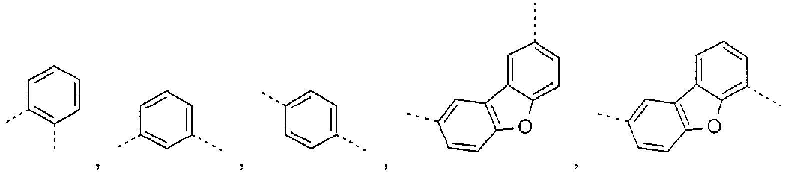 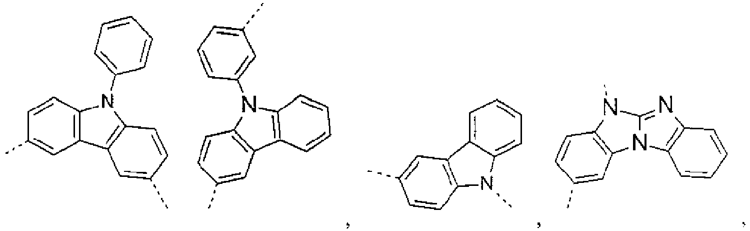 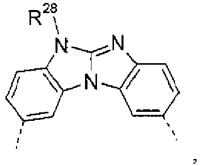 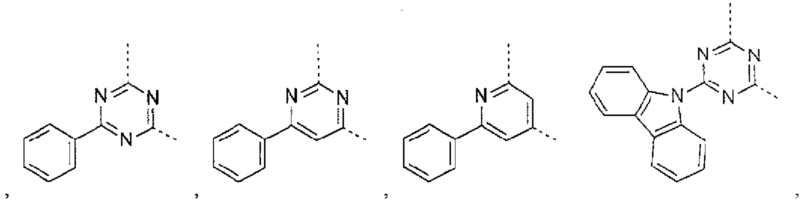 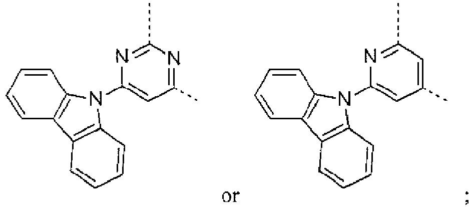 more preferably, Ar¹, Ar² and Ar³ are independently of each other a group of formula 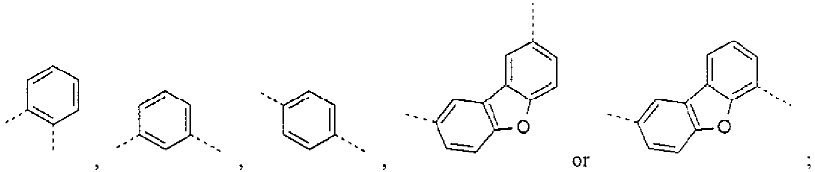 o is 0, or 1;
p is 0, or 1; and
q is 0, or 1.

10. The organic electronic device according to any one of claims 1 to 9, which is an electroluminescent device.

11. A charge transport layer, a charge/exciton blocker layer, or an emitting layer comprising a compound as defined in any one of claims 1 to 9.

12. The emitting layer according to claim 11, comprising a compound as defined in any one of claims 1 to 9 as host material in combination with a phosphorescent emitter.

13. An apparatus selected from the group consisting of stationary visual display units; mobile visual display units; illumination units; keyboards; items of clothing; furniture; wallpaper, comprising the organic electronic device according to any one of claims 1 to 9, or the charge transport layer, the charge/exciton blocker layer, or the emitting layer according to claim 11.

14. Use of the compounds of formula (I) as defined in any one of claims 1 to 9 for electrophotographic photoreceptors, photoelectric converters, organic solar cells, switching elements, organic light emitting field effect transistors, image sensors, dye lasers and electroluminescent devices.

15. A process for the production of a compound of formula (I) 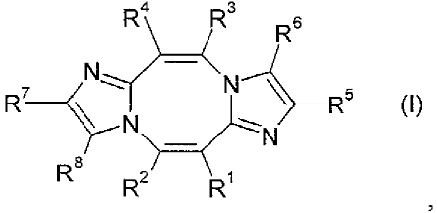 wherein
R¹, R², R³, R⁴, R⁵, R⁶, R⁷ and R⁸ are defined in claim 1, comprising
a) reacting a compound of formula 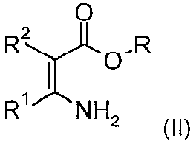 and a compound of formula 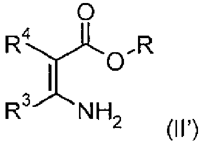 with a base in a solvent to obtain a compound of formula 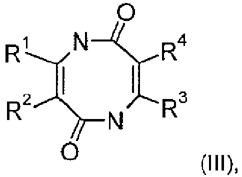 wherein R is C₁-C₁₈alkyl; C₁-C₁₈alkyl which is interrupted by - O-; C₃-C₂₅cycloalkyl; C₆-C₁₈ aryl group; or a heteroaryl group having 5 to 22 ring atoms;
b) reacting the compound of formula (III) with a base and a compound of formula 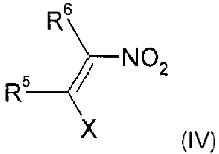 and a compound of formula 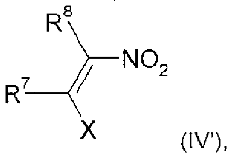 wherein X is F, Cl, Br or I, in a solvent to obtain a compound of formula 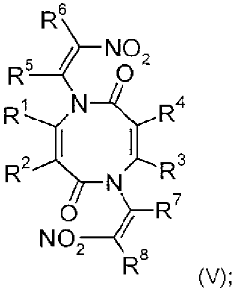
c1) reacting the compound of formula (V) with Sn/HCl in a solvent to obtain a compound of formula (I); or
c2) reducing the compound of formula (V) with H₂ in the presence of Pd/C in a solvent to obtain a compound of formula 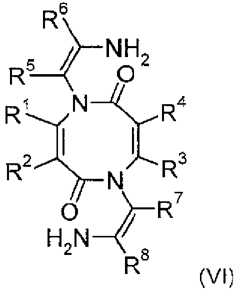 and
d) reacting the compound of formula (VI) with an acid to obtain a compound of formula (I).

16. A compound of formula (I) as defined in any one of claims 1 to 9, with the proviso that the following compounds are excluded:

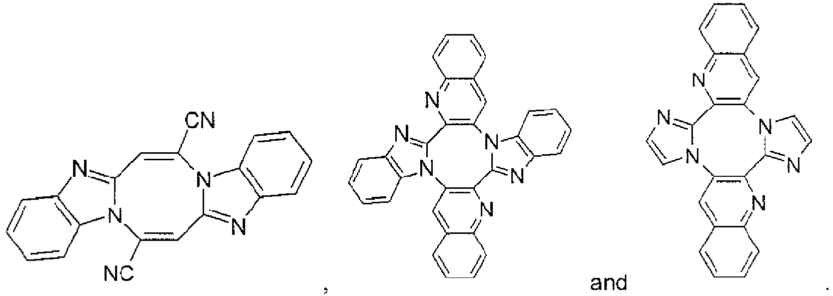

17. The compound as claimed in claim 16, wherein
R¹, R², R³, R⁴, R⁵, R⁶, R⁷ and R⁸
are independently hydrogen, halogen, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
or
R¹ and R² and/or R³ and R⁴ an/or R⁵ and R⁶ and/or R⁷ and R⁸ independently of each other form together with the carbon atoms to which they are bonded a 5- or 6-membered (hetero)aromatic ring, which can optionally be part of an annelated ring system, wherein the (hetero)aromatic ring and/or annelated ring system can optionally be substituted by G, or by a group of formula -(Ar¹)ₒ-(Ar²)ₚ-(Ar³)_{q}-A; and the hetero atoms of the 5- or 6-membered heteroaromatic ring are selected from N, S and O;
preferably,
R¹ and R² and/or R³ and R⁴,
independently of each other form a 5- or 6-membered (hetero)aromatic ring selected from 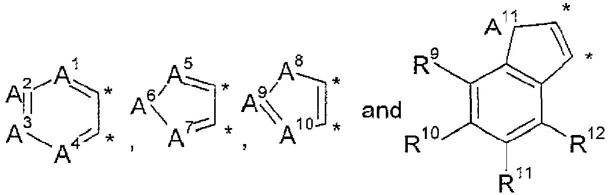 and/or
R⁵ and R⁶ and/or R⁷ and R⁸
independently of each other form a 5- or 6-membered (hetero)aromatic ring selected from 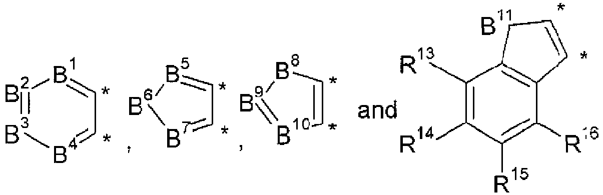 wherein
A¹ and B¹ are independently N, or CR¹⁷,
A² and B² are independently N, or CR¹⁸,
A³ and B³ are independently N, or CR¹⁹,
A⁴ and B⁴ are independently N, or CR²⁰,
A⁵ and B⁵ are independently N, or CR²¹,
A⁶ and B⁶ are independently O, S, NR²⁵ or CR²⁶R²⁷,
A⁷ and B⁷ are independently N, or CR²²,
A⁸ and B⁸ are independently O, S, NR²⁵ or CR²⁶R²⁷,
A⁹ and B⁹ are independently N, or CR²³,
A¹⁰ and B¹⁰ are independently N, or CR²⁴,
A¹¹ and B¹¹ are independently O, S, NR²⁵ or CR²⁶R²⁷,
R⁹, R¹⁰, R¹¹, R¹², R¹³, R¹⁴, R¹⁵, R¹⁶, R¹⁷, R¹⁸, R¹⁹, R²⁰, R²¹, R²², R²³ and R²⁴ are independently hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G, or a group of formula-(Ar¹)ₒ-(Ar²)ₚ-(Ar³)_{q}-A;
R²⁵, R²⁶, R²⁷
are independently hydrogen, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
* represents the carbon atoms of the basic sceleton;
D is -CO-, -COO-, -S-, -SO-, -SO₂-, -O-, -NR⁶⁵-, -SiR⁷⁰R⁷¹-, -POR⁷²-, -CR⁶³=CR⁶⁴-, or - C≡C-,
E is F; Cl; Br; CN; an unsubstituted C₆-C₂₂aryl group, a C₆-C₂₂aryl group, which is substituted by F, C₁-C₁₈alkyl, C₁-C₁₈alkyl which is interrupted by O; an unsubstituted heteroaryl group having 5 to 22 ring atoms, a heteroaryl group having 5 to 22 ring atoms, which is substituted by F, -OR⁶⁹, -SR⁶⁹, -NR⁶⁵R⁶⁶, -COR⁶⁸, -COOR⁶⁷, -CONR⁶⁵R⁶⁶, -CN, or F;
G is E, -OR⁶⁹; -SR⁶⁹; -NR⁶⁵R⁶⁶;
R⁶³ and R⁶⁴ are independently of each other H, C₆-C₁₈aryl; C₆-C₁₈aryl which is substituted by C₁-C₁₈alkyl, or C₁-C₁₈alkoxy; C₁-C₁₈alkyl; or C₁-C₁₈alkyl which is interrupted by -O-; R⁶⁵ and R⁶⁶ are independently of each other a C₆-C₁₈aryl group; a C₆-C₁₈aryl which is substituted by C₁-C₁₈alkyl, or C₁-C₁₈alkoxy; a C₁-C₁₈alkyl group; or a C₁-C₁₈alkyl group, which is interrupted by -O-; or
R⁶⁵ and R⁶⁶ together form a five or six membered ring,
R⁶⁷ is C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁸ is H; a C₆-C₁₈aryl group; a C₆-C₁₈aryl group, which is substituted by C₁-C₁₈alkyl, or C₁-C₁₈alkoxy; a C₁-C₁₈alkyl group; or a C₁-C₁₈alkyl group, which is interrupted by -O-,
R⁶⁹ is a C₆-C₁₈aryl; a C₆-C₁₈aryl, which is substituted by C₁-C₁₈alkyl, or C₁-C₁₈alkoxy; a C₁-C₁₈alkyl group; or a C₁-C₁₈alkyl group, which is interrupted by -O-,
R⁷⁰ and R⁷¹ are independently of each other a C₁-C₁₈alkyl group, a C₆-C₁₈aryl group, or a C₆-C₁₈aryl group, which is substituted by C₁-C₁₈alkyl, and
R⁷² is a C₁-C₁₈alkyl group, a C₆-C₁₈aryl group, or a C₆-C₁₈aryl group, which is substituted by C₁-C₁₈alkyl;
Ar¹, Ar², Ar³ are are independently of each other a C₆-C₂₄arylen group, which can optionally be substituted by G, or a C₂-C₃₀heteroarylen group, which can optionally be substituted by G;
A is hydrogen, halogen, CN, NR²⁶R²⁷, C₁-C₂₅alkyl, which can optionally substituted by E and/or interrupted by D, C₃-C₂₅cycloalkyl, which can optionally substituted by G and/or interrupted by D, C₆-C₂₂aryl, which can optionally substituted by G, heteroaryl having 5 to 22 ring atoms, which can optionally substituted by G;
o is 0, or 1;
p is 0, or 1; and
q is 0, or 1,

## Patentansprüche

1. Organische elektronische Vorrichtung, umfassend eine Verbindung der Formel (I) 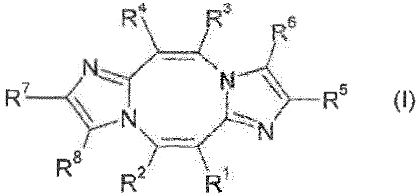 worin
R¹, R², R³, R⁴, R⁵, R⁶, R⁷ und R⁸
unabhängig voneinander Wasserstoff, Halogen, CN, NR²⁶R²⁷, C₁-C₂₅-Alkyl, das wahlweise durch E substituiert sein kann und/oder durch D unterbrochen ist, C₃-C₂₅-Cycloalkyl, das wahlweise durch G substituiert sein kann und/oder durch D unterbrochen ist, C₆-C₂₂-Aryl, das wahlweise durch G substituiert sein kann, Heteroaryl mit 5 bis 22 Ringatomen, das wahlweise durch G substituiert sein kann, sind;
R¹ und R² und/oder R³ und R⁴ und/oder R⁵ und R⁶ und/oder R⁷ und R⁸
R¹ und R² und/oder R³ und R⁴ und/oder R⁵ und R⁶ und/oder R⁷ und R⁸
unabhängig voneinander zusammen mit den Kohlenstoffatomen, an die sie gebunden sind, einen 5- oder 6-gliedrigen (hetero)aromatischen Ring bilden, der wahlweise Teil eines annelierten Ringsystems sein kann,
wobei der (hetero)aromatische Ring und/oder das annelierte Ringsystem wahlweise durch G oder durch eine Gruppe der Formel -(Ar¹)ₒ- (Ar²)ₚ-(Ar³)_{q}-A substituiert sein können; und die Heteroatome des 5- oder 6-gliedrigen heteroaromatischen Rings ausgewählt sind aus N, S und O; worin vorzugsweise
R¹ und R² und/oder R³ und R⁴
unabhängig voneinander einen 5- oder 6-gliedrigen (hetero)aromatischen Ring bilden, der ausgewählt ist aus 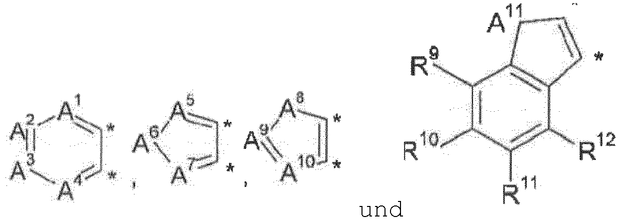 und/oder
R⁵ und R⁶ und/oder R⁷ und R⁸
unabhängig voneinander einen 5- oder 6-gliedrigen (hetero)aromatischen Ring bilden, der ausgewählt ist aus 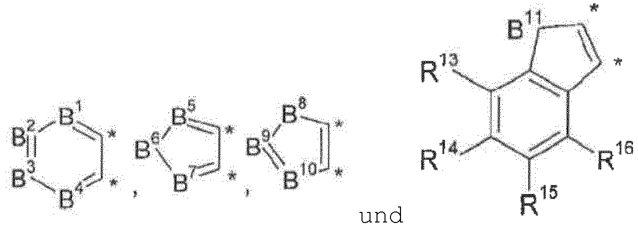 worin
A¹ und B¹ unabhängig voneinander N oder CR¹⁷ sind,
A² und B² unabhängig voneinander N oder CR¹⁸ sind,
A³ und B³ unabhängig voneinander N oder CR¹⁹ sind,
A⁴ und B⁴ unabhängig voneinander N oder CR²⁰ sind,
A⁵ und B⁵ unabhängig voneinander N oder CR²¹ sind,
A⁶ und B⁶ unabhängig voneinander O, S, NR²⁵ oder CR²⁶R²⁷ sind,
A⁷ und B⁷ unabhängig voneinander N oder CR²² sind,
A⁸ und B⁸ unabhängig voneinander O, S, NR²⁵ oder CR²⁶R²⁷ sind,
A⁹ und B⁹ unabhängig voneinander N oder CR²³ sind,
A¹⁰ und B¹⁰ unabhängig voneinander N oder CR²⁴ sind,
A¹¹ und B¹¹ unabhängig voneinander O, S, NR²⁵ oder CR²⁶R²⁷ sind, R⁹, R¹⁰, R¹¹, R¹², R¹³, R¹⁴, R¹⁵, R¹⁶, R¹⁷, R¹⁸, R¹⁹, R²⁰, R²¹, R²², R²³ und R²⁴ unabhängig Wasserstoff, Halogen, CN, NR²⁶R²⁷, C₁-C₂₅-Alkyl, das wahlweise durch E substituiert und/oder durch D unterbrochen sein kann, C₃-C₂₅-Cycloalkyl, das wahlweise durch G substituiert sein kann und/oder durch D unterbrochen ist, C₆-C₂₂-Aryl, das wahlweise durch G substituiert sein kann, Heteroaryl mit 5 bis 22 Ringatomen, das wahlweise durch G oder eine Gruppe der Formel - (Ar¹)ₒ-(Ar²)ₚ-(Ar³)_{q}-A substituiert sein kann, sind;
R²⁵, R²⁶, R²⁷
unabhängig voneinander Wasserstoff, C₁-C₂₅-Alkyl, das wahlweise durch E substituiert sein kann und/oder durch D unterbrochen ist, C₃-C₂₅-Cycloalkyl, das wahlweise durch G substituiert sein kann und/oder durch D unterbrochen ist, C₆-C₂₂-Aryl, das wahlweise durch G substituiert sein kann, Heteroaryl mit 5 bis 22 Ringatomen, das wahlweise durch G substituiert sein kann;
* für die Kohlenstoffatome des Grundgerüsts steht;
D -CO-,-COO-, -S-, -SO-, -SO₂-, -O-, NR⁶⁵, -SiR⁷⁰R⁷¹, -POR⁷²**-,-**CR⁶³=CR⁶⁴ oder -C≡C- ist,
E F; Cl; Br; CN; eine unsubstituierte C₆-C₂₂-Arylgruppe, eine C₆-C₂₂-Arylgruppe, die durch F substituiert ist, C₁-C₁₈-Alkyl, C₁-C₁₈-Alkyl, das durch O unterbrochen ist; eine unsubstituierte Heteroarylgruppe mit 5 bis 22 Ringatomen, eine Heteroarylgruppe mit 5 bis 22 Ringatomen, die durch F substituiert ist, -OR⁶⁹, -SR⁶⁹,-NR⁶⁵R⁶⁶, -COR⁶⁸, -COOR⁶⁷, -CONR⁶⁵R⁶⁶, -CN oder F ist;
G E, -OR⁶⁹ ; -SR⁶⁹ ; -NR⁶⁵R⁶⁶ ist;
R⁶³ und R⁶⁴ unabhängig voneinander H, C₆-C₁₈-Aryl; C₆-C₁₈-Aryl, das durch C₁-C₁₈-Alkyl oder C₁-C₁₈-Alkoxy substituiert ist; C₁-C₁₈-Alkyl; oder C₁-C₁₈-Alkyl, das durch -O- unterbrochen ist, sind;
R⁶⁵ und R⁶⁶ unabhängig voneinander eine C₆-C₁₈-Arylgruppe; ein C₆-C₁₈-Aryl, das durch C₁-C₁₈-Alkyl oder C₁-C₁₈-Alkoxy substituiert ist; eine C₁-C₁₈-Alkylgruppe; oder eine C₁-C₁₈-Alkylgruppe, die durch -O- unterbrochen ist, sind; oder
R⁶⁵ und R⁶⁶ zusammen einen fünf- oder sechsgliedrigen Ring bilden,
R₆₇ eine C₆-C₁₈-Arylgruppe; eine C₆-C₁₈-Arylgruppe, die durch C₁-C₁₈-Alkyl oder C₁-C₁₈-Alkoxy substituiert ist; C₁-C₁₈-Alkylgruppe; oder eine C₁-C₁₈-Alkylgruppe, die durch -O- unterbrochen ist, ist;
R₆₈ H, eine C₆-C₁₈-Arylgruppe; eine C₆-C₁₈-Arylgruppe, die durch C₁-C₁₈-Alkyl oder C₁-C₁₈-Alkoxy substituiert ist; eine C₁-C₁₈-Alkylgruppe; oder eine C₁-C₁₈-Alkylgruppe, die durch -O- unterbrochen ist, ist;
R₆₉ ein C₆-C₁₈-Aryl; ein C₆-C₁₈-Aryl, das durch C₁-C₁₈-Alkyl oder C₁-C₁₈-Alkoxy substituiert ist; eine C₁-C₁₈-Alkylgruppe; oder eine C₁-C₁₈-Alkylgruppe, die durch -O-unterbrochen ist, ist;
R⁷⁰ und R⁷¹ unabhängig voneinander eine C₁-C₁₈-Alkylgruppe, eine C₆-C₁₈-Arylgruppe oder eine eine C₆-C₁₈-Arylgruppe, die durch C₁-C₁₈-Alkyl substituiert ist, sind; und
R⁷² eine C₁-C₁₈-Alkylgruppe, eine C₆-C₁₈-Arylgruppe oder eine C₆-C₁₈-Arylgruppe, die durch C₁-C₁₈-Alkyl substituiert ist, ist;
Ar¹, Ar², Ar³ unabhängig voneinander eine C₆-C₂₄-Arylengruppe, die wahlweise durch G substituiert sein kann, oder eine C₂-C₃₀-Heteroarylengruppe, die wahlweise durch G substituiert sein kann, sind;
A Wasserstoff, Halogen, CN, NR²⁶R²⁷, C₁-C₂₅-Alkyl, das wahlweise durch E substituiert sein kann und/oder durch D unterbrochen ist, C₃-C₂₅-Cycloalkyl, das wahlweise durch G substituiert sein kann und/oder durch D unterbrochen ist, C₆-C₂₂-Aryl, das wahlweise durch G substituiert sein kann, Heteroaryl mit 5 bis 22 Ringatomen, das wahlweise durch G substituiert sein kann, ist;
o 0 oder 1 ist;
p 0 oder 1 ist; und
q 0 oder 1 ist.

2. Organische elektronische Vorrichtung nach Anspruch 1, worin
R¹ und R² und/oder R³ und R⁴
unabhängig voneinander einen 5- oder 6-gliedrigen (hetero)aromatischen Ring bilden, der ausgewählt ist aus

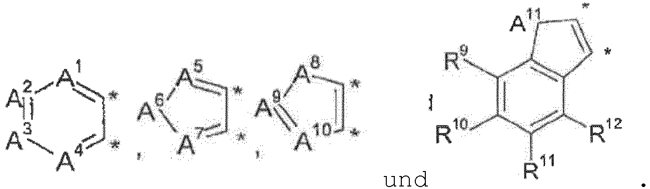

3. Organische elektronische Vorrichtung nach Anspruch 1 oder 2, worin R⁵ und R⁶ und/oder R⁷ und R⁸ unabhängig voneinander einen 5- oder 6-gliedrigen (hetero)aromatischen Ring bilden, der ausgewählt ist aus

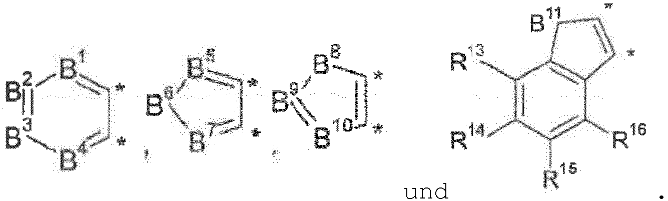

4. Organische elektronische Vorrichtung nach einem der Ansprüche 1 bis 3, worin 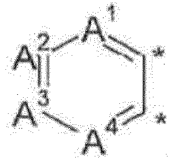 und 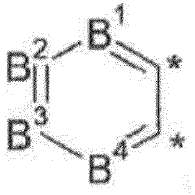 unabhängig ausgewählt sind aus 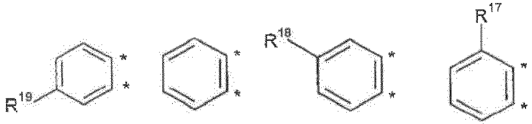 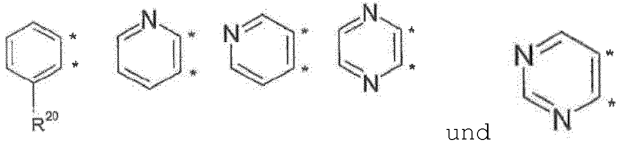 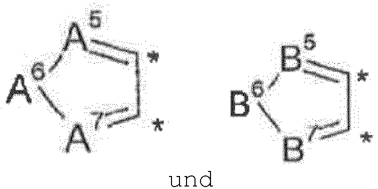 unabhängig ausgewählt sind aus 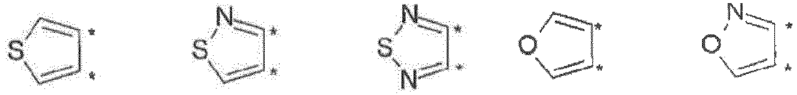 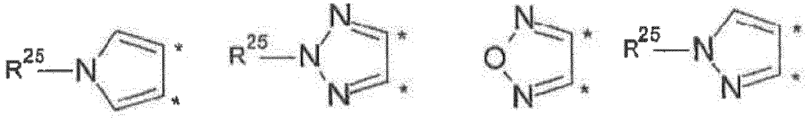 und 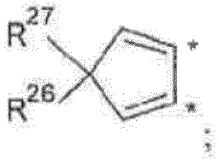 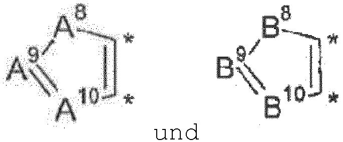 unabhängig ausgewählt sind aus 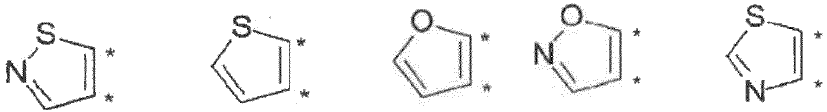 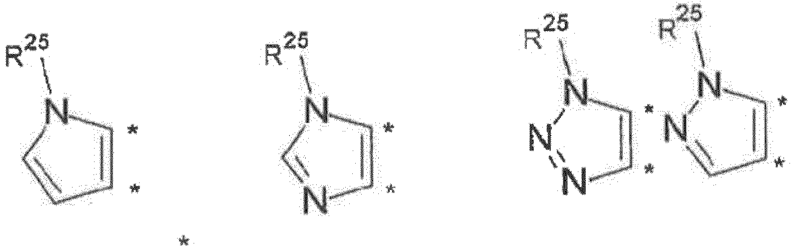 und 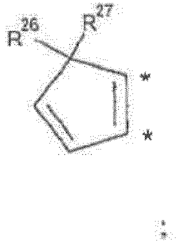 und 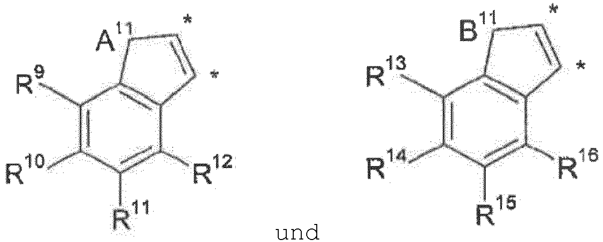 unabhängig ausgewählt sind aus 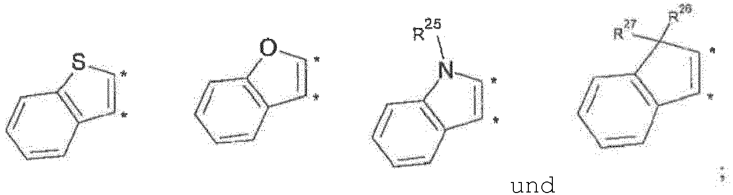 worin
* für die Kohlenstoffatome des Grundgerüsts steht.

5. Organische elektronische Vorrichtung nach einem der Ansprüche 1 bis 4, wobei die Verbindung der Formel (I) eine Verbindung der Formel (Ia) ist 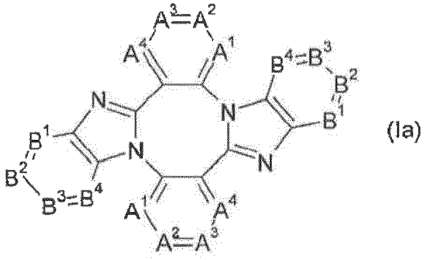 worin jede Gruppe A¹, A², A³, A⁴, B¹, B², B³ und B⁴ mehr als einmal in der Formel (Ia) aufgeführt wird und dieselbe oder eine andere sein kann.

6. Organische elektronische Vorrichtung nach Anspruch 5, wobei die Verbindung der Formel (Ia) aus den folgenden Verbindungen ausgewählt ist 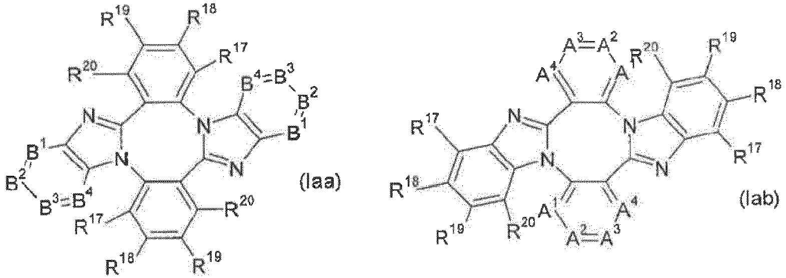 worin jede Gruppe A¹, A², A³, A⁴, B¹, B², B³ und B⁴ und jeder Rest R¹⁷, R¹⁸, R¹⁹, R²⁰ mehr als einmal in der Formel (Iaa) und (Iab) aufgeführt ist und die-/derselbe oder ein/e andere/r sein kann.

7. Organische elektronische Vorrichtung nach Anspruch 6, wobei die Verbindung der Formel (I) die folgende Verbindung ist 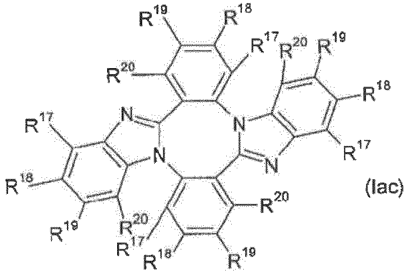 worin jeder Rest R¹⁷, R¹⁸, R¹⁹, R²⁰ mehr als einmal in der Formel (Iac) aufgeführt ist und derselbe oder ein anderer sein kann.

8. Organische elektronische Vorrichtung nach Anspruch 7, wobei die Verbindung der Formel (I) aus den folgenden Verbindungen ausgewählt ist 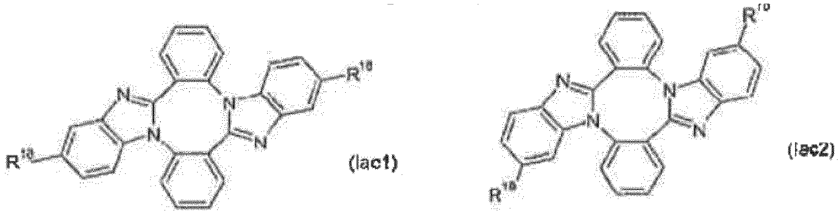 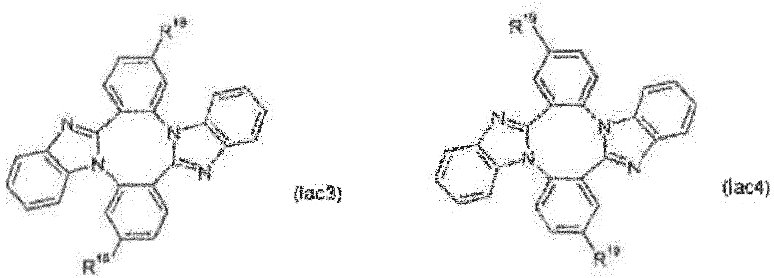 worin jeder Rest R¹⁸ und R¹⁹ mehr als einmal in den Formeln (Iac1), (Iac2), (Iac3), (Iac4) aufgeführt ist und derselbe oder ein anderer sein kann.

9. Organische elektronische Vorrichtung nach einem der Ansprüche 1 bis 8, worin R⁹, R¹⁰, R¹¹, R¹², R¹³, R¹⁴, R¹⁵, R¹⁶, R¹⁷, R¹⁸, R¹⁹, R²⁰, R²¹, R²², R²³ und R²⁴ unabhängig Wasserstoff, F, CN, C₆-C₁₈-Aryl; C₆-C₁₈-Aryl, das durch C₆-C₁₈-Alkyl oder C₁-C₁₈-Alkoxy substituiert ist; C₁-C₁₈-Alkyl; oder C₁-C₁₈-Alkyl, das durch -O- unterbrochen ist, oder eine Gruppe der Formel -(Ar¹)ₒ-(Ar²)ₚ-(Ar³)_{q}-A sind; worin A ausgewählt ist aus der Gruppe, bestehend aus
H, CN, 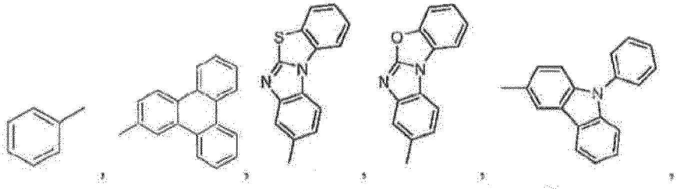 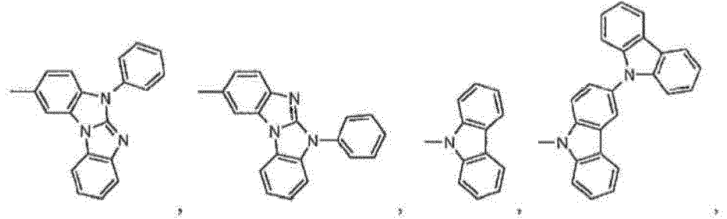 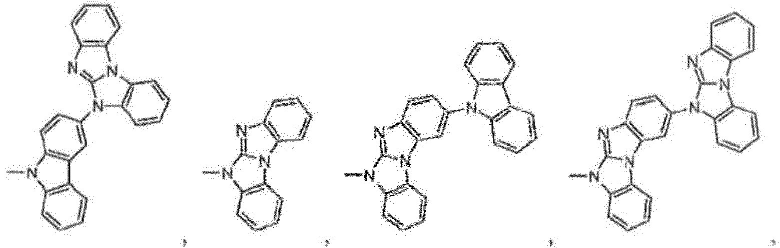 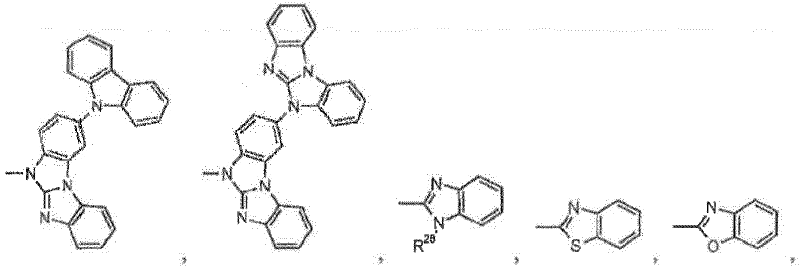 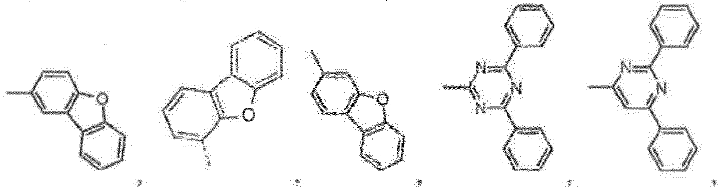 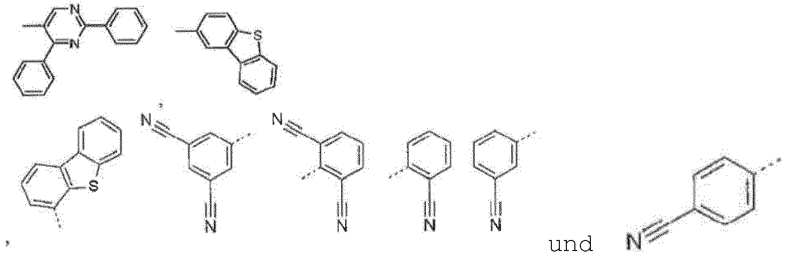 worin
R²⁸ Wasserstoff, C₁-C₂₅-Alkyl, das wahlweise durch E substituiert sein kann und/oder durch D unterbrochen ist, C₃-C₂₅-Cycloalkyl, das wahlweise durch G substituiert sein kann und/oder durch D unterbrochen ist, C₆-C₂₂-Aryl, das wahlweise durch G substituiert sein kann, Heteroaryl mit 5 bis 22 Ringatomen, das wahlweise durch G substituiert sein kann, ist;
worin A vorzugsweise ausgewählt ist aus der Gruppe, bestehend aus H, CN, 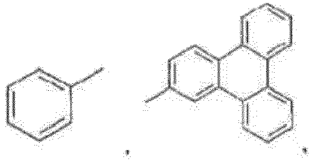 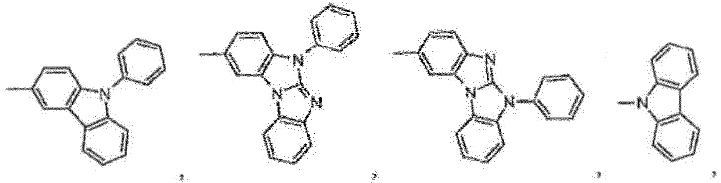 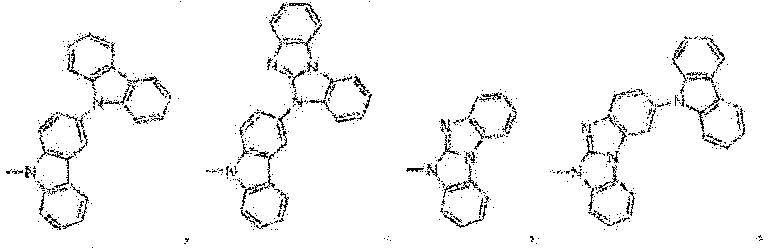 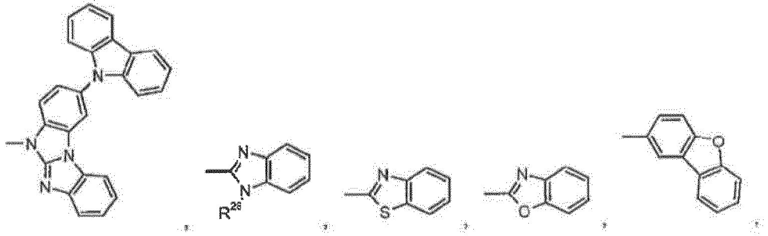 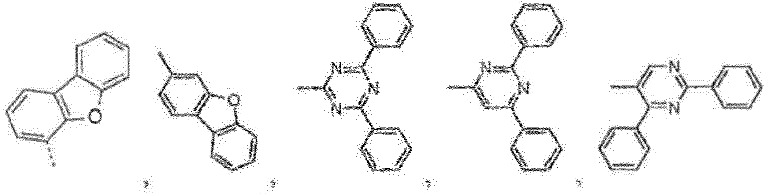 oder 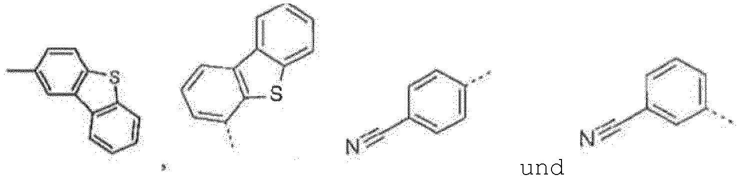 worin R²⁸ Wasserstoff, C₁-C₂₅-Alkyl, das wahlweise durch E substituiert sein kann und/oder durch D unterbrochen ist, C₃-C₂₅-Cycloalkyl, das wahlweise durch G substituiert sein kann und/oder durch D unterbrochen ist, C₆-C₂₂-Aryl, das wahlweise durch G substituiert sein kann, Heteroaryl mit 5 bis 22 Ringatomen, das wahlweise durch G substituiert sein kann, ist; Ar¹, Ar² und Ar³ unabhängig voneinander eine Gruppe der Formel 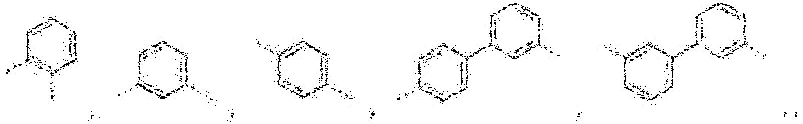 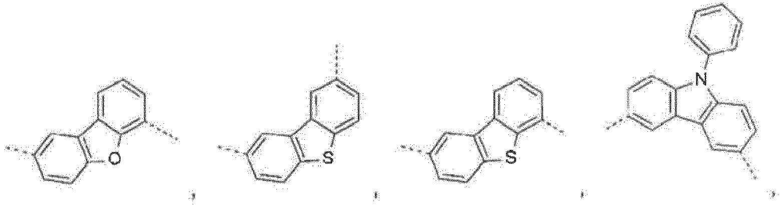 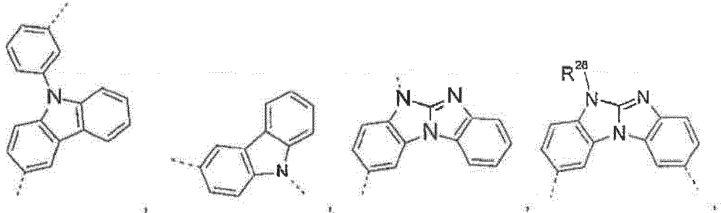 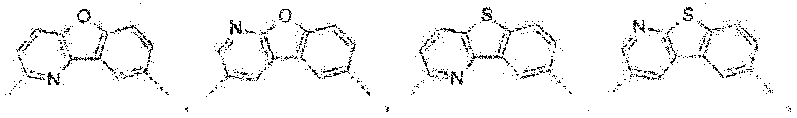 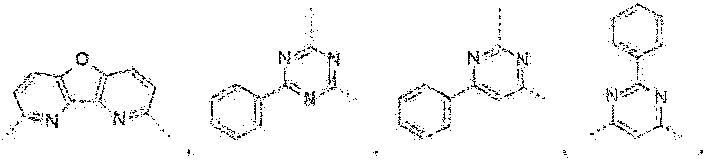 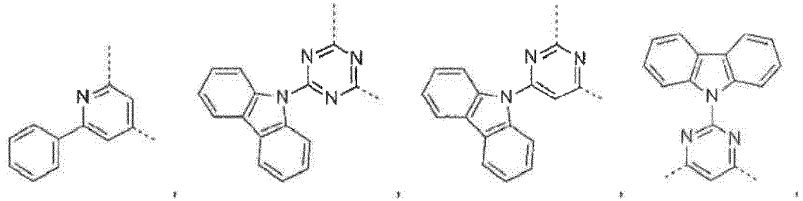 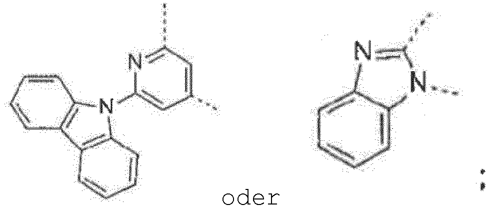 sind, worin
R²⁸ Wasserstoff, C₁-C₂₅-Alkyl, das wahlweise durch E substituiert sein kann und/oder durch D unterbrochen ist, C₃-C₂₅-Cycloalkyl, das wahlweise durch G substituiert sein kann und/oder durch D unterbrochen ist, C₆-C₂₂-Aryl, das wahlweise durch G substituiert sein kann, Heteroaryl mit 5 bis 22 Ringatomen, das wahlweise durch G substituiert sein kann, ist;
Ar¹, Ar² und Ar³ unabhängig voneinander eine Gruppe der Formel 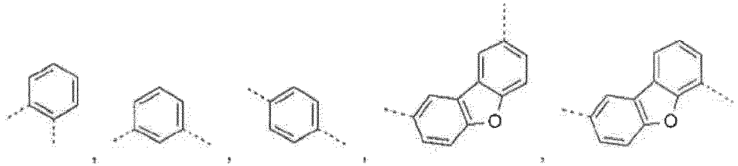 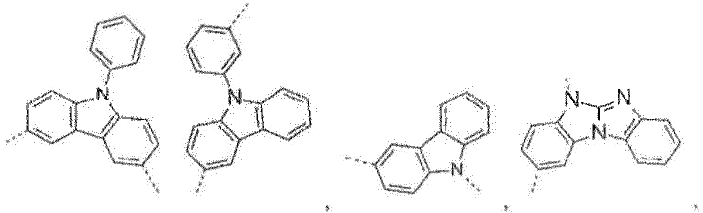 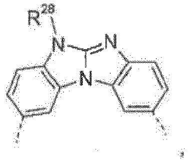 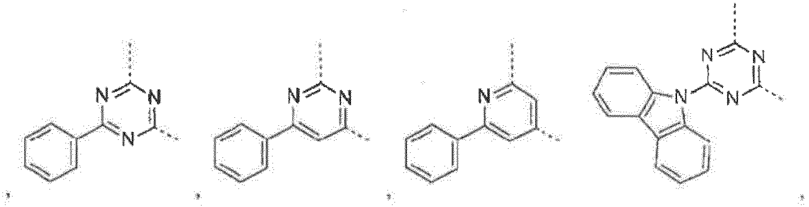 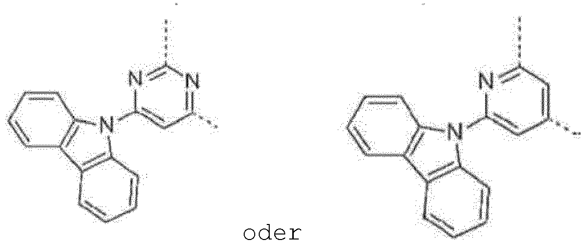 sind,
mehr bevorzugt Ar¹, Ar² und Ar³ unabhängig voneinander eine andere Gruppe der Formel 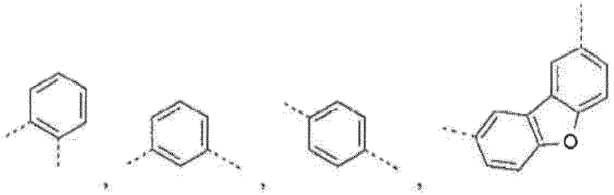 oder 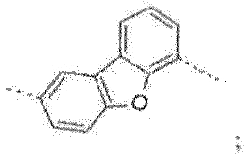 sind,
o 0 oder 1 ist;
p 0 oder 1 ist; und
q 0 oder 1 ist.

10. Organische elektronische Vorrichtung nach einem der Ansprüche 1 bis 9, bei der es sich um eine elektrolumineszierende Vorrichtung handelt.

11. Ladungstransportschicht, Ladungs-/Exziton-Blockerschicht oder emittierende Schicht, umfassend eine Verbindung nach einem der Ansprüche 1 bis 9.

12. Emittierende Schicht nach Anspruch 11, umfassend eine Verbindung nach einem der Ansprüche 1 bis 9 als Wirtsmaterial in Kombination mit einem phosphoreszierenden Emitter.

13. Vorrichtung, ausgewählt aus der Gruppe, bestehend aus stationären visuellen Anzeigeeinheiten; mobilen visuellen Anzeigeeinheiten; Beleuchtungseinheiten; Tastaturen; Kleidungsstücken; Möbeln; Tapete, umfassend die organische elektronische Vorrichtung nach einem der Ansprüche 1 bis 9 oder die Ladungstransportschicht, die Ladungs-/Exziton-Blockerschicht oder die emittierende Schicht nach Anspruch 11.

14. Verwendung der Verbindungen der Formel (I) nach einem der Ansprüche 1 bis 9 für elektrophotographische Photorezeptoren, photoelektrische Wandler, organische Solarzellen, Schaltelemente, organische lichtemittierende Feldeffekttransistoren, Bildsensoren, Farbstofflaser und elektrolumineszierende Vorrichtungen.

15. Verfahren zur Herstellung einer Verbindung der Formel (I) 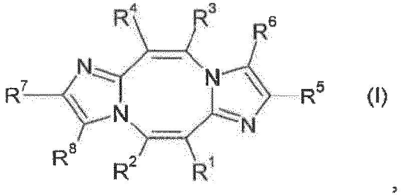 worin
R¹, R², R³, R⁴, R⁵, R⁶, R⁷ und R⁸ in Anspruch 1 definiert sind, umfassend
a) Umsetzen einer Verbindung der Formel 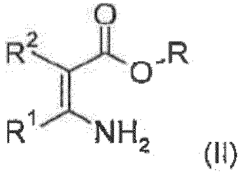 und eine Verbindung der Formel 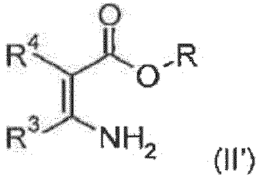 mit einer Base in einem Lösungsmittel, um eine Verbindung der Formel 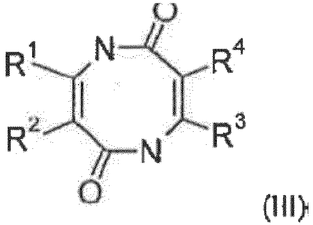 zu erhalten,
worin R C₁-C₁₈-Alkyl; C₁-C₁₈-Alkyl, das durch -O-unterbrochen ist; C₃-C₂₅-Cycloalkyl; C₆-C₁₈-Arylgruppe; oder eine Heteroarylgruppe mit 5 bis 22 Ringatomen ist;
b) Umsetzen der Verbindung der Formel (III) mit einer Base und einer Verbindung der Formel 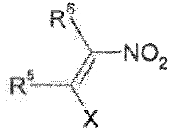 (IV) und eine Verbindung der Formel 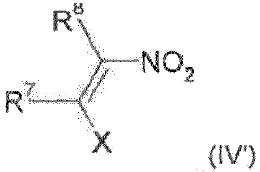 worin X F, Cl, Br oder I in einem Lösungsmittel ist, um eine Verbindung der Formel 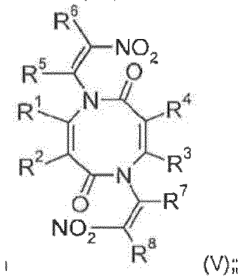 zu erhalten;
c1) Umsetzen der Verbindung der Formel (V) mit Sn/HCl in einem Lösungsmittel zum Erhalten einer Verbindung der Formel (I); oder
c2) Umsetzen der Verbindung der Formel (V) mit H₂ in Gegenwart von Pd/C in einem Lösungsmittel zum Erhalten einer Verbindung der Formel 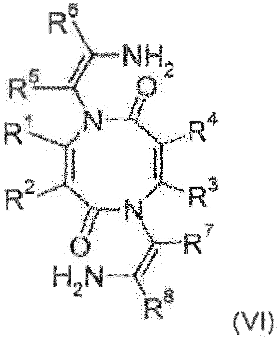 und
d) Umsetzen der der Formel (VI) mit einer Säure zum Erhalten einer Verbindung der Formel (I).

16. Verbindung der Formel (I) nach einem der Ansprüche 1 bis 9, mit der Maßgabe, dass die folgenden Verbindungen ausgeschlossen sind:

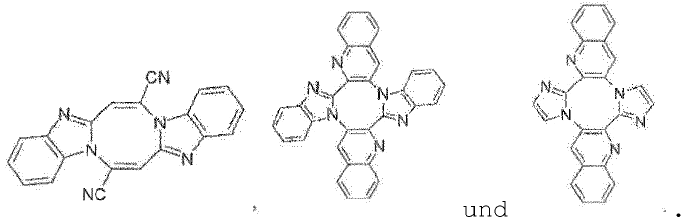

17. Verbindung nach Anspruch 16, wobei R¹, R², R³, R⁴, R⁵, R⁶, R⁷ und R⁸
unabhängig voneinander Wasserstoff, Halogen, NR²⁶R²⁷, C₁-C₂₅-Alkyl, das wahlweise durch E substituiert sein kann und/oder durch D unterbrochen ist, C₃-C₂₅-Cycloalkyl, das wahlweise durch G substituiert sein kann und/oder durch D unterbrochen ist, C₆-C₂₂-Aryl, das wahlweise durch G substituiert sein kann, Heteroaryl mit 5 bis 22 Ringatomen, das wahlweise durch G substituiert sein kann, sind;
R¹ und R² und/oder R³ und R⁴ und/oder R⁵ und R⁶ und/oder R⁷ und R⁸
unabhängig voneinander zusammen mit den Kohlenstoffatomen, an die sie gebunden sind, einen 5-oder 6-gliedrigen (hetero)aromatischen Ring bilden, der wahlweise Teil eines annelierten Ringsystems sein kann, wobei der (hetero)aromatische Ring und/oder das annelierte Ringsystem wahlweise durch G oder durch eine Gruppe der Formel -(Ar¹)ₒ-(Ar²)ₚ-(Ar³)_{q}-A substituiert sein können; und die Heteroatome des 5- oder 6-gliedrigen heteroaromatischen Rings ausgewählt sind aus N, S und 0;
vorzugsweise
R¹ und R² und/oder R³ und R⁴
unabhängig voneinander einen 5- oder 6-gliedrigen (hetero)aromatischen Ring bilden, der ausgewählt ist aus 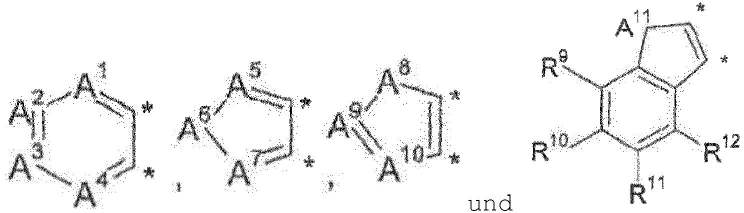 und/oder
R⁵ und R⁶ und/oder R⁷ und R⁸
unabhängig voneinander einen 5- oder 6-gliedrigen (hetero)aromatischen Ring bilden, der ausgewählt ist aus 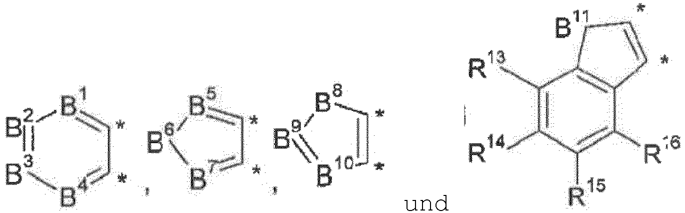 worin
A¹ und B¹ unabhängig voneinander N oder CR¹⁷ sind,
A² und B² unabhängig voneinander N oder CR¹⁸ sind,
A³ und B³ unabhängig voneinander N oder CR¹⁹ sind,
A⁴ und B⁴ unabhängig voneinander N oder CR²⁰ sind,
A⁵ und B⁵ unabhängig voneinander N oder CR²¹ sind,
A⁶ und B⁶ unabhängig voneinander O, S, NR²⁵ oder CR²⁶R²⁷ sind,
A⁷ und B⁷ unabhängig voneinander N oder CR²² sind,
A⁸ und B⁸ unabhängig voneinander O, S, NR²⁵ oder CR²⁶R²⁷ sind,
A⁹ und B⁹ unabhängig voneinander N oder CR²³ sind,
A¹⁰ und B¹⁰ unabhängig voneinander N oder CR²⁴ sind,
A¹¹ und B¹¹ unabhängig voneinander O, S, NR²⁵ oder CR²⁶R²⁷ sind,
R⁹, R¹⁰, R¹¹, R¹², R¹³, R¹⁴, R¹⁵, R¹⁶, R¹⁷, R¹⁸, R¹⁹, R²⁰, R²¹, R²², R²³ und R²⁴ unabhängig Wasserstoff, Halogen, CN, NR²⁶R²⁷, C₁-C₂₅-Alkyl, das wahlweise durch E substituiert und/oder durch D unterbrochen ist, C₃-C₂₅-Cycloalkyl, das wahlweise durch G substituiert sein kann und/oder durch D unterbrochen ist, C₆-C₂₂-Aryl, das wahlweise durch G substituiert sein kann, Heteroaryl mit 5 bis 22 Ringatomen, das wahlweise durch G oder eine Gruppe der Formel -(Ar¹)ₒ-(Ar²)ₚ-(Ar³)_{q}-A substituiert sein kann, sind;
R²⁵, R²⁶, R²⁷
unabhängig voneinander Wasserstoff, C₁-C₂₅-Alkyl, das wahlweise durch E substituiert sein kann und/oder durch D unterbrochen ist, C₃-C₂₅-Cycloalkyl, das wahlweise durch G substituiert sein kann und/oder durch D unterbrochen ist, C₆-C₂₂-Aryl, das wahlweise durch G substituiert sein kann, Heteroaryl mit 5 bis 22 Ringatomen, das wahlweise durch G substituiert sein kann, sind;
* für die Kohlenstoffatome des Grundgerüsts steht;
D -CO-, -COO-, -S-, -SO-, -SO₂-, -O-, NR⁶5, -SiR⁷⁰R⁷¹, -POR⁷²**-,-**CR⁶³=CR⁶⁴ oder -C≡C- ist,
E F; Cl; Br; CN; eine unsubstituierte C₆-C₂₂-Arylgruppe, eine C₆-C₂₂-Arylgruppe, die durch F, C₁-C₁₈-Alkyl substituiert ist, C₁-C₁₈-Alkyl, das durch O unterbrochen ist; eine unsubstituierte Heteroarylgruppe mit 5 bis 22 Ringatomen, eine Heteroarylgruppe mit 5 bis 22 Ringatomen, die durch F, -OR⁶⁹, -SR⁶⁹, -NR⁶⁵R⁶⁶, -COR⁶⁸,-COOR⁶⁷, -CONR⁶⁵R⁶⁶, -CN oder F substituiert ist, ist;
G E, -OR⁶⁹ ; -SR⁶⁹; -NR⁶⁵R⁶⁶ ist;
R⁶³ und R⁶⁴ unabhängig voneinander H, C₆-C₁₈-Aryl; C₆-C₁₈-Aryl, das durch C₁-C₁₈-Alkyl oder C₁-C₁₈-Alkoxy substituiert ist; C₁-C₁₈-Alkyl; oder C₁-C₁₈-Alkyl, das durch -0- unterbrochen ist, sind;
R⁶⁵ und R⁶⁶ unabhängig voneinander eine C₆-C₁₈-Arylgruppe; ein C₆-C₁₈-Aryl, das durch C₁-C₁₈-Alkyl oder C₁-C₁₈-Alkoxy substituiert ist; eine C₁-C₁₈-Alkylgruppe; oder eine C₁-C₁₈-Alkylgruppe, die durch -0- unterbrochen ist, sind; oder
R⁶⁵ und R⁶⁶ zusammen einen fünf- oder sechsgliedrigen Ring bilden,
R₆₇ eine C₆-C₁₈-Arylgruppe; eine C₆-C₁₈-Arylgruppe, die durch C₁-C₁₈-Alkyl oder C₁-C₁₈-Alkoxy substituiert ist; C₁-C₁₈-Alkylgruppe; oder eine C₁-C₁₈-Alkylgruppe, die durch -O- unterbrochen ist, ist;
R₆₈ H; eine C₆-C₁₈-Arylgruppe; eine C₆-C₁₈-Arylgruppe, die durch C₁-C₁₈-Alkyl oder C₁-C₁₈-Alkoxy substituiert ist; eine C₁-C₁₈-Alkylgruppe; oder eine C₁-C₁₈-Alkylgruppe, die durch -O- unterbrochen ist, ist; R₆₉ ein C₆-C₁₈-Aryl; ein C₆-C₁₈-Aryl, das durch C₁-C₁₈-Alkyl oder C₁-C₁₈-Alkoxy substituiert ist; eine C₁-C₁₈-Alkylgruppe; oder eine C₁-C₁₈-Alkylgruppe, die durch -O- unterbrochen ist, ist;
R⁷⁰ und R⁷¹ unabhängig voneinander eine C₁-C₁₈-Alkylgruppe, eine C₆-C₁₈-Arylgruppe oder eine eine C₆-C₁₈-Arylgruppe, die durch C₁-C₁₈-Alkyl substituiert ist, sind; und
R⁷² eine C₁-C₁₈-Alkylgruppe, eine C₆-C₁₈-Arylgruppe oder eine C₆-C₁₈-Arylgruppe, die durch C₁-C₁₈-Alkyl substituiert ist, ist;
Ar¹, Ar², Ar³ unabhängig voneinander eine C₆-C₂₄-Arylengruppe, die wahlweise durch G substituiert sein kann, oder eine C₂-C₃₀-Heteroarylengruppe, die wahlweise durch G substituiert sein kann, sind;
A Wasserstoff, Halogen, CN, NR²⁶R²⁷, C₁-C₂₅-Alkyl, das wahlweise durch E substituiert sein kann und/oder durch D, C₃-C₂₅-Cycloalkyl unterbrochen ist, das wahlweise durch G substituiert sein kann und/oder durch D, C₆-C₂₂-Aryl unterbrochen ist, das wahlweise durch G, Heteroaryl mit 5 bis 22 Ringatomen, substituiert sein kann, das wahlweise durch G substituiert sein kann;
o 0 oder 1 ist;
p 0 oder 1 ist; und
q 0 oder 1 ist.

## Revendications

1. Dispositif électronique organique comprenant un composé de formule (I) 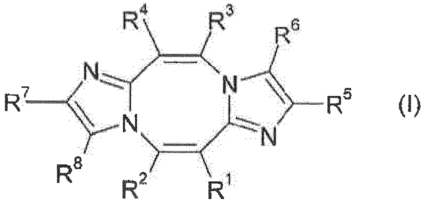 où
chacun des radicaux R¹, R², R³, R⁴, R⁵, R⁶, R⁷ et R⁸ représente indépendamment un atome d'hydrogène ou d'halogène ou un groupement CN, NR²⁶R²⁷, alkyle en C₁-C₂₅, qui est éventuellement substitué par E et/ou interrompu par D ou un groupement cycloalkyle en C₃-C₂₅, qui est éventuellement substitué par G et/ou interrompu par D, aryle en C₆-C₂₂, qui est éventuellement substitué par G, hétéroaryle comportant 5 à 22 chaînons, qui est éventuellement substitué par G ;
ou
R¹ et R² et/ou R³ et R⁴ et/ou R⁵ et R⁶ et/ou R⁷ et R⁸
R¹ et R² et/ou R³ et R⁴ et/ou R⁵ et R⁶ et/ou R⁷ et R⁸ indépendamment les uns des autres, forment ensemble et avec les atomes de carbone auquel ils sont liés un cycle (hétéro) aromatique à 5 ou 6 chaînons, qui fait éventuellement partie d'un système cyclique annelé, où le cycle (hétéro)aromatique et/ou le système cyclique annelé sont éventuellement substitués par G ou par un groupement de formule -(Ar¹)ₒ-(Ar²)ₚ-(Ar³)_{q}-A ; et les hétéroatomes du cycle hétéroaromatique à 5 ou 6 chaînons sont choisis parmi N, S et O ; préférentiellement,
R¹ et R² et/ou R³ et R⁴,
forment indépendamment les uns des autres un cycle (hétéro)aromatique à 5 ou 6 chaînons choisi parmi 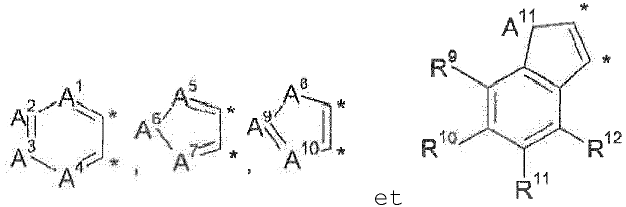 et/ou
R⁵ et R⁶ et/ou R⁷ et R⁸,
forment indépendamment les uns des autres un cycle (hétéro)aromatique à 5 ou 6 chaînons choisi parmi 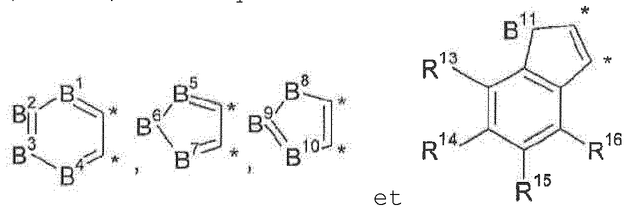 où
A¹ et B¹ représentent indépendamment N ou CR¹⁷,
A² et B² représentent indépendamment N ou CR¹⁸,
A³ et B³ représentent indépendamment N ou CR¹⁹,
A⁴ et B⁴ représentent indépendamment N ou CR²⁰,
A⁵ et B⁵ représentent indépendamment N ou CR²¹,
A⁶ et B⁶ représentent indépendamment O, S, NR²⁵ ou CR²⁶R²⁷,
A⁷ et B⁷ représentent indépendamment N ou CR²²,
A⁸ et B⁸ représentent indépendamment O, S, NR²⁵ ou CR²⁶R²⁷ ;
A⁹ et B⁹ représentent indépendamment N ou CR²³,
A¹⁰ et B¹⁰ représentent indépendamment N ou CR²⁴,
A¹¹ et B¹¹ représentent indépendamment O, S, NR²⁵ ou CR²⁶R²⁷,
chacun des radicaux R⁹, R¹⁰, R¹¹, R¹², R¹³, R¹⁴, R¹⁵, R¹⁶, R¹⁷, R¹⁸, R¹⁹, R²⁰, R²¹, R²², R²³ et R²⁴ représente indépendamment un atome d'hydrogène ou d'halogène ou un groupement CN, NR²⁶R²⁷, alkyle en C₁-C₂₅, qui est éventuellement substitué par E et/ou interrompu par D ou un groupement cycloalkyle en C₃-C₂₅, qui est éventuellement substitué par G et/ou interrompu par D, aryle en C₆-C₂₂, qui est éventuellement substitué par G, hétéroaryle comportant 5 à 22 chaînons, qui est éventuellement substitué par G, ou un groupement de formule -(Ar¹)ₒ-(Ar²)ₚ-(Ar³)_{q}-A ;
chacun des radicaux R²⁵, R²⁶, R²⁷
représente indépendamment un atome d'hydrogène ou un groupement alkyle en C₁-C₂₅, qui est éventuellement substitué par E et/ou interrompu par D ou un groupement cycloalkyle en C₃-C₂₅, qui est éventuellement substitué par G et/ou interrompu par D, aryle en C₆-C₂₂, qui est éventuellement substitué par G, hétéroaryle comportant 5 à 22 chaînons, qui est éventuellement substitué par G ;
* représente les atomes de carbone du squelette de base ;
D représente -CO-, -COO-, -S-, -SO-, -SO₂-, -O-, -NR⁶⁵-, -SiR⁷⁰R⁷¹-, -POR⁷²-, -CR⁶³=CR⁶⁴- ou -C≡C-,
E représente F ; Cl ; Br ; CN ; un groupement aryle en C₆-C₂₂ non substitué, un groupement aryle en C₆-C₂₂, qui est substitué par F, alkyle en C₁-C₁₈, alkyle en C₁-C₁₈ interrompu par O ; un groupement hétéroaryle non substitué comportant 5 à 22 chaînons, un groupement hétéroaryle comportant 5 à 22 chaînons, qui est substitué par F, -OR⁶⁹, -SR⁶⁹, -NR⁶⁵R⁶⁶, -COR⁶⁸, -COOR⁶⁷,-CONR⁶⁵R⁶⁶, -CN ou F ;
G représente E, -OR⁶⁹ ; -SR⁶⁹ ; -NR⁶⁵R⁶⁶ ;
chacun des radicaux R⁶³ et R⁶⁴ représente indépendamment de l'autre H ou un groupement aryle en C₆-C₁₈ ; aryle en C₆-C₁₈ qui est substitué par alkyle en C₁-C₁₈ ou alkoxy en C₁-C₁₈ ; alkyle en C₁-C₁₈ ; ou alkyle en C₁-C₁₈ interrompu par -0- ; chacun des radicaux R⁶⁵ et R⁶⁶ représente indépendamment de l'autre un groupement aryle en C₆-C₁₈ ; un groupement aryle en C₆-C₁₈ qui est substitué par alkyle en C₁-C₁₈ ou alkoxy en C₁-C₁₈ ; un groupement alkyle en C₁-C₁₈ ; ou un groupement alkyle en C₁-C₁₈ interrompu par -O- ; ou
R⁶⁵ et R⁶⁶ forment ensemble un cycle à cinq ou six chaînons,
R⁶⁷ représente un groupement aryle en C₆-C₁₈ ; un groupement aryle en C₆-C₁₈, qui est substitué par alkyle en C₁-C₁₈ ou alkoxy en C₁-C₁₈ ; un groupement alkyle en C₁-C₁₈ ; ou un groupement alkyle en C₁-C₁₈ interrompu par -O-,
R⁶⁸ représente H ; un groupement aryle en C₆-C₁₈ ; un groupement aryle en C₆-C₁₈, qui est substitué par alkyle en C₁-C₁₈ ou alkoxy en C₁-C₁₈ ; un groupement alkyle en C₁-C₁₈ ; ou un groupement alkyle en C₁-C₁₈ interrompu par -O-,
R⁶⁹ représente un groupement aryle en C₆-C₁₈ ; un groupement aryle en C₆-C₁₈, qui est substitué par alkyle en C₁-C₁₈ ou alkoxy en C₁-C₁₈ ; un groupement alkyle en C₁-C₁₈ ; ou un groupement alkyle en C₁-C₁₈ interrompu par -O-,
chacun des radicaux R⁷⁰ et R⁷¹ représente indépendamment de l'autre un groupement alkyle en C₁-C₁₈, un groupement aryle en C₆-C₁₈ ou un groupement aryle en C₆-C₁₈, qui est substitué par alkyle en C₁-C₁₈, et
R⁷² représente un groupement alkyle en C₁-C₁₈, un groupement aryle en C₆-C₁₈ ou un groupement aryle en C₆-C₁₈, qui est substitué par alkyle en C₁-C₁₈ ;
chacun des radicaux Ar¹, Ar², Ar³ représente indépendamment des autres un groupement arylène en C₆-C₂₄, qui est éventuellement substitué par G, ou un groupement hétéroarylène en C₂-C₃₀, qui est éventuellement substitué par G ;
A représente un atome d'hydrogène ou d'halogène ou un groupement CN, NR²⁶R²⁷, alkyle en C₁-C₂₅, qui est éventuellement substitué par E et/ou interrompu par D ou un groupement cycloalkyle en C₃-C₂₅, qui est éventuellement substitué par G et/ou interrompu par D, aryle en C₆-C₂₂, qui est éventuellement substitué par G, hétéroaryle comportant 5 à 22 chaînons, qui est éventuellement substitué par G ;
o est égal à 0 ou à 1 ;
p est égal à 0 ; ou à 1 ; et
q est égal à 0 ou à 1.

2. Dispositif électronique organique selon la revendication 1, où
R¹ et R² et/ou R³ et R⁴,
forment indépendamment les uns des autres un cycle (hétéro)aromatique à 5 ou 6 chaînons choisi parmi

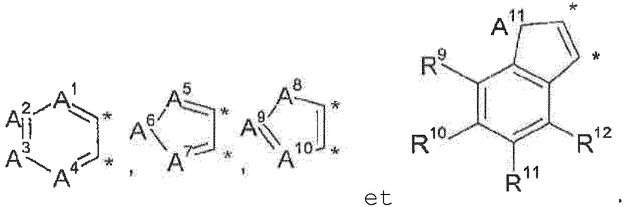

3. Dispositif électronique organique selon la revendication 1 ou 2, où
R⁵ et R⁶ et/ou R⁷ et R⁸,
forment indépendamment les uns des autres un cycle (hétéro)aromatique à 5 ou 6 chaînons choisi parmi

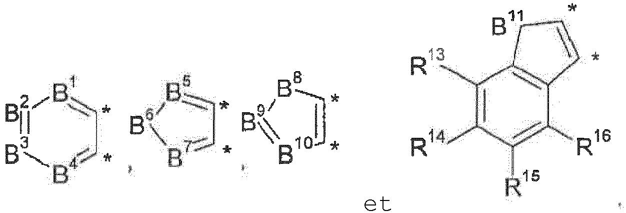

4. Dispositif électronique organique selon l'une quelconque des revendications 1 à 3, où 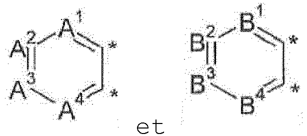 sont indépendamment choisis parmi 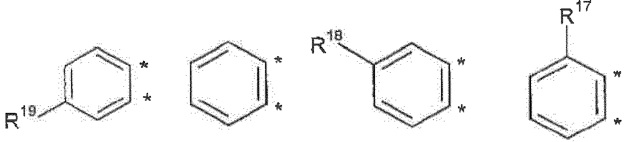 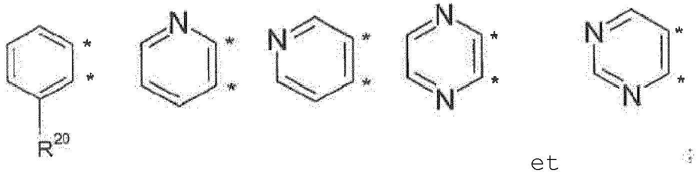 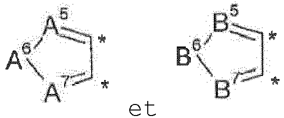 sont indépendamment choisis parmi 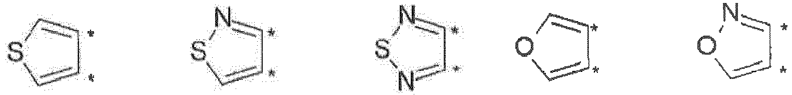 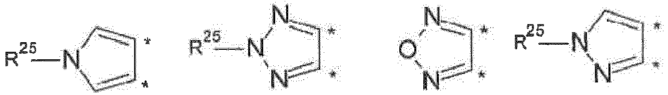 et 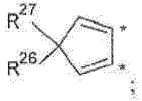 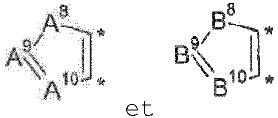 sont indépendamment choisis parmi 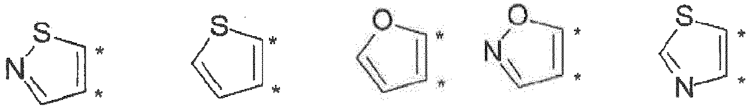 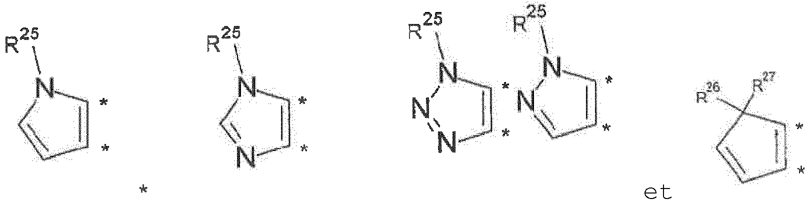 et 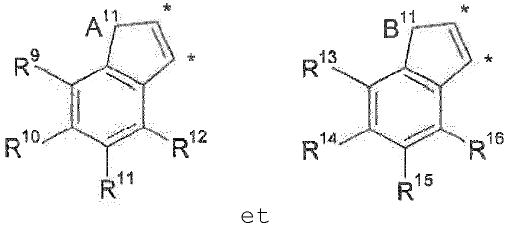 sont indépendamment choisis parmi 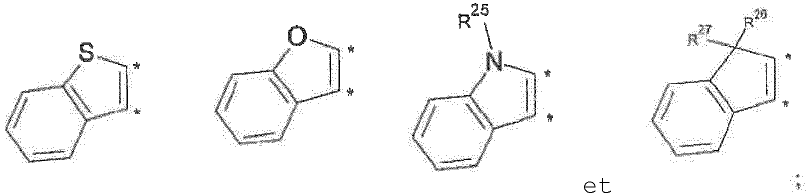 où
* représente les atomes de carbone du squelette de base.

5. Dispositif électronique organique selon l'une quelconque des revendications 1 à 4, où le composé de formule (I) est un composé de formule Ia 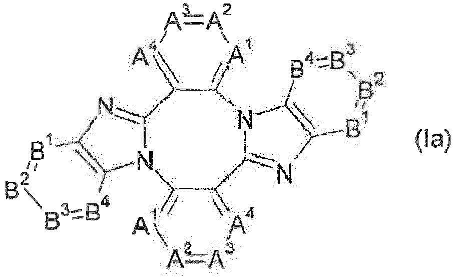 où chacun des groupements A¹, A², A³, A⁴, B¹, B², B³ et B⁴ est mentionné plus d'une fois dans la formule (Ia) et peut être identique ou différent.

6. Dispositif électronique organique selon la revendication 5, où le composé de formule (Ia) est choisi parmi les composés suivants 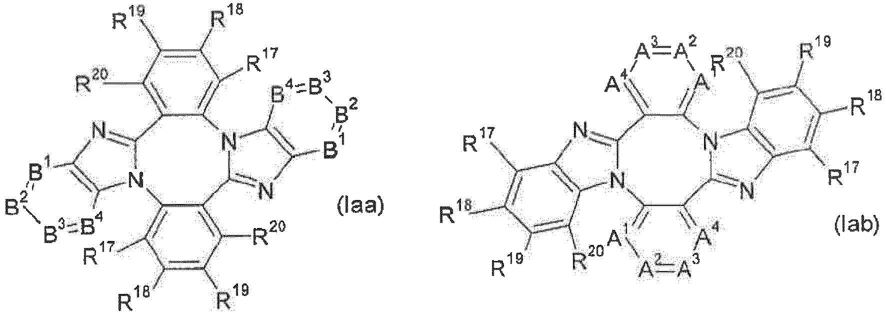 où chacun des groupements A¹, A², A³, A⁴, B¹, B², B³ et B⁴ et chacun des résidus R¹⁷, R¹⁶, R¹⁹, R²⁰ est mentionné plus d'une fois dans la formule (Iaa) et (Iab) et peut être identique ou différent.

7. Dispositif électronique organique selon la revendication 6, où le composé de formule (I) est le composé suivant 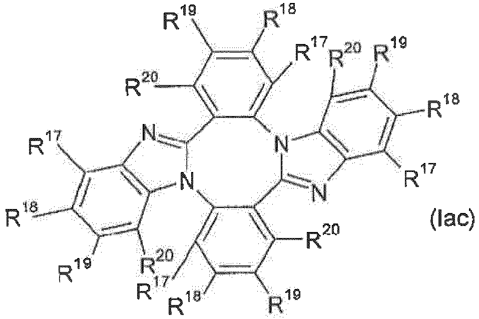 où chacun des résidus R¹⁷, R¹⁸, R¹⁹, R²⁰ est mentionné plus d'une fois dans la formule (Iac) et peut être identique ou différent.

8. Dispositif électronique organique selon la revendication 7, où le composé de formule (I) est choisi parmi les composés suivants 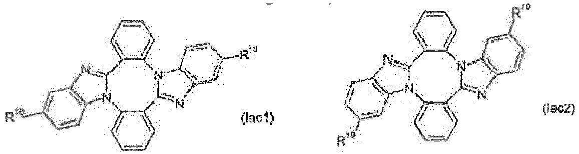 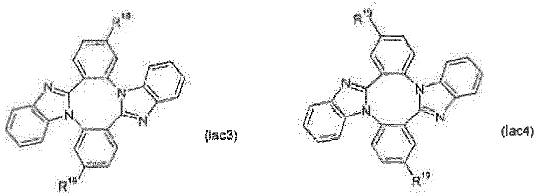 où chacun des résidus R¹⁸ et R¹⁹ est mentionné plus d'une fois dans les formules (Iac1), (Iac2), (Iac3), (Iac4) et peut être identique ou différent.

9. Dispositif électronique organique selon l'une quelconque des revendications 1 à 8, où chacun des radicaux R⁹, R¹⁰, R¹¹, R¹², R¹³, R¹⁴, R¹⁵, R¹⁶, R¹⁷, R¹⁸, R¹⁹, R²⁰, R²¹, R²², R²³ et R²⁴ représente indépendamment un atome d'hydrogène, F ou un groupement CN, aryle en C₆-C₁₈ ; aryle en C₆-C₁₈ qui est substitué par alkyle en C₁-C₁₈ ou alkoxy en C₁-C₁₈ ; alkyle en C₁-C₁₈ ; ou alkyle en C₁-C₁₈ interrompu par -O- ; ou un groupement de formule -(Ar¹)ₒ-(Ar²)ₚ-(Ar³)_{q}-A, où A est choisi dans le groupe constitué par
H, CN, 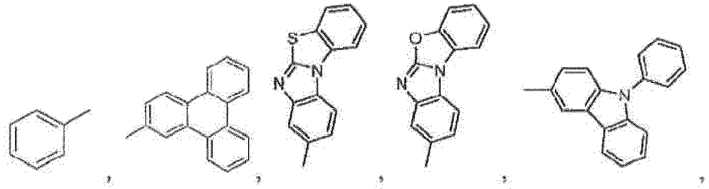 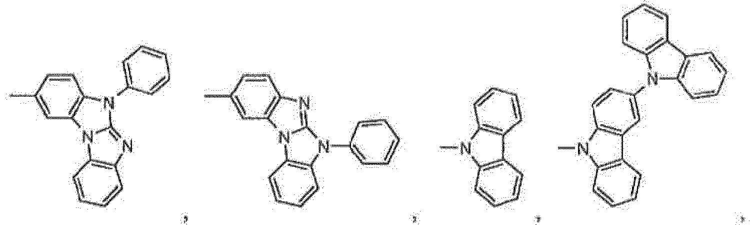 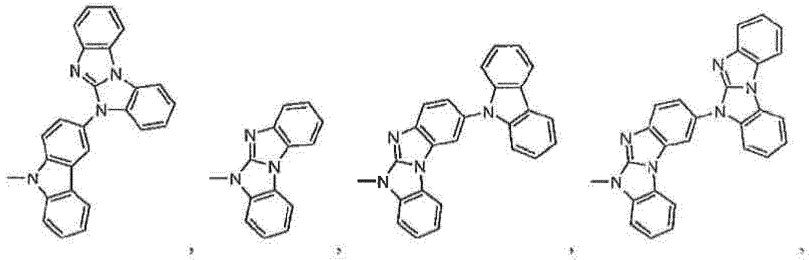 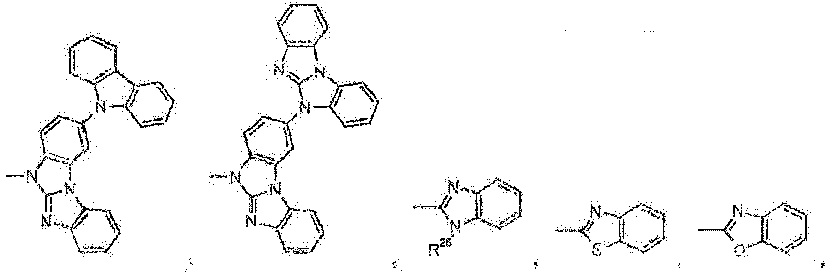 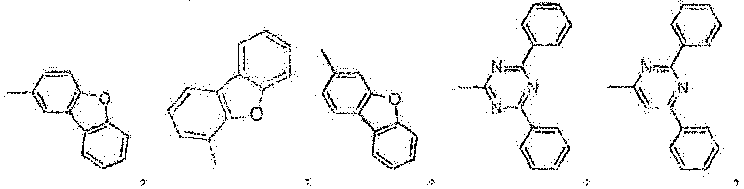 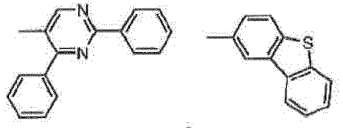 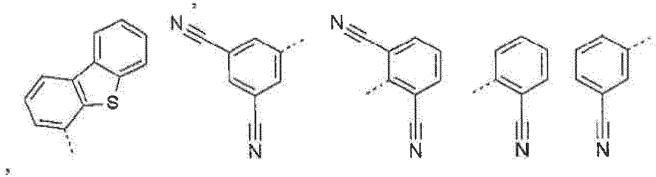 et 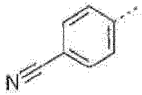 où
R²⁸ représente un atome d'hydrogène ou un groupement alkyle en C₁-C₂₅, qui est éventuellement substitué par E et/ou interrompu par D ou un groupement cycloalkyle en C₃-C₂₅, qui est éventuellement substitué par G et/ou interrompu par D, aryle en C₆-C₂₂, qui est éventuellement substitué par G, hétéroaryle comportant 5 à 22 chaînons, qui est éventuellement substitué par G ;
de façon préférentielle, A est choisi dans le groupe constitué par H, CN, 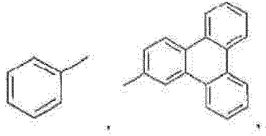 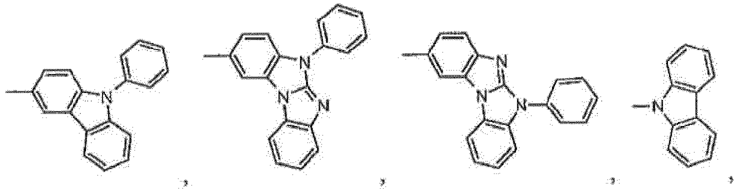 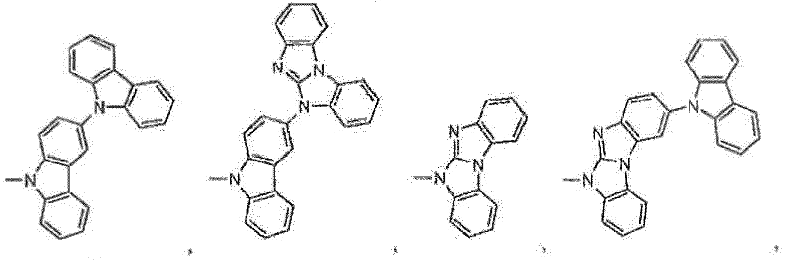 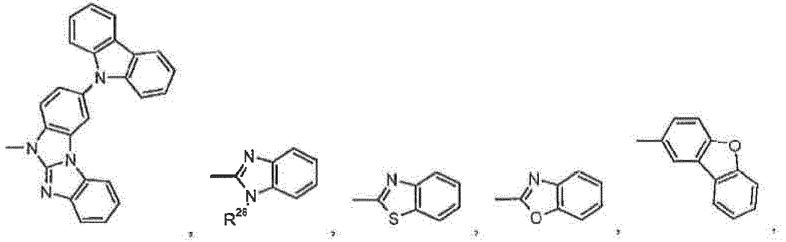 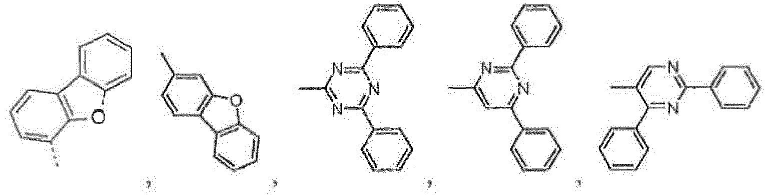 ou 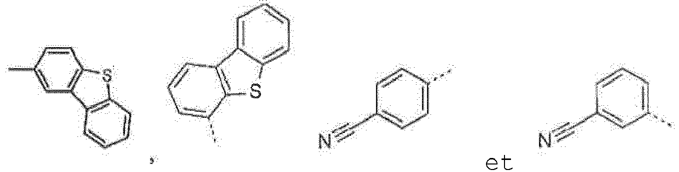 où
R²⁸ représente un atome d'hydrogène ou un groupement alkyle en C₁-C₂₅, qui est éventuellement substitué par E et/ou interrompu par D ou un groupement cycloalkyle en C₃-C₂₅, qui est éventuellement substitué par G et/ou interrompu par D, aryle en C₆-C₂₂, qui est éventuellement substitué par G, hétéroaryle comportant 5 à 22 chaînons, qui est éventuellement substitué par G ;
chacun des radicaux Ar¹, Ar² et Ar³ représente indépendamment des autres un groupement de formule 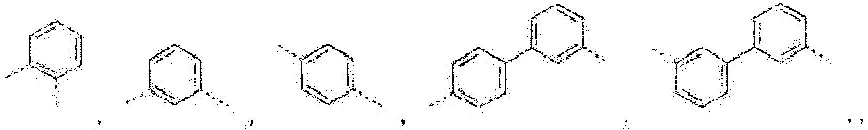 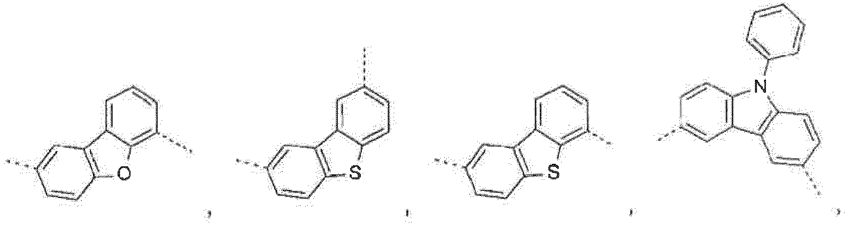 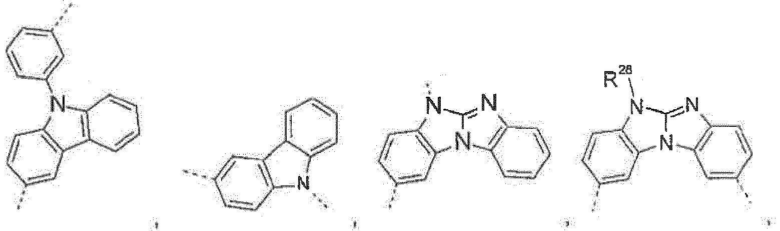 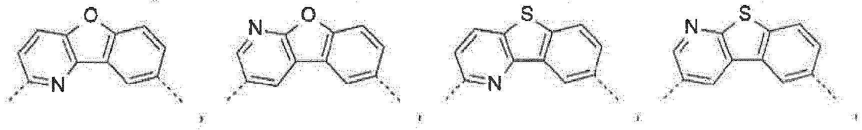 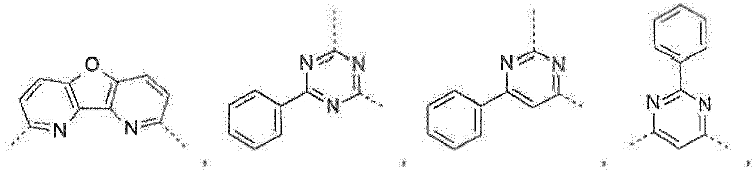 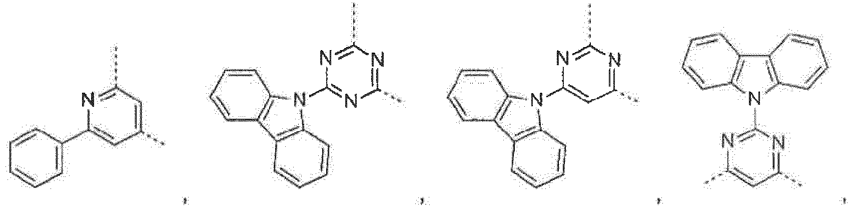 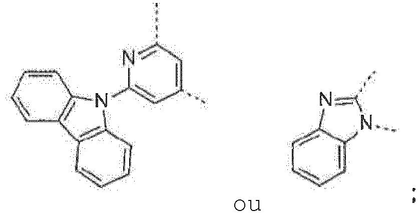 où
R²⁸ représente un atome d'hydrogène ou un groupement alkyle en C₁-C₂₅, qui est éventuellement substitué par E et/ou interrompu par D ou un groupement cycloalkyle en C₃-C₂₅, qui est éventuellement substitué par G et/ou interrompu par D, aryle en C₆-C₂₂, qui est éventuellement substitué par G, hétéroaryle comportant 5 à 22 chaînons, qui est éventuellement substitué par G ;
de façon préférentielle, chacun des radicaux Ar¹, Ar² et Ar³ représente indépendamment des autres un groupement de formule 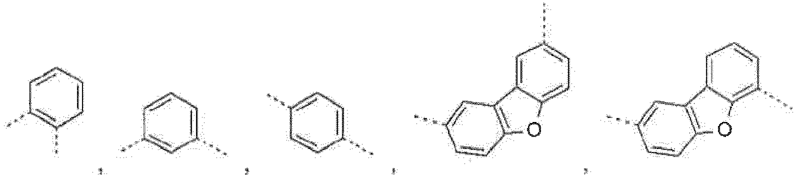 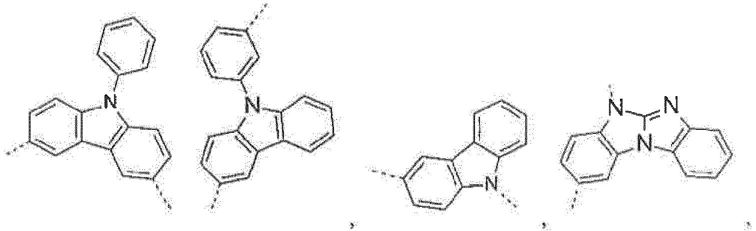 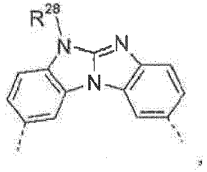 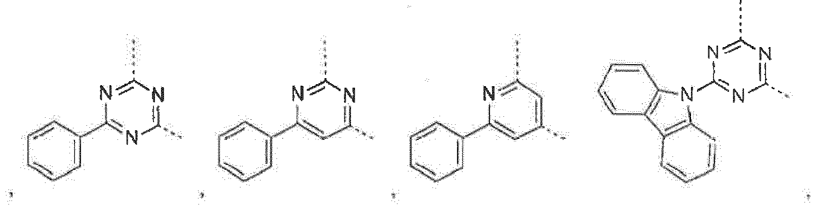 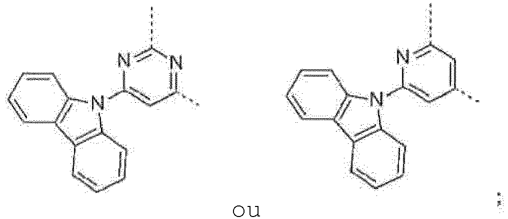 est préférentiellement, chacun des radicaux Ar¹, Ar² et Ar³ représente indépendamment des autres un groupement de formule 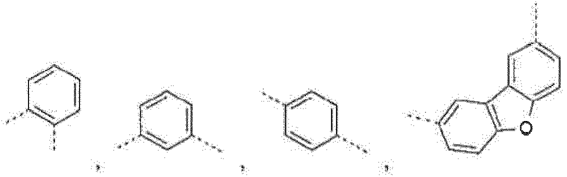 ou 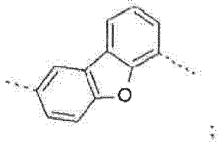 o est égal à 0 ou à 1 ;
p est égal à 0 ; ou à 1 ; et
q est égal à 0 ou à 1.

10. Dispositif électronique organique selon l'une quelconque des revendications 1 à 9, qui est un dispositif électroluminescent.

11. Couche de transport de charges, couche bloquant les excitons/charges, ou couche d'émission comprenant un composé selon l'une quelconque des revendications 1 à 9.

12. Couche d'émission selon la revendication 11, comprenant un composé selon l'une quelconque des revendications 1 à 9 en tant que matériau hôte en combinaison avec un émetteur phosphorescent.

13. Dispositif choisi dans le groupe constitué par les unités d'affichage visuel stationnaire ; les unités d'affichage visuel mobile ; les unités d'éclairage ; les claviers ; les vêtements ; les meubles ; les papiers peints, comprenant le dispositif électronique organique selon l'une quelconque des revendications 1 à 9, ou la couche de transport de charges, la couche bloquant les excitons/charges, ou la couche d'émission selon la revendication 11.

14. Utilisation des composés de formule (I) selon l'une quelconque des revendications 1 à 9 dans des photorécepteurs électrophotographiques, des convertisseurs photoélectriques, des cellules photovoltaïques organiques, des éléments de commutation, des transistors photoémetteurs organiques à effet de champ, des capteurs d'images, des lasers colorés et des dispositifs électroluminescents.

15. Procédé de production d'un composé de formule (I) 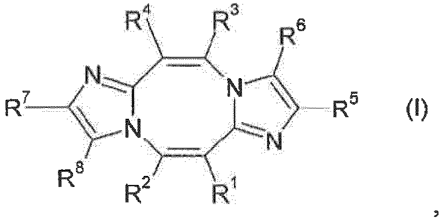 où
chacun des radicaux R¹, R², R³, R⁴, R⁵, R⁶, R⁷ et R⁸ est tel que défini dans la revendication 1, comprenant
a) la réaction d'un composé de formule 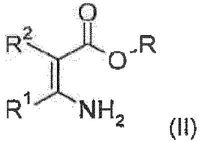 et d'un composé de formule 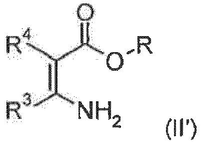 avec une base dans un solvant pour obtenir un composé de formule 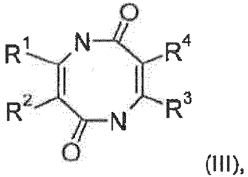 où R représente un groupement alkyle en C₁-C₁₈ ; alkyle en C₁-C₁₈ interrompu par -O- ; cycloalkyle en C₃-C₂₅ ; un groupement aryle en C₆-C₁₈ ; ou un groupement hétéroaryle comportant 5 à 22 chaînons ;
b) la réaction du composé de formule (III) avec une base et un composé de formule 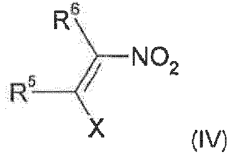 et un composé de formule 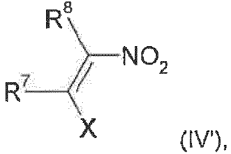 où X représente F, Cl, Br ou I, dans un solvant pour obtenir un composé de formule 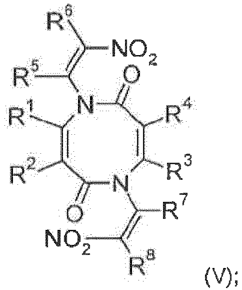
c1) la réaction du composé de formule (V) avec Sn/HCl dans un solvant pour obtenir un composé de formule (I) ; ou
c2) la réduction du composé de formule (V) avec H₂ en présence de Pd/C dans un solvant pour obtenir un composé de formule 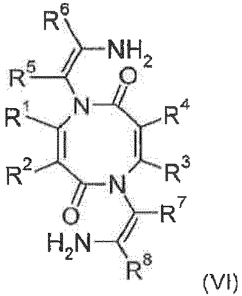 et
d) la réaction du composé de formule (VI) avec un acide pour obtenir un composé de formule (I).

16. Composé de formule (I) selon l'une quelconque des revendications 1 à 9, à la condition que les composés suivants soient exclus : 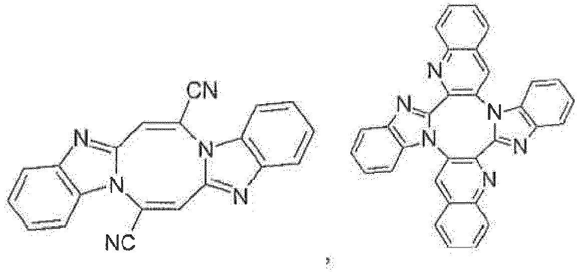 et

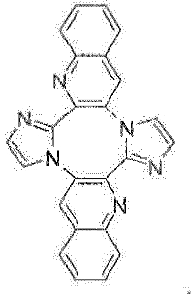

17. Composé selon la revendication 16, où
chacun des radicaux R¹, R², R³, R⁴, R⁵, R⁶, R⁷ et R⁸ représente indépendamment un atome d'hydrogène ou d'halogène ou un groupement NR²⁶R²⁷, alkyle en C₁-C₂₅, qui est éventuellement substitué par E et/ou interrompu par D ou un groupement cycloalkyle en C₃-C₂₅, qui est éventuellement substitué par G et/ou interrompu par D, aryle en C₆-C₂₂, qui est éventuellement substitué par G, hétéroaryle comportant 5 à 22 chaînons, qui est éventuellement substitué par G ; ou
R¹ et R² et/ou R³ et R⁴ et/ou R⁵ et R⁶ et/ou R⁷ et R⁸ indépendamment les uns des autres, forment ensemble et avec les atomes de carbone auquel ils sont liés un cycle (hétéro) aromatique à 5 ou 6 chaînons, qui fait éventuellement partie d'un système cyclique annelé, où le cycle (hétéro)aromatique et/ou le système cyclique annelé sont éventuellement substitués par G ou par un groupement de formule -(Ar¹)ₒ-(Ar²)ₚ-(Ar³)_{q}-A ; et les hétéroatomes du cycle hétéroaromatique à 5 ou 6 chaînons sont choisis parmi N, S et O ; préférentiellement,
R¹ et R² et/ou R³ et R⁴,
forment indépendamment les uns des autres un cycle (hétéro)aromatique à 5 ou 6 chaînons choisi parmi 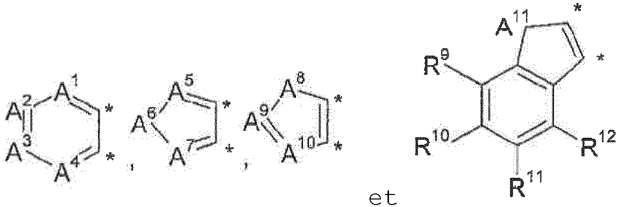 et/ou
R⁵ et R⁶ et/ou R⁷ et R⁸,
forment indépendamment les uns des autres un cycle (hétéro)aromatique à 5 ou 6 chaînons choisi parmi 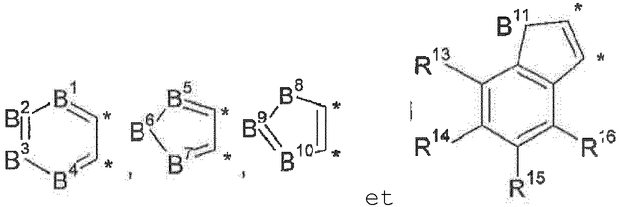 où
A¹ et B¹ représentent indépendamment N ou CR¹⁷,
A² et B² représentent indépendamment N ou CR¹⁸,
A³ et B³ représentent indépendamment N ou CR¹⁹,
A⁴ et B⁴ représentent indépendamment N ou CR²⁰,
A⁵ et B⁵ représentent indépendamment N ou CR²¹,
A⁶ et B⁶ représentent indépendamment O, S, NR²⁵ ou CR²⁶R²⁷,
A⁷ et B⁷ représentent indépendamment N ou CR²²,
A⁸ et B⁸ représentent indépendamment O, S, NR²⁵ ou CR²⁶R²⁷,
A⁹ et B⁹ représentent indépendamment N ou CR²³,
A¹⁰ et B¹⁰ représentent indépendamment N ou CR²⁴,
A¹¹ et B¹¹ représentent indépendamment O, S, NR²⁵ ou CR²⁶R²⁷,
chacun des radicaux R⁹, R¹⁰, R¹¹, R¹², R¹³, R¹⁴, R¹⁵, R¹⁶, R¹⁷, R¹⁰, R¹⁹, R²⁰, R²¹, R²², R²³ et R²⁴ représente indépendamment un atome d'hydrogène ou d'halogène ou un groupement CN, NR²⁶R²⁷, alkyle en C₁-C₂₅, qui est éventuellement substitué par E et/ou interrompu par D ou un groupement cycloalkyle en C₃-C₂₅, qui est éventuellement substitué par G et/ou interrompu par D, aryle en C₆-C₂₂, qui est éventuellement substitué par G, hétéroaryle comportant 5 à 22 chaînons, qui est éventuellement substitué par G, ou un groupement de formule -(Ar¹)ₒ-(Ar²)ₚ-(Ar³)_{q}-A ;
chacun des radicaux R²⁵, R²⁶, R²⁷
représente indépendamment un atome d'hydrogène ou un groupement alkyle en C₁-C₂₅, qui est éventuellement substitué par E et/ou interrompu par D ou un groupement cycloalkyle en C₃-C₂₅, qui est éventuellement substitué par G et/ou interrompu par D, aryle en C₆-C₂₂, qui est éventuellement substitué par G, hétéroaryle comportant 5 à 22 chaînons, qui est éventuellement substitué par G ;
* représente les atomes de carbone du squelette de base ;
D représente -CO- -COO-, -S-, -SO-, -SO₂, -O-, -NR⁶⁵-,-SiR⁷⁰R⁷¹-, -POR⁷²-, CR⁶³=CR⁶⁴- ou - C≡C-,
E représente F ; Cl ; Br ; CN ; un groupement aryle en C₆-C₂₂ non substitué, un groupement aryle en C₆-C₂₂, qui est substitué par F, alkyle en C₁-C₁₈, alkyle en C₁-C₁₈ interrompu par O ; un groupement hétéroaryle non substitué comportant 5 à 22 chaînons, un groupement hétéroaryle comportant 5 à 22 chaînons, qui est substitué par F, -OR⁶⁹, -SR⁶⁹, -NR⁶⁵R⁶⁶, -COR⁶⁸, -COOR⁶⁷,-CONR⁶⁵R⁶⁶, -CN ou F ;
G représente E, -OR⁶⁹ ; -SR⁶⁹ ; -NR⁶⁵R⁶⁶ ;
chacun des radicaux R⁶³ et R⁶⁴ représente indépendamment de l'autre H ou un groupement aryle en C₆-C₁₈ ; un groupement aryle en C₆-C₁₈, qui est substitué par alkyle en C₁-C₁₈ ou alkoxy en C₁-C₁₈ ; un groupement alkyle en C₁-C₁₈ ; ou un groupement alkyle en C₁-C₁₈ interrompu par -O- ;
chacun des radicaux R⁶⁵ et R⁶⁶ représente indépendamment de l'autre un groupement aryle en C₆-C₁₈ ; un groupement aryle en C₆-C₁₈, qui est substitué par alkyle en C₁-C₁₈ ou alkoxy en C₁-C₁₈ ; un groupement alkyle en C₁-C₁₈ ; ou un groupement alkyle en C₁-C₁₈ interrompu par -O- ; ou
R⁶⁵ et R⁶⁶ forment ensemble un cycle à cinq ou six chaînons,
R⁶⁷ représente un groupement aryle en C₆-C₁₈ ; un groupement aryle en C₆-C₁₈, qui est substitué par alkyle en C₁-C₁₈ ou alkoxy en C₁-C₁₈ ; un groupement alkyle en C₁-C₁₈ ; ou un groupement alkyle en C₁-C₁₈ interrompu par -O-,
R⁶⁸ représente H ; un groupement aryle en C₆-C₁₈ ; un groupement aryle en C₆-C₁₈, qui est substitué par alkyle en C₁-C₁₈ ou alkoxy en C₁-C₁₈ ; un groupement alkyle en C₁-C₁₈ ; ou un groupement alkyle en C₁-C₁₈ interrompu par -O-,
R⁶⁹ représente un groupement aryle en C₆-C₁₈ ; un groupement aryle en C₆-C₁₈, qui est substitué par alkyle en C₁-C₁₈ ou alkoxy en C₁-C₁₈ ; un groupement alkyle en C₁-C₁₈ ; ou un groupement alkyle en C₁-C₁₈ interrompu par -O-,
chacun des radicaux R⁷⁰ et R⁷¹ représente indépendamment de l'autre un groupement alkyle en C₁-C₁₈, un groupement aryle en C₆-C₁₈ ou un groupement aryle en C₆-C₁₈, qui est substitué par alkyle en C₁-C₁₈, et
R⁷² représente un groupement alkyle en C₁-C₁₈, un groupement aryle en C₆-C₁₈ ou un groupement aryle en C₆-C₁₈, qui est substitué par alkyle en C₁-C₁₈ ;
chacun des radicaux Ar¹, Ar², Ar³ représente indépendamment des autres un groupement arylène en C₆-C₂₄, qui est éventuellement substitué par G, ou un groupement hétéroarylène en C₂-C₃₀, qui est éventuellement substitué par G ;
A représente un atome d'hydrogène ou d'halogène ou un groupement CN, NR²⁶R²⁷, alkyle en C₁-C₂₅, qui est éventuellement substitué par E et/ou interrompu par D ou un groupement cycloalkyle en C₃-C₂₅, qui est éventuellement substitué par G et/ou interrompu par D, aryle en C₆-C₂₂, qui est éventuellement substitué par G, hétéroaryle comportant 5 à 22 chaînons, qui est éventuellement substitué par G ;
o est égal à 0 ou à 1 ;
p est égal à 0 ou à 1 ; et
q est égal à 0 ou à 1.
